(19) Europäisches Patentamt
European Patent Office
Office européen des brevets

(11) EP 2 428 200 A2

(12) EUROPEAN PATENT APPLICATION

(43) Date of publication:
**14.03.2012 Bulletin 2012/11**

(21) Application number: **11181313.5**

(22) Date of filing: **29.11.2007**

(51) Int Cl.:
***A61K 8/02*** (2006.01) ***A61K 8/14*** (2006.01)
***A61K 8/55*** (2006.01) ***A61K 8/97*** (2006.01)
***A61Q 19/00*** (2006.01) ***A61K 9/107*** (2006.01)
***G01N 33/68*** (2006.01) ***A61K 47/48*** (2006.01)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.11.2006 GB 0623838**
**05.12.2006 GB 0624290**
**08.12.2006 US 873560 P**

(62) Document number(s) of the earlier application(s) in accordance with Art. 76 EPC:
**07824940.6 / 2 099 410**

(71) Applicant: **Malvern Cosmeceutics Limited**
**Malvern, Worcestershire WR14 3SZ (GB)**

(72) Inventors:
- **Tonge, Stephen**
  **Malvern, Worcestershire WR14 3SZ (GB)**
- **Harper, Andrew**
  **Malvern, Worcestershire WR14 3SZ (GB)**

(74) Representative: **Goodall, Scott**
**Sagittarius IP**
**Taylor House**
**39 High Street**
**Marlow**
**Buckinghamshire SL7 1AF (GB)**

Remarks:
This application was filed on 14-09-2011 as a divisional application to the application mentioned under INID code 62.

# (54) Novel compositions

(57) A composition comprising lipid and surfactant, characterised in that the surfactant has an HLB number of less than 20 and in that the lipid and surfactant are in the form of macromolecular assemblies of less than 100 nm in diameter.

Figure 3

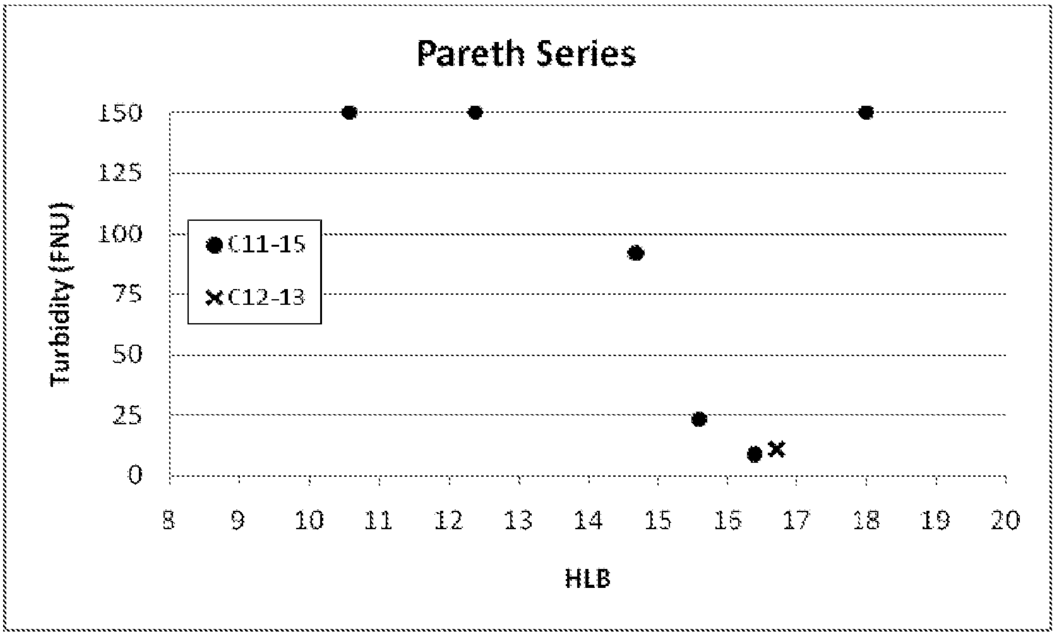




Printed by Jouve, 75001 PARIS (FR)

## Description

The present invention relates *inter alia* to compositions of use in the solubilisation of hydrophobic substances, particularly in the solubilisation of hydrophobic active agents which are of use in the field of cosmetics or pharmaceuticals, and in the solubilisation of peptides and proteins for the investigation of their structure and their interactions with other substances.

## BACKGROUND

Poor water solubility presents a fundamental problem in delivering oil-soluble active materials to sites within or topically upon the body. Numerous formulating aids have been adopted to overcome this limitation, aiming to produce aqueous formulations that are more functionally and/or aesthetically acceptable. Approaches include the use of surfactant systems, liposomes, niosomes and cyclodextrins, amongst others. However, all of these systems have particular drawbacks. For example: liposomes and cyclodextrins may have a low loading capacity; liposomal formulations may be rapidly removed from the systemic circulation after intravenous administration; both liposomes and niosomes may suffer from a lack of clarity; and the use of certain surfactants may result in the formation of irritating compositions.

Oil soluble active materials are frequently applied to the skin as part of water-in-oil or oil-in-water emulsions, typically in the form of creams or lotions. These are generally oily to the touch and may be aesthetically unpleasant, leading to a low consumer appeal. Furthermore, they may be physically unstable, tending to separate out or "cream" on standing, limiting both the shelf-life and potentially causing heterogeneity in the composition which may lead to unpredictability in the application of active agents.

## The HLB System

In order to function as a surfactant, a compound must necessarily include at least one hydrophilic moiety (polar or charged) and at least one hydrophobic/lipophilic moiety (non-polar). The HLB system provides an empirical parameter often assigned to a surfactant in order to characterise its hydrophilic/hydrophobic balance (see Griffin, WC Journal of the Society of Cosmetic Chemists 1949: 1:311-326; Griffin WC Journal of the Society of Cosmetic Chemists 1954 5: 249-256; Florence AT et al Physiochemical Principles of Pharmacy, Chapman & Hall, London, England, 1982 (in particular pages 234-235); Aulton ME Pharmaceutics - The Science of Dosage Form Design, Churchill Livingstone, 2002 (in particular Chapter 6 pages 96-97, Chapter 23 pages 345-347)). Surfactants having higher HLB values are generally more hydrophilic, with those having lower HLB values generally being more hydrophobic.

The HLB of polyhydric alcohol fatty acid esters such as glycerol monostearate may be obtained from the equation:

$$HLB = 20\,[1-(S/A)]$$

where S is the saponification number of the ester and A is the acid number of the fatty acid. Based on this relationship, the HLB of polyoxyethylene-20 sorbitan monolaurate is determined to be 16.7 (S being 45.5, A being 276).

In the case of materials for which it is not possible to determine saponification numbers, HLB is calculated from:

$$HLB = (E+P)/5$$

where E is the percentage by weight of oxyethylene chains and P is the percentage by weight of polyhydric alcohol groups (glycerol or sorbitol). If the hydrophile consists only of oxyethylene groups, the HLB equation may be simplified to:

$$HLB = (E)/5$$

Calculation of the contributions made by the various functional groups present within the molecule is possible using the formula:

$$HLB = [(\text{sum of hydrophilic group numbers})-(\text{sum of lipophilic group numbers})] + 7$$

where the group numbers associated with specific moieties have been determined quantitatively (see Davies JT et al Interfacial Phenomena, Academic Press, New York, 1961).

Although the HLB system was developed for application to non-ionic surfactants, it is possible to estimate equivalent numbers for ionic surfactants by taking account of the hydrophilic contribution of the ionic groups under given conditions. Sodium lauryl sulphate (also known as SDS) is considered to be among the most potent of common detergents (McCutcheon's Volume 1: Emulsifiers & Detergents, International Edition, MC Publishing Company, Glen Rock, NJ, USA, 2005).

The HLB of a mixture of two surfactants containing fraction f of component A and (1-f) of component B is an algebraic mean of the two HLB numbers:

$$HLB_{mixture} = f[HLB_A] + (1\text{-}f)[HLB_A]$$

Additionally, it should be noted that many commercial surfactant products are not pure compounds, rather being complex mixtures of compounds, and the HLB value reported in the literature for a particular surfactant may more accurately be characteristic of a commercial product of which the compound is the major component. As a result, commercial products having the same primary surfactant component can have slightly different HLB values when sourced from different suppliers, due to manufacturing variations which lead to the presence of different impurities and quantities thereof. Variation can also occur, to some degree, between different batches obtained from the same supplier (particularly where the surfactants are derived from a mixture of natural products, for example, castor oil or lanolin based surfactants).

HLB theory is explained quantitatively by Israelachvili JN *Intermolecular and Surface Forces,* 2nd edition, Academic Press, London, 1991, using a theory of critical packing parameters defined by:

$$P = v/(a_0 l_c)$$

where P is the critical packing parameter (defining the 'shape' of the surfactant assembly - cone, truncated cone, cylinder or inverted truncated cone), v is the volume of the hydrophobic chain, $a_0$ is the surface area of the polar headgroup and $l_c$ is the critical chain length of the hydrophobic tail of the surfactant.

The HLB values for a range of surfactants are provided in the Examples.

**Conventional carrier systems**

Solid lipid nanoparticles (SLN), also known as nanostructured lipid carriers (NLC), have been developed by PharmaSol GmbH and are described by Müller RH et al Advanced Drug Delivery Reviews 2002 54(Suppl 1):S131-S155 and in US6,770,299. SLN consist of lipid in water emulsions where the lipid chosen is solid at body temperature (e.g. melting at >50°C). An active compound is first dissolved, solubilised or dispersed in melted lipid. This mixture is then either (i) dispersed, while melted, into a hot surfactant solution and homogenised before being allowed to cool to form solid lipid nanoparticles or (ii) allowed to cool, milled into microparticles which are then dispersed into cold surfactant solution and homogenised to form solid lipid nanoparticles. Solid lipid nanoparticles are typically in the range of 200 to 600 nm. SLN may protect incorporated active compounds against chemical degradation and can also demonstrate flexibility in modulating the release of compounds. However, SLN are insoluble in aqueous formulation and as a result of their large size the ability of SLN to effectively penetrate the skin may be expected to be limited.

US5,853,755 and US6,656,499 (PharmaDerm Laboratories Ltd) describe large biphasic lipid vesicles of 0.1-100 um, which form milky solutions. In this system, phospholipid bilayers are present as multilamellar vesicles within which a surfactant stabilised emulsion containing a hydrophobic active agent is present.

Liposomes are closed phospholipid bilayer systems and exist in two main forms - either as unilamellar vesicles (ULV) or multi-lamellar vesicles (MLV) in which bilayers are arranged concentrically in an 'onion-like' arrangement. The amphiphilic character of the bilayer structure enables entrapment of hydrophobic agents between the fatty acyl chains of the bilayer and hydrophilic agents within the aqueous regions between bilayers and within the core. Vesicles can range in size from around 20 nm to around 3 um (20-50 nm, Jamil H et al. Modern Drug Discovery 2004 7:37-39; 40-180 nm Zumbuehl O and Weder H Biochem. Biophys. ACTA 1981 640:252-262; 100-500 nm, Chapter 5, Section 5.2.1 - Liposomes, Transdermal Drug Delivery, Williams A (Ed), Pharmaceutical Press (London) 2003). The principle disadvantages of liposomes are lack of stability and storage problems, which have limited their application. Liposomes have been suggested for cosmetic use, such as in US4,508,703, where such particles are said to form an opalescent suspension having particle sizes of less than 3 um. Still larger phospholipid bilayer particulate structures such as bicelles have also been described.

US6,165,500 (Idea AG) describes an adaptable bilayer vesicle comprising a phospholipid combined with edge activators which include alcohols and surfactants such as cholates or polyoxyethylene ethers. These ultradeformable particles are termed Transferosomes® and are suitable for delivering hydrophilic and lipophilic agents through the hydrophilic pores in the skin. Transferosomes® ranging from 200 to 600 nm in size are exemplified, physically appearing in the form of milky emulsions. For dermal delivery applications, a preference for particle sizes in the range of 100 to 200 nm is given.

Cevc G Advanced Drug Delivery Reviews 2004 56:675-711, which is authored by the inventor of US6,165,500, provides a review of lipid vesicles and other colloids as drug carriers for application to the skin, discussing in some detail the skin structure and the requirements this imposes on effective delivery systems. The author mentions that poorly deformable systems, such as most lipid/surfactant/oil mixtures require high energy input to transform them into small particles, meaning that they are seldom stable on long-term storage. Furthermore, the variation of relative or absolute surfactant/phospholipid/water/oil concentrations frequently triggers phase transition, which can be accompanied by collapse of the system. In the author's opinion, only vesicular forms of lipid/surfactant mixtures in water are practically meaningful for colloid-mediated transdermal drug delivery, since mixed lipid micelles and such like are confined to the skin surface.

WO00/50007 (Lipocine Inc) discloses pharmaceutical compositions containing a hydrophilic surfactant, a hydrophobic surfactant and a hydrophobic therapeutic agent which when diluted in aqueous medium form clear dispersions. The compositions are primarily directed for oral delivery applications. Particle size analysis indicates that exemplary compositions when diluted contain particles in the region of 6 to 15 nm in diameter. The inventors describe the particles as meta-stable, and state that the particles do not suffer problems of precipitation in the time frame relevant for absorption (tested over 6 hours). The solubilisation capabilities of the system are demonstrated using progesterone as an active agent - aqueous dispersions were prepared with progesterone at a maximum concentration of 1.76 mg/ml using 198 mg of carrier (i.e. approximately 1.0% active loading by dry weight). An extensive range of potential compositions are described, although no compositions comprising phospholipids are exemplified. US6,267,985 (Lipocine Inc) describes the use of a triglyceride based system for topical application comprising a triglyceride, a hydrophilic surfactant, a hydrophobic surfactant and a therapeutic agent soluble therein. The system apparently forms clear dispersions upon dilution in an aqueous solvent that remain stable upon further dilution.

Studies with phospholipid vesicles with differing phosphatidylcholine (PC) content (Hofland HEJ et al British Journal of Dermatology 1995 132:853-856) suggest that those with high phosphatidylcholine content and high content of lysophosphatidylcholine (lysoPC) can penetrate into the *stratum corneum,* possibly due the action of lysoPC acting as an edge activator and increasing the elasticity of the vesicles. Hence, liquid-state elastic vesicles are able to penetrate the skin more readily than gel-state vesicles and this may enhance drug permeation. Elastic vesicles of 100 to 150 nm in diameter have been produced from polyoxyethylene laurate ester PEG-8 laurate (HLB number 7) and egg phosphatidylcholine, as described by van den Bergh BAI et al Biochemica et Biophysica Acta 1999 1461:155-173.

Bouwstra JA et al Advanced Drug Delivery Reviews 2002 54(Suppl 1):S41-S55 provides a review article on the skin structure and mode of action of vesicles in dermal delivery applications, citing du Plessis J et al International Journal of Pharmaceutics 1994 103:277-282, it is commented that vesicle size does not effect drug deposition as vesicles do not penetrate the skin intact. Furthermore, it was noted that rigid vesicles and micelles only penetrated into the superficial *stratum corneum* layers.

Niosomes or non-ionic surfactant based liposomes are analogous to the bilayer structures found in liposomes and composed of phospholipid-free mixtures of non-ionic surfactants and other membrane additives such as cholesterol. The advantage of niosomes is improved stability and the use of cheaper raw materials. Niosomes were first described in US4,217,344 (L'Oreal) which suggested the use of vesicles of 100 to 1000 nm in diameter. Examples include the use of mixtures of oleth-10 and oleth-2 together with glycerol which form "milky dispersions". Subsequently, the more readily available sorbitan fatty acid esters with an HLB of 4-8 were found to be compatible with niosome vesicle formation - these materials are biodegradable, cheap and non-toxic and have been extensively applied in the cosmetic and pharmaceutical fields (Uchegbu IF et al Advances in Colloid and Interface Science 1995 58:1-55; Uchegbu IF et al International Journal of Pharmaceutics 1998 172:33-70). The ability of the surfactant mixture to form bilayer membranes seems to be essential for the formation of niosomes, e.g. polysorbate 20 when used in combination with cholesterol is also able to form niosomes despite its relatively high HLB of 16.7 (Santucci E et al STP Pharma Sciences 1996 6:29-32). Large disc shaped non-ionic surfactant structures such as discomes (ca. 15 to 100 um) have also been described.

Microemulsions represent another form of oil in water system (Krielgaard M Advanced Drug Delivery Reviews 2002 54(Suppl 1):S41-S55). Microemulsions have been used in cosmetic applications to solubilise and deliver oily active agents to the skin (International Federation of Societies of Cosmetic Chemists Monograph Number 7, Microemulsions in Cosmetics, Micelle Press, Dorset, England, 2001 - ISBN 1-870228-20-0).

Microemulsions show a thermodynamic equilibrium between components present and are therefore generally unstable. Microemulsions formed from phospholipid/alcohol/water can be considered as bicontinuous systems, which only operate under precise conditions of concentration (Cevc G Advanced Drug Delivery Reviews 2004 56:675-711),

being sensitive to dilution. Such microemulsions are usually present as high viscosity organo-gels. Bicontinuous microemulsion systems are distinct from microemulsions that may be considered to be oil in water emulsions on a nanoscale (Krielgaard M Advanced Drug Delivery Reviews 2002 54(Suppl 1):S41-S55). Microemulsion particles are generally considered to be between 10 to 100 nm in size (Gattefosse Technical Brochure, 1st Edition 1998).

US6,004,580 (Leiras Oy) describes the use of microemulsions for the delivery of pharmaceutical agents, which microemulsions comprise a hydrophilic component, a lipophilic component, a surfactant and a drug. Phospholipids are mentioned as examples of surfactants. Notably, the authors indicate that lecithin is too hydrophobic a surfactant to facilitate the formation of stable microemulsions in water alone and suggest that lower alcohols (such as ethanol) are used as a co-solvent. All example formulations using lecithin as surfactant contained at least 18% ethanol.

The high amounts of surfactant required to stabilise some microemulsions can prove to be irritating, and the presence of co-solvent in the aqueous phase can cause drying of the skin (Krielgaard M Advanced Drug Delivery Reviews 2002 54(Suppl 1):S41-S55).

Microemulsions suitable for use as injectables are described in US6,245,349 (Elan). These compositions contain phospholipids, propylene glycol and a surfactant (having an HLB of at least 12, preferably at least 15), water being an optional component. The presence of propylene glycol or PEG was found to be necessary, as in the absence of these components the compositions failed to produce clear emulsions and phase separated.

WO00/37042 (Beiersdorf AG), which corresponds to US20020146375A1, discloses transparent microemulsions which comprise phospholipids and surfactants; all of the exemplified compositions contain at least 5% glycerol and 2.5% oil, the oil being present in an amount at least 1.25 times the quantity of phospholipid present. WO03/082222 (Beiersdorf AG), which corresponds to US20050124705A1, discloses low viscosity emulsions which comprise phospholipids and surfactants; all of the exemplified compositions contain a minimum of 5% glycerol and 7% oil, the oil being present in an amount at least 1.75 times the quantity of phospholipid present.

Nanocapsules (US20030152635A1), unlike microemulsions, are kinetically stable and have been described as carriers for the delivery of pharmaceutical agents (Malzert-Fréon A et al International Journal of Pharmaceutics 2006 320(1-2):157-164), for example to release a tripentone cytotoxic agent. The nanocapsule materials are 25 to 100 nm in diameter (with an average of less than 50 nm), are stable to dilution, and consist of a liquid lipid core, surrounded by a lipid coat which is solid at room temperature. These nanocapsules may be considered to be a hybrid between polymeric nanocapsules and liposomes. Other authors have used a similar system to deliver docetaxel (Gaucher G et al in *Delivery of Hydrophobic Drugs through Self-Assembling Nanostructures,* Proceedings of the 2004 International Conference on MEMS, NANO and Smart Systems).

WO2006/013369 (Camurus AB) provides particulate compositions forming non-lamellar dispersions comprising a monoacyl lipid, a diacyl glycerol and a fragmentation agent. Particle sizes of exemplified compositions are in the region of 100 nm or greater, meaning that solutions would not be clear. WO2006/077362 (Camurus AB) provides particulate compositions comprising phosphatidylcholine, a diacyl glycerol component and a non-ionic stabilising amphiphile. Again, exemplified compositions are in the region of 100 nm or greater, meaning that solutions would not be clear.

**Conventional membrane solubilisation and bilayer discs**

There is an extensive literature describing the use of surfactants, such as non-ionic surfactants, to solubilise phospholipids, particularly for the study of biomembrane proteins.

Egan RW et al Journal of Biological Chemistry 1976 251:4442-4447 investigated the hydrophile-lipophile balance and critical micelle concentration as factors influencing disruption of membranes, noting that membrane bound enzymes when removed from their protective hydrophobic environment are frequently inactive. As phospholipid extraction levels mimicked protein extraction levels, the author concluded that at least a portion of solubilised membrane proteins may be in the form of lipid-protein complexes, small membrane fragments or resealed vesicles. The optimal HLB for protein and lipid extraction was found to be between 12.5 and 13.5.

The interaction of membrane forming phospholipids in the form of multilamellar vesicles with Triton™ X-100 (HLB number of 13.4) is discussed by Goni FM et al European Journal of Biochemistry 1986 160:659-665. The authors apply a number of experimental techniques for investigating the transition between multilamellar vesicles and micellar solutions, noting a clear transition between states.

Walter et al Biophysics Journal 1991 60:1315-1325 discusses the existence of intermediate structures in the vesicle-micelle transition resulting from the presence of increasing amounts of sodium cholate surfactant. It is noted by the author that bilayer discoidal intermediates had been proposed previously in the field, although no such structures had specifically been observed. Cryo-EM studies indicated the existence of extended flexible micellar tube-like structures (ca. 100 to 300 nm in length and 3 to 5 nm in diameter, having an 'effective particle size' comparing well with the 16 nm observed previously by small angle neutron scattering), as an intermediate phase between vesicles and micelles, with conventional micelles (ca. 5 to 7 nm) being the form present when samples cleared.

Almgren M Biochemica et Biophysica Acta 2000 1508:146-163 provides a review of the area of micelles and

membrane solubilisation, and mentions the existence of planar and circular discs as intermediate states between micelle/vesicle transitions which have sometimes been observed.

Funari SS et al Proceedings of the National Academy of Sciences 2001 98(16):8938-8943 discloses mixed micelles comprising the non-ionic detergent octaethyleneglycol-mono-*n*-dodecylether and either DMPC or DPPC. Octaethyleneglycol-mono-*n*-dodecylether (also known as $C_{12}E_8$, or laureth-8) has an HLB number of 13.1. The mixed micelles were formed after rapid cooling of the component mixture, this pre-treatment being stated to be particularly important in the case of DPPC containing mixed micelles. Analysis of particle size by dynamic light scattering indicated that mixed micelles containing DMPC had an average particle size of around 7 nm below 20°C and an average particle size of greater than 200 nm above 30°C. Samples utilised by the authors for NMR analysis are said to have been translucent. The mixed micelles were found to be unstable: DPPC containing mixed micelles aggregating and precipitating after 24 hours; DMPC containing mixed micelles visibly aggregating and precipitating after 1 week. The authors state that the mixed micelles represent a kinetically trapped state, the stability of which depends on the gel-state free energy of the phospholipids. A model of a mixed micelle is proposed, consisting of a discoidal phospholipid aggregate surrounded by a toroidal detergent hoop. The mixed micelles are discussed only in the context of the detergent solubilisation of partially ordered microdomains in biological membranes. Partearroyo MA et al Journal of Colloid and Interface Science 1996 178:156-159 discusses the solubilisation of phospholipid bilayers by Triton X series surfactants. Triton™ X-102 (HLB number of 14.6) was identified as the most efficient member of the series in respect of its lytic and sublytic effects.

PEG lipids are used to sterically stabilise liposomes, although high concentrations result in their solubilisation. The existence of a small bilayer discs was shown by cryo-EM in Edwards K et al Biophysical Journal 1997 73:258-266 during investigation of the effect of PEG(2000)-distearylphosphatidylethanolamine on cholesterol containing distearylphosphatidylcholine liposome and dipalmitylphosphatidylcholine liposome structure (2000 in this case indicating PEG molecular weight, which is not the convention generally used in the surfactant field). In the absence of cholesterol, the PEG(2000)-DSPE induced the formation of threadlike structures rather than discs. The authors conclude that the presence of PEG(2000)-DSPE stabilises the intermediate structures, while cholesterol is required for the preference of discoidal intermediates over threadlike intermediates.

A doctoral thesis by Elisabet Boija (Uppsalla University 2006, published as ISBN 91-554-6628-1) provides a summary of work in the area of PEG-lipid stabilised bilayer discs and discusses the use of PEG(5000)-DSPE or PEG (5000)-ceramide surfactants in the formation of disc structures with DSPC and cholesterol. Johnsson M et al Biophysics Journal 2003 85:3839-3847 uses PEG-lipids to prepare DSPC and DPPC containing discs, noting that subtle differences in the lipid composition may have a huge impact in the behaviour of the system. Johansson E et al Biophysical Chemistry 2005 113:183-192 shows that careful optimisation of a mixture of DSPC, cholesterol and PEG(5000)-DSPE can provide stable dispersions of flat bilayer discs which may be of use in the study of protein structure and delivery of protein/peptide and hydrophilic drugs.

Hydrophobically associating polymers (also known as amphipols or hypercoiling polymers, due to their amphiphilic character) may associate with phospholipids to form flattened disc-like molecular assemblies. For example, homopolymers of ethacrylic acid (i.e. poly[2-ethacrylic acid], also known as PEAA) have been shown to interact with pure DLPC, DM PC, DPPC, DSPC (respectively di-lauryl, di-myristyl, di-palmityl and di-stearyl phosphatidyl choline) and DPPG (di-palmityl phosphatidyl glycerol), and also a mixture of DPPC/DPPA (di-palmityl phospatidic acid) resulting in the formation of optically clear, aqueous solutions (Seki, K and Tirrell, D Macromolecules 1983 17:1692-1698; Tirrell, D, Takigawa, D and Seki, K Ann. New York Acad. Sci. 1985 446:237-248; Thomas, JL, Devlin BP and Tirrell, DA Biochimica et Biophysica Acta 1996 1278:73-78). This effect is the result of a conformational transition from the extended chain typical of a polyelectrolyte, through an intermediate state as a random coil, to a compact hypercoiled state at low pH.

Other hydrophobically associating polymers are also known to interact with phospholipids to form macromolecular assemblies, such as copolymers which contain hydrophilic and hydrophobic monomer components. International Patent Application WO99/009955 (equivalent to granted patents EP1007002 and US6426905) discloses the use of hydrolysed alternating copolymers of maleic anhydride (anionic, hydrophilic in its hydrolysed maleic acid form) and either styrene or an alkyl vinyl ether (hydrophobic). Structures in the region of 10 to 40 nm in diameter were prepared using a hydrolysed alternating polymer of maleic anhydride and styrene, in conjunction with pure DLPC or DPPC (for further information see the review article - Tonge, SR and Tighe, BJ Advanced Drug Delivery Reviews 2001 53:109-122).

Such polymer/lipid macromolecular complexes have been proposed as a means for the solubilisation of active agents with poor aqueous solubility. However, both of these polymer systems suffer from a number of disadvantages. PEAA is not commercially available and its suitability for use in cosmetics and pharmaceuticals has not yet been determined. Furthermore, these synthetic polymers only interact with phospholipids to form macromolecular assemblies at a pH level near or below their respective $pK_a$ value, in the case of PEAA this is 6.5 (Fichtner, F and Schonert, H Colloid & Polymer Sci. 1977 255:230-232; Thomas, JL, Devlin BP and Tirrell DA Biochimica et Biophysica Acta 1996 1278:73-78).

Alternating copolymers of styrene and maleic acid (i.e. hydrolysed styrene/maleic anhydride polymers) have a $pK_a$ value in the region of 3.75-4.0 (Sugai, S and Ohno, N Biophys. Chem. 1980 11:387-395), the $pK_a$ for the individual acid functions being approximately 1.97 and 6.24. Preparation of clear solutions, and hence macromolecular assemblies,

requires a lowering of the pH to between 3 - 5. Such pH levels are not generally suitable for compositions which are to be applied to sensitive surfaces of the body. Although the pH of these alternating copolymer formulations may be raised after the formation of the polymer/lipid complex, such adjustment leads to instability, which may be observed as a loss of clarity over time as the macromolecular assemblies degrade.

There remains a need for alternative formulating aids that enable oil-soluble agents to be incorporated into an aqueous medium at high concentration, while at the same time forming macromolecular complexes that are small enough not to disrupt the passage of light through the resultant solution, i.e. to remain substantially clear.

Contrary to the expectations of one skilled in the art, and in contrast to the teaching of WO99/009955, hydrolysed block copolymers of styrene/maleic anhydride (i.e. block copolymers of styrene/maleic acid) may be used in the preparation of macromolecular polymer/lipid complexes, such polymer/lipid complexes being of use for example in the solubilisation of oil-soluble active agents and membrane proteins. International patent application number PCT/GB2006/050134, publication number WO2006129127, discloses compositions comprising a lipid and copolymer of styrene and maleic acid, wherein the ratio of styrene to maleic acid monomer units is greater than 1:1, and wherein the polymer and lipid are in the form of macromolecular assemblies.

The present inventors have surprisingly found that certain other surfactants may be combined with lipids to form macromolecular assemblies.

Compositions of the present invention may have one or more of the following advantages compared to the solubilisation approaches of the prior art:

(i) be more stable and/or have controlled stability;
(ii) result in less irritation when used in pharmaceutical/cosmetic applications;
(iii) allow a higher loading of active agent (either by dry weight or absolute weight);
(iv) enable oil-soluble active agents to be formulated as substantially clear aqueous solutions;
(v) facilitate enhanced penetration through the skin;
(vi) be pH insensitive;
(vii) be more easily or economically produced (e.g. utilising fewer components and/or less expensive components);
(viii) contain only cosmetically/pharmaceutically acceptable components ;
(ix) contain only components of natural and/or non-animal origin;
(x) as may be desired for specific applications, utilise low molecular weight surfactant components or high molecular weight surfactant components; and
(xi) enable membrane proteins and/or peptides to be solubilised in an environment which closely mimics native membranes.

It is contrary to the expectations of one skilled in the art that the ability of a surfactant to solubilise a hydrophobic active agent may be *increased* by the addition of a lipid.

**SUMMARY OF THE INVENTION**

According to the present invention there is provided a composition comprising lipid and surfactant, characterised in that the surfactant has an HLB number of less than 20 and in that the lipid and surfactant are in the form of macromolecular assemblies of less than 100 nm in diameter.

Also there is provided a composition comprising lipid and surfactant, characterised in that the surfactant has an HLB number in the range of about 10.5 to about 17.5 and in that the lipid and surfactant are in the form of macromolecular assemblies of less than 100 nm in diameter.

Further, there is provided a composition comprising lipid and surfactant, characterised in that the surfactant is an ether surfactant and in that the lipid and surfactant are in the form of macromolecular assemblies of less than 100 nm in diameter.

Additionally, there is provided a composition comprising lipid and surfactant, characterised in that the surfactant is an ester surfactant and in that the lipid and surfactant are in the form of macromolecular assemblies of less than 100 nm in diameter.

There is also provided, a composition comprising lipid and surfactant, characterised in that the surfactant is an ionic surfactant and in that the lipid and surfactant are in the form of macromolecular assemblies of less than 100 nm in diameter.

Such compositions may be referred to herein as compositions of the invention.

According to the present invention there is provided a formulation comprising a composition of the invention and an active agent. In particular, there is provided a formulation comprising a composition of the invention and an active agent, in which the active agent is within the macromolecular assemblies.

There is provided a cosmetic preparation comprising a formulation of the invention and a cosmetically acceptable

carrier or excipient.

There is also provided a pharmaceutical preparation comprising a formulation of the invention and a pharmaceutically acceptable carrier or excipient.

In a further aspect of the present invention there is provided the use of a composition of the invention as a solubilising agent.

## BRIEF DESCRIPTION OF THE FIGURES

Figure 1 provides an illustration of the turbidity of samples prepared in Example 1 using surfactants having HLB values up to 20.

Figure 2 provides an illustration of the turbidity of samples prepared in Example 1 using ethoxyalkylated aromatic alcohol ether surfactants.

Figure 3 provides an illustration of the turbidity of samples prepared in Example 1 using ethoxyalkylated PEG pareth ether surfactants.

Figure 4 provides an illustration of the turbidity of samples prepared in Example 1 using ethoxyalkylated PEG oleth ether surfactants.

Figure 5 is the particle size analysis for an aqueous compostion of the invention containing the surfactant polysorbate 20 (2.5% w/w), the lipid 90H (0.45% w/w) and co-surfactant lyso-PC (ca. 0.01% w/w, provided in the form of SL80-3 at 0.05% w/w) - principal particle size 16.98 nm, polydispersity 0.363.

Figure 6 is the particle size analysis for an aqueous compostion of the invention containing the surfactant isoceteth-20 (2.5% w/w), the lipid 90H (0.45% w/w) and co-surfactant lyso-PC (ca. 0.01% w/w, provided in the form of SL80-3 at 0.05% w/w) - principal particle size 13.44 nm, polydispersity 0.211.

Figure 7 is the particle size analysis for an aqueous control composition containing the surfactant polysorbate 80 (2.5% w/w), the lipid 90H (0.45% w/w) and co-surfactant lyso-PC (ca. 0.01% w/w, provided in the form of SL80-3 at 0.05% w/w) - principal particle size 1107 nm, polydispersity 0.853.

Figure 8 is the particle size analysis for an aqueous compostion of the invention containing the surfactant laureth-23 (2.5% w/w), the lipid 90H (0.45% w/w), co-surfactant lyso-PC (in the form of SL80-3 at 0.05% w/w) and the active agent TECA (0.5% w/w) - principal particle size 46.76 nm, polydispersity 0.216.

## DETAILED DESCRIPTION OF THE INVENTION

The present invention relates to compositions comprising a lipid and a surfactant wherein the lipid and surfactant are in the form of macromolecular assemblies.

### Surfactants

By the term surfactant when used herein is meant a surface active component which is capable of interacting with the lipid component to form the macromolecular assemblies of the invention.

The surfactant may consist of a single component, although will often be a mixture of components (typically, though not necessarily, of similar chemical structure).

Typically, the surfactant of use in the present invention will have an HLB number in the range of about 10.5 to about 17.5, suitably about 12 to about 17, more suitably about 13.5 to about 17. In one embodiment of the invention the surfactant will have an HLB which is between 12 to less than 13. In a second embodiment of the invention the surfactant will have an HLB which is between 13 to less than 14. In a third embodiment of the invention the surfactant will have an HLB which is between 14 to less than 15. In a fourth embodiment of the invention the surfactant will have an HLB which is between 15 to less than 16. In a fifth embodiment of the invention the surfactant will have an HLB which is between 16 to less than 17. In a sixth embodiment of the invention the surfactant will have an H LB which is between 17 to less than 18.

Typically the surfactant will have a molecular weight of less than about 10000 Da, suitably less than about

8000 Da, especially less than about 5000 Da, in particular less than about 3000 Da, such as less than about 2500 (e.g. less than about 1800 Da). In certain embodiments the surfactant will have a molecular weight of between 3000 to 8000 Da.

For pharmaceutical and cosmetic applications it is desirable that the surfactant selected is suitable for pharmaceutical or cosmetic use respectively (e.g. it has been approved for pharmaceutical or cosmetic use by an appropriate authority). For certain applications it is desirable that the surfactants are biodegradable (e.g. for injectable formulations). In some applications it is desirable that the surfactant is of natural origin and/or from a non-animal source (e.g. of natural origin and from a non-animal source, such as from plants).

The surfactant of use in the present invention can be ionic (such as the anionic, cationic, and amphoteric surfactant classes described below) or non-ionic (such as the ether and ester surfactant classes described below).

A number of standard texts are available which provide detailed summaries of the more common types of surfactant: McCutcheon's Volume 1: Emulsifiers & Detergents, International Edition, MC Publishing Company, Glen Rock, NJ, USA, 2005; Handbook of Industrial Surfactants, M Ash & I Ash, Gower Publishing Company, Aldershot, England, 1993; Surfactant Encyclopaedia, Cosmetics & Toiletries Resource Series, 2nd Edition, MM Rieger, Allured Publishing Corporation, Carol Stream, USA, 1996.

The surfactant will typically not be silicone based.

*Ethers*

In one embodiment of the invention the surfactant is an ether surfactant.

The broad class of ether surfactants may be separated into a number of sub-classes which include:

- ethoxylated alcohols
- propoxylated/ethoxylated ethers
- polyglyceryl ethers
- sugar ethers

In an embodiment of the invention of particular interest the ether surfactant is an ethoxylated alcohol. In a second embodiment of the invention the ether surfactant is a propoxylated/ethoxylated ether. In a third embodiment of the invention the ether surfactant is a polyglyceryl ether. In a fourth embodiment of the invention the ether surfactant is a sugar ether.

Ethoxylated alcohol surfactants are ethylene oxide derivatives of alcohols, usually mono-functional primary alcohols or aromatic alcohols (which often have an alkyl substituent), although other alcohol derivatives are also available (e.g. sterol derivatives). Ethoxylated alcohol surfactants have the general formula:

$$R\text{-}(OCH_2CH_2)_nOH$$

wherein the group R is the moiety from the original alcohol. For convenience herein, ethoxylated alcohol surfactants are separated into those having an aromatic alcohol (ethoxylated aromatic alcohol surfactants) and those which do not have an aromatic alcohol (ethoxylated non-aromatic alcohol surfactants).

In respect of ethoxylated aromatic alcohol surfactants, suitably the surfactant HLB will be in the range from about 14.0 to about 17.0, in particular from about 14.5 to about 16.5 (such as from 14.5 to less than 15.5, or alternatively between 15.5 and 16.5). Ethoxylated aromatic alcohol surfactants of particular interest are those derived from phenol with an alkyl substituent having between 6 and 12 carbon atoms (which substituent is typically unbranched), e.g. those derived from octylphenol and nonylphenol (in particular nonylphenol). Ethoxylated aromatic alcohol surfactants of use in the present invention will typically contain between 5 and 150 PEG units, suitably between 5 and 40 PEG units, especially between 10 and 25 PEG units, in particular between 12 and 20 PEG units. Exemplary octoxynol surfactants of interest are those having 11 to 29 PEG units, such as 12 to 25 PEG units, especially 15 to 20 PEG units. Exemplary nonoxynol surfactants of interest are those having 10 to 29 PEG units, such as 10 to 25 PEG units, especially 12 to 20 PEG units (e.g. 12 to 16 PEG units). Specific examples of ethoxylated aromatic alcohol surfactants of use in the present invention are octoxynol-12, nonoxynol-15, octoxynol-16 and nonoxynol-20.

In respect of ethoxylated non-aromatic alcohol surfactants, suitably the surfactant HLB will be in the range from about 12.5 to about 17.5, in particular about 13.0 to about 17.0.

Ethoxylated non-aromatic alcohol surfactants include the groups of surfactants known as propylene glycol POE ethers (e.g. alkyl or alkenyl ethers, in particular alkyl) of the general formula:

$$R\text{-}OCH(CH_3)CH_2\text{-}(OCH_2CH_2)_nOH$$

Ethoxylated non-aromatic alcohol surfactants of particular interest are those derived from alkyl or alkenyl alcohols (typically monofunctional alcohols, e.g. primary alcohols) having between 10 and 24 carbon atoms (which is typically unbranched and may optionally contain 1 or 2 double bonds, such as 1 double bond), e.g. laureth, trideceth, myristeth, ceteth, isoceteth, steareth, isosteareth, oleth and beheneth, or mixtures such as pareth and ceteareth (in particular laureth, ceteth, isoceteth, isosteareth, oleth, C11-15 pareth, C12-13 pareth and ceteareth). A further group of ethoxylated non-aromatic alcohol surfactants of particular interest are those derived from coceth. Ethoxylated non-aromatic alcohol surfactants of use in the present invention will typically contain between 5 and 150 PEG units, suitably between 5 and 50 PEG units, especially between 5 and 40 PEG units, in particular between 8 and 30 PEG units.

One group of ethoxylated non-aromatic alcohol surfactants of use in the present invention are the laureth series having between 5 and 150 PEG units, such as between 8 and 50 PEG units, for example between 8 and 23 PEG units (those having an HLB of 13.1 or greater, such as 13.5 or greater, are of particular interest, for example those having an HLB of 13.1 to 17.5, especially 13.5 to 17.0). Exemplary laureth series ethoxylated non-aromatic alcohol surfactants of interest are those having 10 to 40 PEG units, especially 10 to 25 PEG units. Specific examples of laureth series ethoxylated non-aromatic alcohol surfactants of use in the present invention are laureth-8, laureth-10 and laureth-23 (especially laureth-10 and laureth-23).

Another specific group of ethoxylated non-aromatic alcohol surfactants of use in the present invention are the ceteth series having between 5 and 150 PEG units, such as between 10 and 50 PEG units, for example between 15 and 20 PEG units (those having an HLB of 13.0 or greater, such as 15.5 or greater, are of particular interest, for example those having an HLB of 14.0 to 17.5, especially 15.0 to 16.0). Exemplary ceteth series ethoxylated non-aromatic alcohol surfactants of interest are those having 10 to 40 PEG units, such as 10 to 24 PEG units, especially 10 to 20 PEG units. Specific examples of ceteth series ethoxylated non-aromatic alcohol surfactants of use in the present invention are ceteth-10, ceteth-15 and ceteth-20 (especially ceteth-15 and ceteth-20).

A further specific group of ethoxylated non-aromatic alcohol surfactants of use in the present invention are the oleth series having between 5 and 150 PEG units, such as between 10 and 50 PEG units, for example between 15 and 20 PEG units (those having an HLB of 12.5 or greater, such as 14.2 or greater, are of particular interest, for example those having an HLB of 13.0 to 17.0, especially 14.2 to 16.0). Exemplary oleth series ethoxylated non-aromatic alcohol surfactants of interest are those having 12 to 50 PEG units, such as 12 to 40 PEG units, especially 15 to 30 PEG units. Specific examples of oleth series ethoxylated non-aromatic alcohol surfactants of use in the present invention are oleth-15, oleth-20 and oleth-30 (especially oleth-15 and oleth-20).

Ethoxylated non-aromatic alcohol surfactants of the pareth series (e.g. C11-15 pareth, or alternatively C12-13 pareth) are also of interest, such as those having between 5 and 150 PEG units, such as between 10 and 35 PEG units, for example between 12 and 23 PEG units (those having an HLB of between 14.0 and 17.5, such as those between 14.7 and 16.7, are of particular interest). Exemplary pareth series ethoxylated non-aromatic alcohol surfactants of interest are those having 12 to 30 PEG units. Specific examples of pareth series ethoxylated non-aromatic alcohol surfactants of use in the present invention are C11-15 pareth-12, C11-15 pareth-15, C11-15 pareth-20 and C12-C13 pareth-23 (especially C11-15 pareth-15, C11-15 pareth-20 and C12-C13 pareth-23).

Another specific group of ethoxylated non-aromatic alcohol surfactants of use in the present invention are the ceteareth series having between 5 and 150 PEG units, such as between 10 and 50 PEG units, for example between 20 and 30 PEG units, especially 22 to 28 PEG units (those having an HLB between 15.5 and 17.0, such as those between 15.7 and 16.7, are of particular interest). Specific examples of ceteareth series ethoxylated non-aromatic alcohol surfactants of use in the present invention are ceteareth-20, ceteareth-25 and ceteareth-30 (especially ceteareth-25).

Other ethoxylated non-aromatic alcohol surfactants of use in the present invention include the isoceteth series having between 5 and 150 PEG units, such as between 10 and 50 PEG units, for example between 15 and 25 PEG units (those having an HLB between 14.0 and 17.0, such as those between 15.2 and 16.2, are of particular interest). A specific example of an isoceteth series ethoxylated non-aromatic alcohol surfactant of use in the present invention is isoceteth-20.

Further ethoxylated non-aromatic alcohol surfactants of use in the present invention include the isosteareth series having between 5 and 150 PEG units, such as between 10 and 50 PEG units, for example between 15 and 25 PEG units (those having an HLB between 14.0 and 17.0, such as those between 14.5 and 15.5, are of particular interest). A specific example of an isosteareth series ethoxylated non-aromatic alcohol surfactant of use in the present invention is isosteareth-20.

Another specific group of ethoxylated non-aromatic alcohol surfactants of use in the present invention are the coceth series having between 5 and 150 PEG units, such as between 5 and 50 PEG units, especially 8 to 30 PEG units, for example 10 and 20 PEG units (those having an HLB between 13.0 and 17.0, such as those between 13.5 and 16.5, especially between 14 and 16, are of particular interest). Specific examples of coceth series ethoxylated non-aromatic alcohol surfactants of use in the present invention are coceth-10 and coceth-20.

Propoxylated/ethoxylated ethers covers a number of groups of surfactants including ethoxylated PPG alkyl ethers, ethoxylated PPG ethers and propoxylated POE ethers.

Ethoxylated PPG alkyl ethers have the general formula:

$$R\text{-}(OCH(CH_3)CH_2)_m(OCH_2CH_2)_nOH$$

wherein R represents an alkyl or alkenyl chain. Typically the R group is an unbranched alkyl of 10 to 22 carbon atoms in length.

Ethoxylated PPG ethers have the general formula:

$$H(OCH_2CH_2)_m(OCH(CH_3)CH_2)_x(OCH_2CH_2)_nOH$$

Propoxylated POE ethers have the general formula:

$$H(OCH(CH_3)CH_2)_m(OCH_2CH_2)_x(OCH(CH_3)CH_2)_nOH$$

Polyglyceryl ethers can be prepared by the reaction of an alcohol (e.g. monofunctional) with polyglycerol. Suitably the polyglyceryl chain will be from 2 to 50 units in length. Suitably the alcohol is an alkyl or alkenyl alcohol (e.g. primary alcohols) having between 10 and 24 carbon atoms (which is typically unbranched and may optionally contain 1 or 2 double bonds, such as 1 double bond), e.g. laureth, trideceth, myristeth, ceteth, isoceteth, steareth, isosteareth, oleth and beheneth, or mixtures such as pareth and ceteareth (in particular laureth, ceteth, isoceteth, isosteareth, oleth, C11-15 pareth, C12-13 pareth and ceteareth). Further examples are those derived from coceth. Polyglyceryl ethers may be mono or polyethers.

Sugar ethers are a class of surfactant prepared from the derivatisation of an alcohol (e.g. a monofunctional alcohol) with mono or polysaccharides. Suitably the alcohol is a primary alcohols having between 10 and 24 carbon atoms (which is typically unbranched and may optionally contain 1 or 2 double bonds, such as 1 double bond), e.g. laureth, trideceth, myristeth, ceteth, isoceteth, steareth, isosteareth, oleth and beheneth, or mixtures such as pareth and ceteareth (in particular laureth, ceteth, isoceteth, isosteareth, oleth, C11-15 pareth, C12-13 pareth and ceteareth). Further examples are those derived from coceth. Suitably the number of sugar residues will be from 1 to 10 (e.g. 1 sugar residue). Suitably the mono or polysaccharide is a glycoside.

*Esters*

In one embodiment of the invention the surfactant is an ester surfactant.

The broad class of ester surfactants may be separated into a number of sub-classes which include:

- ethoxylated carboxylic acids
- ethoxylated glycerides
- polyglyceryl esters
- sugar esters

In one embodiment of the invention the ester surfactant is an ethoxylated carboxylic acid. In a second embodiment of the invention the ester surfactant is an ethoxylated glyceride. In a third embodiment of the invention the ester surfactant is a polyglyceryl ester. In a fourth embodiment of the invention the ester surfactant is a sugar ester.

Ethoxylated carboxylic acid surfactants are ethylene oxide derivatives of carboxylic acids, usually mono-functional primary alkyl or alkenyl acids. Ethoxylated carboxylic acid surfactants have the general formula:

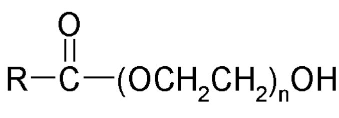

for monoacylates

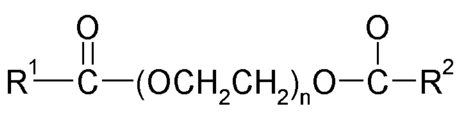

for diacylates

wherein the group R is the moiety from the original acid (in diacylates, $R^1$ and $R^2$ both typically represent the same moiety).

In respect of ethoxylated carboxylic acid surfactants, suitably the surfactant HLB will be in the range from about

12.5 to about 17.5, in particular about 13.0 to about 17.0. Ethoxylated carboxylic acid surfactants of particular interest are those derived from alkyl or alkenyl acids (typically monofunctional acids, e.g. primary acids) having between 10 and 24 carbon atoms (which is typically unbranched and may optionally contain 1 or 2 double bonds, such as 1 double bond), e.g. laurate, myristate, palmitate, stearate and oleate (in particular stearate), or mixtures thereof. Ethoxylated carboxylic acid surfactants of use in the present invention will typically contain between 5 and 150 PEG units, suitably between 5 and 50 PEG units, especially between 10 and 45 PEG units, in particular between 20 and 40 PEG units.

In one embodiment of the invention the ethoxylated carboxylic acid surfactant is substantially monoacylated. In a second embodiment of the invention the ethoxylated carboxylic acid surfactant is substantially diacylated. In a third embodiment of the invention the ethoxylated carboxylic acid surfactant is a mixture of the ethoxylated carboxylic acid surfactants having varying degrees of acylation (e.g. averaging 1.5 acyl units).

One group of ethoxylated carboxylic acid surfactants of use in the present invention are the stearate series having between 5 and 150 PEG units, such as between 10 and 50 PEG units, for example between 20 and 40 PEG units (those having an HLB between 15.5 and 17.5, such as those between 16.0 and 16.9, are of particular interest). Specific examples of stearate series ethoxylated carboxylic acid surfactants of use in the present invention are PEG-20 stearate and PEG-40 stearate.

Ethoxylated glycerides are of the general formula:

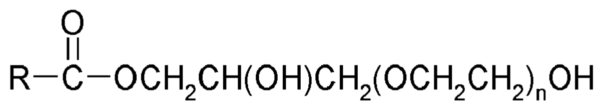

where R is the moiety from the carboxylic acid. Ethoxylated glyceride surfactants of particular interest are those derived from alkyl or alkenyl acids (typically monofunctional acids, e.g. primary acids) having between 10 and 24 carbon atoms (which is typically unbranched and may optionally contain 1 or 2 double bonds, such as 1 double bond), e.g. laurate, myristate, palmitate, stearate and oleate, or mixtures thereof. Ethoxylated glyceride surfactants of use in the present invention will typically contain between 5 and 150 PEG units, suitably between 5 and 50 PEG units, especially between 10 and 45 PEG units.

Polyglyceryl esters can be prepared by the reaction of a carboxylic acid with polyglycerol. Suitably the polyglyceryl chain will be from 2 to 50 units in length. Suitably the carboxylic acid is an alkyl or alkenyl acid (typically monofunctional acids, e.g. primary acids) having between 10 and 24 carbon atoms (which is typically unbranched and may optionally contain 1 or 2 double bonds, such as 1 double bond), e.g. laurate, myristate, palmitate, stearate and oleate, or mixtures thereof. Polyglyceryl esters may be mono or polyesters.

Sugar esters can be divided into two main groups, the sorbitan esters and the non-sorbitan esters.

Sorbitan/sorbitol esters are based around a sorbitan/sorbitol core which is derivatised by reaction with a carboxylic acid. The simplest sorbitan ester surfactants are acylated, generally being monoacylated on average, containing only the hydrophilic sorbitan ring and the hydrophobic moiety from an alkyl or alkenyl acid. Typically, the alkyl or alkenyl acid (typically monofunctional acids, e.g. primary acids) has between 10 and 24 carbon atoms (which is typically unbranched and may optionally contain 1 or 2 double bonds, such as 1 double bond), e.g. laurate, myristate, palmitate, stearate and oleate, or mixtures thereof (in particular laurate and oleate, especially laurate). Such acylated sorbitan esters generally have a very low HLB which precludes them from being of use in the present invention. However, acylated sorbitan esters can be further derivatised by ethoxylation to provide PEG sorbitan esters which are more hydrophilic and have higher HLB numbers.

PEG sorbitan esters typically contain between 5 and 150 PEG units, such as between 10 and 50 PEG units, especially 10 to 30 PEG units, in particular 15 to 25 PEG units, such as 20 PEG units (those having an HLB between 15.7 and 17.5, such as those between 16.2 and 17.2, are of particular interest. Exemplary oleate and laurate series PEG sorbitan esters of interest are those having 10 to 30 PEG units, such as 15 to 25 PEG units. A specific example of a PEG sorbitan ester of use in the present invention is polysorbate 20.

Non-sorbitan sugar esters form an analogous group to the sorbitan esters, having a sugar core (e.g. sucrose, glucose or methyl glucose, in particular sucrose or glucose, especially sucrose) which is derivatised by reaction with a carboxylic acid. Typically the carboxylic acid is an alkyl or alkenyl acid (typically monofunctional acids, e.g. primary acids) having between 6 and 22 carbon atoms (which is typically unbranched and may optionally contain 1 or 2 double bonds, such as 1 double bond), e.g. octanoate, decanoate, laurate, myristate, palmitate, stearate and oleate (in particular decanoate, laurate and myristate), or mixtures thereof. Sugar ester surfactants may be mono or polyacylated (or a mixture of such), typically those monoacylated or diacylated on average are of particular interest, especially monoacylated. Specific examples of sugar ester surfactants of use in the present invention include sucrose laurate, sucrose myristate and decyl glucoside.

Non-sorbitan sugar esters can be further derivatised to provide PEG non-sorbitan sugar ester surfactants,

typically containing between 5 and 150 PEG units, such as between 10 and 50 PEG units.

Suitably, when the surfactant is a sugar ester, the sugar ester is a PEG sorbitan ester or a non-sorbitan sugar ester.

*Ionic surfactants*

Ionic surfactants are a further broad class of surface active agents which may be used in the present invention.

Ionic surfactants include:

- cationic surfactants
- anionic surfactants
- amphoteric surfactants

Cationic surfactants are those having a positive charge in aqueous solution at neutral pH. One series of cationic surfactants of particular interest is the PEG alkyl amines.

PEG alkyl amines have the following general structure:

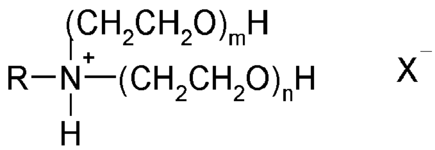

wherein R is typically an alkyl or alkenyl group ($X^-$ is a counter anion (typically a halide, such as chloride). PEG alkyl amines of particular interest have between 6 and 22 carbon atoms (which is typically unbranched and may optionally contain 1 or 2 double bonds, such as 1 double bond), e.g. being derived from decylamine, laurylamine, myristylamine, cetylamine, stearylamine and oleylamine, or mixtures such as cocamine. The total number of PEG units (i.e. m+n) typically being from 2 to 50, such as 2 to 30, in particular 2 to 15. Exemplary cocamine series PEG alkyl amines of interest are those having 2 to 30 PEG units, such as 2 to 25 PEG units, for example 5 to 10 PEG units. Specific examples of PEG alkyl amines of use in the present invention include PEG-5 cocamine and PEG-15 cocamine.

Anionic surfactants are those having a negative charge in aqueous solution at neutral pH. Anionic surfactants include, for example, the alkyl and alkenyl acids, amino acid amides, esters of alpha-hydroxycarboxylic acids and a range of other materials such as sulphate or phosphate based surfactants. Alkyl and alkenyl acids may be typically expected to have insufficient hydrophilicity for use in the present invention. Anionic surfactants of the amino acid amide group are of particular interest.

Anionic amino acid amide surfactants are amino acids (i.e. non-basic amino acids) which have been acylated by reaction with a carboxylic acid. Suitably the amino acid is glutamic acid or glycine, although a number of commercial surfactants are available based on plant derived mixtures of amino acids (e.g. wheat and oat). Typically the carboxylic acid is an alkyl or alkenyl acid (typically monofunctional acids, e.g. primary acids) having between 6 and 22 carbon atoms

(which is typically unbranched and may optionally contain 1 or 2 double bonds, such as 1 double bond), e.g. lauroyl and stearoyl, or mixtures such as cocoyl (in particular lauroyl and cocoyl). Specific examples of amino acid amide surfactants of use in the present invention include sodium lauroyl glutamate, sodium cocoyl glycinate, sodium cocoyl methyl taurate, sodium cocoyl glutamate, disodium cocoyl glutamate, sodium lauryl wheat amino acids, potassium lauryl wheat amino acids, sodium lauryl oat amino acids and sodium cocoyl apple amino acids (especially sodium lauroyl glutamate, sodium cocoyl glycinate, sodium cocoyl glutamate, potassium lauryl wheat amino acids and sodium lauryl oat amino acids).

Another anionic amino acid derived surfactant is surfactin (Aminofect).

Esters of alpha-hydroxycarboxylic acids are materials wherein the hydroxyl function of an alpha-hydroxycarboxylic acid (e.g. lactic acid) is esterified with a carboxylic acid, typically the carboxylic acid is an alkyl or alkenyl acid (typically monofunctional acids, e.g. primary acids) having between 6 and 22 carbon atoms (which is typically unbranched and may optionally contain 1 or 2 double bonds, such as 1 double bond), e.g. lauryl. Such materials generally have relatively low HLB values, therefore would not typically be expected to be of use in the present invention.

Phosphate based surfactants include groups such as the alkyl and alkenyl phosphates (e.g. cetyl phosphate and such like). Other phosphate based surfactants are the PPG ethoxylated alkyl phosphates (e.g. PPG-5 ceteth-10 phosphate), wherein the number of PPG units will typically vary from 2 to 20, the number of PEG units typically vary

from 5 to 50 and the aliphatic ether will be derived from an alkyl or alkenyl alcohol (typically monofunctional alcohols, e.g. primary alcohols) having between 10 and 24 carbon atoms (which is typically unbranched and may optionally contain 1 or 2 double bonds, such as 1 double bond) such as ceteth.

Sulphate based surfactants include sodium cholate and sodium deoxycholate. Another sulphate based surfactant is sodium lauryl sulphate. Sulphate based surfactants such as sodium cholate, sodium deoxycholate and sodium lauryl sulphate are highly potent surfactants and are recognised as irritants.

Zwiterionic or amphoteric surfactants are those having a positive and a negative charge in aqueous solution at neutral pH. Amphoteric surfactants include amino acid amide surfactants wherein the amino acid is a basic amino acid and which has been acylated by reaction with a carboxylic acid. Typically the carboxylic acid is an alkyl or alkenyl acid (typically monofunctional acids, e.g. primary acids) having between 6 and 22 carbon atoms (which is typically unbranched and may optionally contain 1 or 2 double bonds, such as 1 double bond).

Other amphoteric surfactants include materials such as cocamidopropyl betaine, wherein a betaine hydrophile is attached to a hydrophobic chain which incorporates an amide linkage.

Amphoteric polymeric surfactants include amphipol A8-35 (see Gohon Y et al Analytical Biochemistry 2004 334:318-334; Pocanschi CL et al Biochemistry 2006 45:13954-13961).

*Other surfactants*

In addition to the surfactants outlined above, it will be is clear to those skilled in the art that polymers or copolymers with a suitable balance of hydrophilic/lipophilic blocks (i.e. having a suitable HLB value) could also be envisaged which would be suitable for use as surfactants in the present invention.

Non-biodegradable polymeric surfactants which may be of use in the present invention include non-alternating co-polymers of hydrolysed maleic anhydride and alkyl vinyl ethers in which the ratio of monomer units is such that the polymer has the correct HLB value by virtue of the charge on the carboxylic acid groups under the conditions of use (e.g. pH between 5.5-8.5) and the proportion (e.g. about 2:1, 3:1 or 4:1, based on an excess of hydrophobic groups) and type of hydrophobic groups present (e.g. propyl or butyl).

Biodegradable polymeric surfactants include polyester co-polymers of mandelic and malic acid in which the ratio of monomer units is such that the polymer has the correct HLB value by virtue of the charge on the carboxylic acid groups on the malic acid units under the conditions of use (e.g. pH between 5.5-8.5) and the proportion (e.g. about 2: 1, 3:1 or 4:1, based on an excess of hydrophobic groups) of the hydrophobic groups provided by the mandelic acid units.

*Particularly suitable surfactants*

Suitably the surfactant will be an ethoxylated alcohol ether surfactant, an ethoxylated carboxylic acid surfactant, a sugar ester surfactant, a PEG alkyl amine surfactant, anionic amino acid amide surfactant or surfactin.

Specific examples of surfactants of use in the present invention include octoxynol-12, nonoxynol-15, octoxynol-16, nonoxynol-20, laureth-8, laureth-10, laureth 23, ceteth-10, ceteth-15, ceteth-20, oleth-15, oleth-20, C11-15 pareth-12, C11-15 pareth-15, C11-15 pareth-20, C11-15 pareth-20, C12-C13 pareth-23, ceteareth-20, ceteareth-25, ceteareth-30, isoceteth-20, isosteareth-20, PEG-20 stearate, PEG-40 stearate, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, disodium cocoyl glutamate, potassium lauryl wheat amino acids, sodium lauryl oat amino acids, sodium lauryl wheat amino acids, sodium cocoyl apple amino acids, sodium cocoyl methyl taurate and surfactin; especially octoxynol-12, nonoxynol-15, octoxynol-16, nonoxynol-20, laureth-10, laureth 23, ceteth-10, ceteth-15, ceteth-20, oleth-15, oleth-20, C11-15 pareth-12, C11-15 pareth-15, C11-15 pareth-20, C11-15 pareth-20, C12-C13 pareth-23, ceteareth-20, ceteareth-25, isoceteth-20, isosteareth-20, PEG-20 stearate, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, disodium cocoyl glutamate, potassium lauryl wheat amino acids, sodium lauryl oat amino acids, sodium lauryl wheat amino acids, sodium cocoyl apple amino acids, sodium cocoyl methyl taurate and surfactin; in particular octoxynol-12, nonoxynol-15, octoxynol-16, nonoxynol-20, laureth-10, laureth 23, ceteth-10, ceteth-15, ceteth-20, oleth-15, oleth-20, C11-15 pareth-15, C11-15 pareth-20, C11-15 pareth-20, C12-C13 pareth-23, ceteareth-25, isoceteth-20, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, potassium lauryl wheat amino acids, sodium lauryl wheat amino acids, sodium lauryl oat amino acids and surfactin. Additional examples include coceth-10 and coceth-20.

The suitability of a particular surfactant or surfactant mixture for use in the present invention may be determined by those skilled in the art by routine experimentation based on the guidance provided herein.

**Lipid**

The term lipid is well known in the art. The lipid of use in the present invention will typically be selected from phospholipids, ceramides, sphingomyleins, phosphatidic acids, cardiolipins, lysophospholipids, plasmalogens, phosphosphingolipids and mixtures thereof.

Phospholipids (for example phosphatidylcholine, phosphatidylethanolamine, phosphatidylglycerol, phosphatidylinositol, phosphatidylserine and mixtures thereof) have a polar head group (which in a membrane aligns towards the aqueous phase) and two hydrophobic tail groups (which in a bilayer membrane associate to form a hydrophobic core). The hydrophobic tail groups will typically be in the form of acyl esters, which may vary both in their length (for example from 8 to 26 carbon atoms, especially 10 to 20 carbon atoms) and their degree of unsaturation (for example one, two or three double bonds, especially one double bond). Generally, the two hydrophobic tail groups are identical, though they need not be so.

Lipids of use in the present invention may be of natural or synthetic origin, and may be: a single pure component (e.g. at least 80% pure, especially at least 90% pure, in particular at least 95% pure and suitably at least 99% pure on a weight basis); a single class of lipid components (for example a mixture of phosphatidylcholines, or alternatively, a mixture of lipids with a conserved acyl chain type) or may be a mixture of many different lipid types.

In one embodiment of the invention the lipid is a single pure component.

Pure lipids are generally of synthetic or semi-synthetic origin. Examples of pure lipids of use in the present invention include pure phosphatidylcholines (for example, DMPC, DLPC, DPPC and DSPC, in particular DLPC and DPPC, especially DLPC) and phosphatidylglycerols (for example DPPG), suitably phosphatidylcholines. The use of pure lipids is desirable due to their clearly defined composition, however, they are generally prohibitively expensive for many commercial applications.

In a second embodiment of the invention the lipid is a mixture of components.

Mixtures of lipids of use in the present invention may be of natural origin, obtained by extraction and purification by means known to those skilled in the art. Lipid mixtures of natural origin are generally significantly cheaper than pure synthetic lipids. Naturally derived lipids include lipid extracts from egg or soy, which extracts will generally contain lipids with a mixture of acyl chain lengths, degrees of unsaturation and headgroup types. Lipid extracts of plant origin may typically be expected to demonstrate higher levels of unsaturation than those of animal origin. It should be noted that, due to variation in the source, the composition of lipid extracts may vary from batch to batch. Hydrogenated lipids are less prone to peroxidation due to the absence of unsaturation, typically have less coloration and have lower odour.

Lipid mixtures may also be prepared by the combination of pure lipids, or by the combination of one lipid extract with either other lipid extracts or with pure lipids. The preparation of lipid mixtures by the combination of lipid extracts and/or pure lipids is of particular relevance to compositions for use in the analysis of membrane proteins/peptides and their interactions with other agents, wherein it is highly desirable to control the lipid constituents such that the natural environment is closely mimicked.

Suitably, a lipid extract of use in the present invention will comprise at least 50% phospholipids by weight (for example, phosphatidylcholines and phosphatidylethanolamines), especially at least 55% phospholipids by weight, in particular at least 60% phospholipids by weight (such as 75% or 90%).

In one embodiment of the invention the lipid mixture is a lipid extract containing at least 50%, such as at least 60%, especially at least 75% and suitably at least 90% by weight of phospholipids of a single headgroup type (e.g. phosphatidylcholines). In a second embodiment of the invention particular lipid extracts may be of particular interest due to their relatively cheap cost. In a third embodiment of the invention lipid extracts of particular interest are those which result in solutions of highest clarity. In a fourth embodiment of the invention the lipid is a lipid mixture having a conserved acyl chain length (e.g. at least 50%, such as at least 60%, especially at least 75% and suitably at least 90% by weight), for example 12 (e.g. lauryl), 14 (e.g. myristyl), 16 (e.g. palmityl) or 18 (e.g. stearyl or alternatively oleyl) carbons atoms in length, in particular 12-16 carbon atoms (such acyl chains optionally having one, two or three double bonds, though suitably being fully saturated). In another embodiment of the invention the lipid is a lipid mixture which is hydrogenated (i.e. the acyl chains are fully saturated). In a further embodiment of the invention the lipid mixture is a lipid extract of plant origin (e.g. soy).

In another embodiment of the invention the lipid mixture is a lipid extract of animal origin (e.g. egg).

Exemplary lipid extracts of use in the present invention include: Epikuron 200, Epikuron 145V, Epikuron 130P, Emulmetik 950, Emulmetik 900 and Emulmetik 300 available from Degussa Texturant Systems UK Ltd; S 75, S 100, S PC and SL 80 available from Lipoid GmbH; Phospholipon® 90 H, Phospholipon® 80 H, and Phospholipon® 90 NG available from Phospolipid GmbH; EMULTOP® IP and EMULPUR® IP available from Lucas Meyer (Degussa Texturant Systems UK Ltd).

One suitable lipid extract is derived from soy and comprises: at least 92% phosphatidyl cholines, a maximum of 3% lyso-phosphatidyl cholines and a maximum of 2% oils; of which 14-20% of the acyl chains are palmityl, 3-5% stearyl, 8-12% oleic, 62-66% linoleic and 6-8% linolenic. A second suitable lipid extract is derived from soy and comprises:

at least 90% hydrogenated phosphatidyl cholines, a maximum of 4% hydrogenated lyso-phosphatidyl cholines and a maximum of 2% oils and triglycerides; of which at least 80% of the acyl chains are stearyl and at least 10% are palmityl.

The lipid, or lipid mixture, of use in the present invention will typically be membrane forming.

Those skilled in the art will recognise that lipid mixtures of use in the invention may comprise non-membrane forming lipid components (e.g. cholesterol). In some circumstances lipid mixtures of use in the invention may be a mixture of only non-membrane forming lipids which in combination demonstrate membrane forming ability.

For cosmetic and pharmaceutical applications typically the lipid (for example the pure lipid or the lipid mixture) is one which has been approved for use in cosmetic and/or pharmaceutical applications as appropriate.

Suitably the lipid is a pure lipid, a plant derived lipid extract or an egg derived lipid extract (especially a pure lipid or a plant derived lipid extract).

The suitability of a particular pure lipid or lipid mixture for use in the present invention may be determined by those skilled in the art by routine experimentation based on the guidance provided herein.

**Macromolecular assemblies**

The presence of a macromolecular assembly (an association of individual surfactant and lipid molecules within a macromolecular structure which is not maintained by covalent bonding), also referred to herein as a macromolecular complex, may be confirmed by a number of means available to those skilled in the art for the determination of particle size, for example, electron microscopy (such as used in Tonge, SR and Tighe, BJ Advanced Drug Delivery Reviews 2001 53:109-122 for macromolecular assemblies incorporating alternating styrene/maleic acid copolymers), laser diffraction techniques and such like. A particularly suitable method for the determination of particle size is dynamic light scattering, with instrumentation available from Malvern Instruments, UK (e.g. Malvern Zetasizer Nano ZS).

As shown in the Examples, compositions according to the present invention offer an alternative solubilisation system to those previously described in the art. Without being limited by theory, it is believed that the macromolecular assemblies of the present invention are bilayer discs (as opposed to thread/tube-like micelles or conventional mixed micelles) the bilayer discs being a stable intermediate state between vesicles and mixed micelles. The surfactants of use in the present invention are believed to act as 'lipid chaperones', arranging the lipid bilayers into nanostructured assemblies of a defined size.

Although the precise structure of the macromolecular assemblies of the present invention is of academic interest, it is the surprising beneficial properties exhibited by compositions of the invention which are of more general interest (e.g. the high solubilising capability for hydrophobic agents, resulting from the synergistic interaction of the surfactant and lipid component present). Unexpectedly, high solubilisation levels are achievable using compositions of the present invention with mild surfactants (e.g. ethoxylated alcohol surfactants, sugar ester surfactants, anionic amino acid amide surfactants and the like) which are comparable to those achievable using potent irritating surfactants such as SDS (e.g. surfactants having an HLB of greater than 17.6, especially those having an HLB of 18 or greater, in particular those having an HLB of 20 or greater are considered herein to generally be irritants). It is contrary to the expectations of one skilled in the art that the ability of a surfactant to solubilise an active agent which is poorly water solubile may be *increased* by the addition of a lipid.

The macromolecular assemblies of the present invention will typically be of less than 100 nm in diameter, such as less than 75 nm in diameter, especially less than 50 nm in diameter, such as less than 30 nm in diameter (e.g. less than 20 nm). The diameter of macromolecular assemblies of the present invention may readily be determined by means known to those skilled in the art. Suitably, at least 50%, such as at least 60%, especially at least 70%, in particular at least 80% and most suitably at least 90% (such as at least 95%) of the macromolecular assemblies have the specified diameter. Suitably, the macromolecular assemblies of the present invention will be of at least 5 nm in diameter, such as at least 6 nm in diameter, especially at least 7 nm in diameter, in particular at least 8 nm in diameter (e.g. at least 9 nm, or at least 10 nm). Suitably the the macromolecular assemblies of the present invention will be of 6-75 nm in diameter, in particular 7-60 nm in diameter, such as 8-50 nm in diameter.

It will be understood that the above typical dimensions refer to macromolecular assemblies in the presence or absence of active agent (i.e. whether the macromolecular assemblies have active agent within them or not).

Those skilled in the art will understand that the term diameter can be applied to non-spherical particles. For bilayer discs the term diameter refers to the disc diameter. For thread/tube-like micelles the term diameter applies to the 'effective diameter' when the sizing technique applied is unable to distinguish between different morphologies (see for example Walter et al Biophysics Journal 1991 60:1315-1325, where tube-like micelles of ca. 100 to 300 nm in length and 3 to 5 nm in diameter, are said to compare well to an 'effective particle size' of around 16 nm). In one embodiment of the invention the particle size is determined by laser diffraction. In a second embodiment of the invention the particle size is determined by electron microscopy. In a third embodiment of the invention the particle size is determined by neutron scattering.

When the macromolecular assembly particle size is determined by laser diffraction (e.g. by dynamic light

scattering), suitably it will be performed using a Malvern Zetasizer. In such cases the principal particle size detected in compositions of the invention will typically be of less than 100 nm in diameter, such as less than 75 nm in diameter, especially less than 50 nm in diameter, such as less than 30 nm in diameter (e.g. less than 20 nm). Suitably, the principal particle size will be of at least 5 nm in diameter, such as at least 6 nm in diameter, especially at least 7 nm in diameter, in particular at least 8 nm in diameter (e.g. at least 9 nm, or at least 10 nm). Suitably, the intensity of the principal particle size will be at least 50%, such as at least 60%, especially at least 70%, in particular at least 80% and most suitably at least 90% (such as at least 95%). The polydispersity index will suitably be less than 0.7, especially less than 0.6, in particular less than 0.5, such as less than 0.4. Suitably the the principal particle size will be will be of 6-75 nm in diameter, in particular 7-60 nm in diameter, such as 8-50 nm in diameter.

*Clarity and stability*

Clarity provides a convenient and ready means for determining that a solution contains particles generally having a small size and a low size dispersion. Changes in clarity over time can provide an indication of particle size instability.

The clarity of a solution may be determined by methods known to those skilled in the art, for example, through the use of a turbidity meter, such as those provided by Orbeco-Helling or Hach-Lange. Turbidity may be based on a number of standard units, such as nephelometric turbidity units (NTU), which are directly interchangeable with formazin nephelometric units (FNU).

By the term "clear", when used herein in respect of solutions, is meant a solution with a turbidity reading of less than 150 FNU, especially less than 100 FNU, in particular less than 75 FNU, suitably less than 50 FNU, more suitably less than 25 FNU (e.g. less than 15 FNU, such as less than 5 FNU). A clarity of less than 75 FNU will typically be indicative of a particle size of less than 100 nm.

Colourless solutions are those that transmit light without absorbance of any particular visible wavelength. Clear solutions may be coloured where they contain a component which absorbs light within the visible range (e.g. certain active agents, or colorants).

The terms "stable", and where appropriate "stability", when used herein in a solution, refer to the ability of a solution to remain at a constant clarity or to remain within a chosen clarity limit as may be required for a particular use.

In one embodiment of the invention the clarity of a stable solution will remain substantially unchanged (for example, changing by less than 100 FNU, especially less than 50 FNU, in particular less than 25 FNU and suitably less than 5 FNU) over a period of time (for example, at least one hour, such as at least one day, especially at least one week, in particular at least one month and suitably at least six months) when stored at constant temperature (for example, at 4 °C, suitably at 25°C).

In a second embodiment of the invention the clarity of a stable solution, although showing some degree of variation over a given time period, remains within a desired turbidity limit. In this case, typically the solution will maintain a turbidity reading of less than 150 FNU, especially less than 100 FNU, in particular less than 75 FNU, suitably less than 50 FNU, more suitably less than 25 FNU (e.g. less than 15 FNU, such as less than 5 FNU) over a period of time (for example, at least one hour, such as at least one day, especially at least one week, in particular at least one month and suitably at least six months) when stored at constant temperature (for example, at 4°C, suitably at 25°C).

In respect of compositions of the invention which are absent of water, suitably these may be reconstituted into water to form clear and stable solutions at a dry weight of around 0.1-10% (based on the surfactant, lipid and active components), such as about 4%.

One skilled in the art will recognise that stability may also be determined in respect of the consistency of particle size. As such, a stable solution is one in which: the particle size remains within a defined size limit as may be required for a particular use, for example: the principal particle size detected will remain less than 100 nm in diameter, such as less than 75 nm in diameter, especially less than 50 nm in diameter, such as less than 30 nm in diameter (e.g. less than 20 nm); the intensity of the principal particle size will consistently be at least 50%, such as at least 60%, especially at least 70%, in particular at least 80% and most suitably at least 90% (such as at least 95%); and the polydispersity index will suitably remain less than 0.7, especially less than 0.6, in particular less than 0.5, such as less than 0.4. over a period of time (for example, at least one hour, such as at least one day, especially at least one week, in particular at least one month and suitably at least six months) when stored at constant temperature (for example, at 4 °C, suitably at 25 °C).

*Surfactant/lipid ratios*

Insufficient quantities of surfactant may result in solutions with sub-optimal clarity, due to the presence of larger particles which disrupt the passage of light. Typically, the ratio of surfactant to lipid in the compositions of the present invention will be at least 0.5:1 on a weight basis (e.g. at least 0.75:1), especially at least 1:1, suitably at least 1.25:1, more suitably at least 1.5:1 (for example at least 2.0:1, such as about 2.5:1).

Excess quantities of surfactant may not provide substantial benefit (such as with respect to clarity) and their use may therefore be unnecessarily wasteful and undesirable in pharmaceutical (or cosmetic) applications were large amounts of surfactant may be irritating. Suitably the ratio of surfactant to lipid in the compositions of the present invention will be 10:1 1 or lower on a weight basis, especially 7:1 or lower, in particular 5:1 or lower, such as 3.5:1 or lower (e.g. 3:1 or lower).
Suitably the surfactant to lipid ratio will be in the range 10:1 1 to 1:1, especially in the range 10:1 1 to 1.25:1, in particular 10:1 to 1.5:1 (e.g. 10:1 to 2:1).
The precise minimum ratio of surfactant to lipid which provides solutions of a desired clarity level may vary to some degree between different surfactant/lipid combinations. Suitably, the ratio of surfactant to lipid will be sufficient to provide a solution of less than 150 FNU, especially less than 100 FNU, in particular less than 50 FNU (for example less than 25 FNU).
The presence of a co-surfactant and/or active agent (also the identity and the actual quantity thereof present) may also impact the ratio of surfactant to lipid necessary to obtain a desired clarity level.

*Co-surfactant*

The presence of a small quantity of co-surfactant material may enhance the ability of the main surfactant to solubilise lipid (in particular lipid mixtures). This co-surfactant can take the form of a low molecular weight material, such as lyso (i.e. monoacylated) phospholipids, including the naturally occurring lyso-phospatidyl choline (lyso-PC) which is available under the tradename S LPC from Lipoid GmbH. Alternatively, the co-surfactant may be in the form of a polymeric surfactant material, such as the synthetic block copolymer polyoxyethylene/polyoxypropylene known as a poloxamer and supplied by BASF Corporation (e.g. the specific grade known under the tradename Lutrol® F127). The co-surfactant may also be a combination of more than one surfactant. The co-surfactant will typically have a high HLB (e.g. 18-20) relative to the main surfactant.
Suitably, co-surfactant is added in an amount equivalent to between 0.1-5% of the weight of lipid in the composition, especially 0.5-2.5% and in particular 0.75-1.5% (for example about 1%).
In one embodiment of the invention the co-surfactant is a block copolymer of polyoxyethylene/polyoxypropylene (for example having a molecular weight of 5000 to 15000 Da, in particular 10000 to 13000 Da, such as around 12700 Da as is found in Lutrol® F127). In a second embodiment of the invention the co-surfactant is lyso-PC.
It may be noted that certain lipid extracts may already contain lyso-PC, however, this does not preclude the addition of a co-surfactant (although high lyso-PC lipids may not benefit from the addition of co-surfactant to the same extent as low lyso-PC lipids).
Lyso-PC as co-surfactant may be added either in its pure form (e.g. S LPC from Lipoid GmbH), or as one component of a lipid mixture (e.g. a high lyso-PC content lecithin, such as those having at least 10% lyso-PC content by weight, especially at least 15% lyso-PC by weight). An exemplary high lyso-PC content lecithin is SL 80-3 from Lipoid GmbH. The addition of lyso-PC co-surfactant as a component of a high lyso-PC content lipid mixture is desirable due to the relatively high cost of the pure material.

*Physical form*

The compositions of the present invention may be in the form of an aqueous solution, especially a clear aqueous solution (e.g. a stable clear aqueous solution), suitably a clear and colourless aqueous solution (e.g. a stable clear and colourless aqueous solution). However, for ease of transportation and handling, once prepared, the compositions may be dried (e.g. by freeze-drying, rotary evaporation or such like) to form a solid which has the benefits of being lower in both volume and weight.
In one embodiment of the present invention the composition is in the form of an aqueous solution. Aqueous solutions include aqueous semi-solids, such as gels. In a further embodiment of the present invention the composition is in dried form (for example as a powder, resin or flake). Suitably compositions of the invention in dried form can be reconstituted into aqueous solution to provide aqueous solutions.
Suitably an aqueous solution of the compositions of the present invention will contain at least 60% water by weight, such as at least 70%, especially at least 80%, in particular at least 90% (e.g. at least 95%, or at least 99%).
Suitably dried compositions of the present invention will be substantially free of water, for example containing less than 5% water by weight, especially less than 2.5%, in particular less than 1.0%, such as less than 0.25%.
Aqueous solutions of compositions according to the present invention may be prepared at relatively high concentrations, for example concentrations in excess of 30% by total weight have been prepared from reconstituted freeze-dried compositions containing the active agent TECA. High concentration aqueous compositions may demonstrate an increased viscosity. In one embodiment of the invention there is provided an aqueous solution comprising more than 0.001 and less than 10% by weight of the compositions of the invention, such as less than 5% or less than 2.5% (the

percentage being determined by the dry weight of composition of the invention relative to the total weight of composition with water). In a second embodiment of the invention there is provided an aqueous solution comprising 10-20% by weight of the compositions of the invention. In a third embodiment of the invention there is provided an aqueous solution comprising greater than 20% by weight of the compositions of the invention, such as up to 30% by weight.

Exemplary aqueous solutions of compositions of the invention may comprise 0.001-1 %, 1-15% (e.g. 1-2.5%, 2.5-5%, 5-10% or 10-15%) or 15-25% (e.g. 15-20% or 20-25%) active by weight of the compositions of the invention (i.e. the total weight of surfactant, lipid, co-surfactant and active agent).

*Manufacture*

Methods for the production of compositions according to the present invention are described in the Examples.

Compositions of the present invention may suitably be prepared by mixing an aqueous solution of a surfactant with an aqueous emulsion containing lipid (suitably at elevated temperature, e.g. approximately 50°C).

The surfactant solution may be prepared by dissolving the surfactant in water, optionally with stirring and heating (for example to approximately 50 °C). The lipid emulsion may be prepared by mixing dried lipid with water, suitably with stirring and heating (suitably to a temperature above the phase transition temperature of the lipid component, for example approximately 50 °C), followed by homogenisation. Suitably the surfactant solution and lipid emulsion are mixed by the addition (e.g. the slow addition) of lipid emulsion to the surfactant solution.

Should the properties of the surfactant be pH dependent, the pH of solutions may be adjusted using acids or bases as appropriate. Compositions for use in the fields of cosmetics or pharmaceuticals will typically utilise acids and/or bases which are physiologically acceptable. Physiologically acceptable acids include hydrochloric acid. Physiologically acceptable bases include sodium or potassium hydroxide.

Co-surfactant, in particular when present as a component of a high lyso-PC lipid extract, will typically be mixed to form a fine aqueous emulsion prior to the addition of the lipid component. The resultant emulsion is then added to the aqueous surfactant solution. When added as a pure co-surfactant it will typically be combined with the surfactant prior to the formation of the aqueous solution thereof.

In a further aspect of the present invention there is provided a method for the production of a composition comprising lipid and surfactant wherein the surfactant and lipid are in the form of macromolecular assemblies, comprising the steps of:

(i) Preparing an aqueous solution of surfactant;
(ii) Preparing an aqueous lipid emulsion; and
(iii) Mixing the aqueous lipid emulsion and aqueous solution of surfactant;
such that macromolecular assemblies are formed.

Optionally, co-surfactant is included in the aqueous solution of (i) or the aqueous emulsion (ii).

If desirable, a further optional step of removing the water may be performed to provide dried compositions of the present invention.

Compositions of the present invention in the form of an aqueous solution may be dried (e.g. by freeze-drying, alternatively by rotary evaporation) to produce compositions of the present invention in dry form. Dried compositions of the invention may be readily reconstituted into aqueous solution by the addition of water with stirring and suitably with warming.

There is also provided a method for the production of a composition comprising lipid and surfactant wherein the surfactant and lipid are in the form of macromolecular assemblies, comprising the steps of mixing the surfactant and lipid in a short chain alcohol (e.g. ethanol or isopropanol, especially isopropanol), suitability at elevated temperature (around 50 °C), and subsequently removing the alcohol (e.g. by rotary evaporation) such that dried macromolecular assemblies are formed. Aqueous solutions of the compositions may then be prepared by solubilisation in water, suitably with warming. Co-surfactant, if present, will be mixed with the surfactant and lipid in the alcohol before the composition is dried.

**Formulations**

One use of the compositions of the present invention is as a solubilising agent.

Solubilising agents may be of use as formulating aids, solubilising active agents which have poor aqueous solubility (for example aqueous solubility of less than 1% w/w, suitably less than 0.1 % w/w, such as less than 0.01% w/w or 0.001% w/w at pH 7 and room temperature, such as 22 °C).

By the term 'active agent' is meant a material having a desirable cosmetic or therapeutic activity. In one embodiment of the present invention the active agent is a cosmetic agent. In a second embodiment of the invention the

active agent is a pharmaceutical agent. Alternatively the active agent is one with organoleptic activity (e.g. a flavour or fragrance).

Active agents having poor aqueous solubility include the oil-soluble vitamins (including vitamins A, D, E and K) and oil soluble derivatives of water soluble vitamins (including vitamin C), which materials are frequently applied to the skin as part of water-in-oil or oil-in-water emulsions as antioxidants, depigmenting agents, moisturisers, collagen stimulators, anti-aging, anti-wrinkle, anti-inflammatory, anti-psoriatic and anti-fragility agents.

The vitamin A family includes retinol, retinol palmitate, retinol acetate, and related retinoids, and also pro-vitamin A, such as β-carotene. Oil-soluble derivatives of vitamin C include ascorbyl palmitate, ascorbyl dipalmitate and ascorbyl tetraisopalmitate (in particular ascorbyl palmitate and ascorbyl dipalmitate). Vitamin D and its derivatives include cholecalciferol/calcitriol (vitamin $D_3$), calcipotriol and tacalcitol (in particular cholecalciferol), which may be used in the treatment of psoriasis. Vitamin K series, including $K_1$ (phytonadione), may be used in the treatment of bruised skin and in the repair of capillary damage. 7-dehydrocholesterol is a pre-cursor for vitamin D. Another oil soluble vitamin is vitamin E.

A large number of active agents demonstrating a poor aqueous solubility are based around a triterpenoid or steroidal nucleus. Many of these agents have potent biological activity and are widely used in cosmetics and pharmaceuticals.

Oil-soluble actives based upon a triterpenoid structure include natural extracts (for example from *Centella asiatica* (Hydrocotyl), such as TECA, asiaticoside, asiatic acid and madecassic acid (in particular TECA, alternatively asiaticoside), which are of use in regulating and activating collagen synthesis; or liquorice (*Glycyrrhiza glabra*) extracts such as glabridin (e.g. PT-40), which is of use as an anti-tyrosinase and anti-microbial, and licochalcone A, which is of use as an inhibitor of 5-alpha-reductase and as an anti-microbial. Additional triterpenoid actives include extracts from *Aesculus* (Horse chestnut), including escin and also the coumarin esculoside (esculin). Further triterpenoid actives include extracts from *Ruscus* (Butcher's broom), including ruscogenin and neuroruscogenin. Extracts of *Boswellia* (Frankincense) including Boswellin CG® from Sabinsa Corporation USA are also examples of actives in this class. Stearyl glycrrhetinate which is of use as an anti-inflammatory. *Glycyrrhiza inflata* extracts such as licochalcone A (e.g. as P-U) which is of use as an inhibitor of 5-alpha-reductase and as an anti-microbial. Polyphenol-containing extracts derived from *Curcuma longa,* including tetrahydrocurcuminoids, are of use as anti-inflammatory agents.

Oil soluble rubrefacients, cooling actives and venoprotective agents can also be incorporated into the complexes of the invention. Example agents to increase skin blood flow include; benzyl and hexyl nicotinate, and capsaicin; actives to induce skin cooling include menthyl PCA (Questice CQ U/A, Quest International (UK)); while a combination of escin/lecithin (Edemine, Vama FarmaCosmetica Srl (Italy)) can be used as a venoprotectant and act to treat spider veins.

Other oil-soluble actives based upon a steroidal structure include those used to treat inflammatory conditions (such as hydrocortisone, clobetasone butyrate, betamethasone valerate, hydrocortisone butyrate, clobetasol propionate, fluticasone propionate and dexamethasone) and hormones (such as testosterone, progesterone and oestrogens). Additional steroidal compounds include dexamethasone acetate anhydride, hydrocortisone acetate and cortisone acetate. Steroidal like compounds include cholesterol and cholesterol sulphate which may, for example, be used in moisturising (cholesterol, when present, is considered to be present as part of the lipid component). Actives based upon a steroidal structure which are of particular interest are hydrocortisone, betamethasone valerate, hydrocortisone butyrate, clobetasol propionate. Also of interest is progesterone.

Examples of non-steroidal anti-inflammatories include ketoprofen, diclofenacand naproxen.

Other active agents include soy isoflavones; liquorice extracts, such as Licorice CG® from Sabinsa Corporation USA, P-U and PT-40 from Maruzen Pharmaceuticals Co. Ltd. Japan.

Endogenous skin lipids, including ceramides (e.g. ceramide IIIa) have poor aqueous solubility and are of use as skin moisturisers and whitening agents. Other ceramides include ceramide IIIb and synthetic ceramides, such as ceramide HO3 from Sederma (France). Ceramides, when present, are considered to be present as part of the lipid component.

Other relatively oil-soluble actives include lawsone (2-hydroxy-1,4-naphthoquinone), natural henna extract of *Lawsonia alba* and minoxidil, which agents may act on the hair.

Antimicrobial active agents include: anti-bacterials, such as erythromycin, neomycin (e.g. as the sulphate); anti-fungals, such as ciclopirox olamine, piroctone olamine, clotrimazole, econazole (as the nitrate), ketaconazole and nystatin (e.g. clotrimazole, ketaconazole and nystatin).

Oil-soluble derivatives of active agents which have a peptide structure include Matrixyl™ (palmitoyl-KTTKS, which downregulates collagenase and therefore increases collagen production) and Argireline® (acetyl hexapeptide-3, which inhibits acetylcholine binding, decreasing the strength of neuromuscular signals and thus decreasing muscle contraction).

Further oil-soluble active botanical extracts include rosmarinic acid and green tea extract, e.g. from Sabinsa Corporation USA, which may be used as antioxidants. An oil-soluble anti-oxidant is NDGA (nordihydroguaiaretic acid), e.g. from Whyte Chemicals UK.

Cosmoperine® from Sabinsa Corporation USA is an oil-soluble penetration enhancer.

Another class of active agents includes sunscreens. Exemplary sunscreens include octyl-methylcinnimate, benzophenone 3, 3-benzylidene camphor, avobenzene, para-aminobenzoic acid (PABA) and galanga (ethylhexyl para-methoxy cinnamate, which may be extracted from *Kaempferia Galanga).*

Other botanical active agents of benefit to the skin include; oil-soluble botanical extracts; melaleucol (terpinen-4-ol, SNP Natural Products Pty Ltd. (Australia)) extract from *Melaleuca alternifolia,* rosemary extract from *Rosmarinus officinalis,* rosmarinic acid extract from *Melissa officinalis,* soy isoflavones CG (50% extract from *Glycine soja,* Sabinsa Corp (USA)) and Cosmoperine® tetrahydropiperine-containing extract from *Piper nigrum* (Sabinsa Corporation (USA)) as an oil-soluble penetration enhancer.

Flavour and fragrance actives may be included within the complexes to add organoleptic effects. Fragrances include Apricosal, Fougere, and Unisex Bouquet (Arriva Fragrances (UK)).

Flavours can include agents such as the tingle compounds producing tingling parasthesia or a numbing or anaesthetic effect in the mouth, typically alkamides possess such properties giving rise to a pungent taste and causing a numbing sensation in the mouth, as reviewed by Ley P. 2005. 11th Weurmann Flavour Research Symposium, Roskilde, Denmark. Such effects are the result of stimulation of receptors e.g. TRVP, to activate afferent nerves in the mouth and nose particularly in the trigeminal system and are reported to occur with capsaicin, and the isobutylamides; spilanthol and the sanshools.

Many naturally occurring alkamides may be useful actives for delivery in the complexes described, since these materials are generally unstable in aqueous conditions but are stable in hydrocarbon solvents over extended periods (an environment which may be expected to be similar to that experienced in the compositions of the invention).

A review of chemotaxonomy indicates that the alkamides are primarily distributed in: the genus *Spilanthes,* particularly *S. oleracea (Acmella oleracea,* or *S. acmella)* also known as the toothache plant, akarkara, jambu, paracress, mafane; the genus *Echinacea* particularly *E. angustifolia* and *E. purpurea;* especially the genus *Heliopsis,* such as *H. helianthoides* var. scabra.

Spilanthol, (2E,6Z,8E)-deca-2,6,8-trienoic acid N-isobutyl amide, can additionally be produced synthetically but at such low yields that extraction from plants is the preferred option. The active pain-relieving or anti-inflammatory agent spilanthol (also known as affinin) can be extracted from the flowers of *Spilanthes oleracea.* Extracts of *Spilanthes* are commercially available from Robertet, Grasse (France) under the name Jambu Oleoresine or supplied by Gattefossé, Saint-Priest (France) under the name Gatuline® Expression. Spilanthes alcoholic extracts include those supplied by A.Vogel (Switzerland). Extracts may typically be produced using solvents e.g. hexane, petroleum ether, ethanol or by means of supercritical $CO_2$ extraction procedures such as described by Stashenko E et al. J. Chromatog. A. 1996 752: 223-232. Typical procedures include extracting approximately 200g of dried spilanthes flower heads by using supercritical $CO_2$ at 30 MPa and 60°C with a flow rate of 6 $kg.h^{-1}$ using 60 Kg $CO_2$. In addition to pain-relieving (analgesic) and anti-inflammatory properties extracts of spilanthes also have antifungal, anti-protozoal, insecticidal and molluscicidal effects.

Further active agents heliopsin and scabrin can be extracted from the roots of *Heliopsis helianthoides* var. scabra.

Still further alkamides are present in plants of the *Piperacea* family and are suitable for incorporation into the complexes of the invention, especially those from the genus *Piper* including *P. samentosum* (also known as Cha Plu), containing the alkamide sarmentine.

Additional analgesic alkamides include materials such as capsaicin (8-methyl-*N*-vanillyl-6-nonenamide) extracted from the genus *Capsicum* e.g. *Capsicum annum* and associated capsaicinoids e.g. containing >55% capsaicin (Sabinsa Europe GmbH) or 100% capsaicinoids (Sigma Co. Ltd.) or synthetically derived versions of the same.

Camphor, a terpenoid extracted from *Cinnamomum camphora* or alternmatively a synthetic version of the same, e.g. D(+)-camphor Ph Eur, BP, USP. (Merck KGaA.) is an other example of an active agent.

Anti-nociceptive sesquiterpenes and sesquiterpene lactones can also be delivered in the complexes described herein e.g. tasmanian pepper *(Tasmania laceolata)* and humulene and lupulone from *Humulus lupulus* (hops).

The quantity of active agent which may be combined with and solubilised in the compositions of the present invention will typically be in the range of 0.001-50% of the weight of surfactant and lipid, suitably in the range of 0.001-30%, especially 1.1-25%, such as 2-20% (e.g. 1.1 % to less than 5%; 5% to less than 10%; or 10% to less than 20%).

In respect of aqueous formulations, the quantity of active agent will typically be in the range of 0.001-20% of the total weight, suitably in the range of 0.001-15%, such as 0.01-10%, especially 0.1-10% (e.g. 0.05% to less than 0.1%; 0.1% to less than 5%; or 5% to less than 10%).

In a further aspect of the present invention there is provided a formulation comprising a composition of the invention and an active agent. In particular, there is provided a formulation comprising a composition of the invention and an active agent, in which the active agent is within the macromolecular assemblies.

In one embodiment of the invention the active agent is an oil soluble vitamin or oil soluble vitamin derivative (for example ascorbyl palmitate, ascorbyl dipalmitate and ascorbyl tetraisopalmitate). In a second embodiment of the invention the active agent has a triterpenoid (e.g. TECA) or steroidal nucleus (e.g. hydrocortisone or progesterone). In a third embodiment of the invention the active agent is an oil soluble peptide (e.g. palmityl-KTTKS or acetyl hexapeptide-3). In a fourth embodiment of the invention the active agent is a sunscreen. In a fifth embodiment of the invention the active agent is an antimicrobial. Specific active agents of interest are those individually listed in the Examples.

Related isobutylamides or alkylamides are the sanshools present in species of *Xanthoxylum,* also referred to as *Zanthoxylum* such as Japanese pepper *(Xanthoxylum piperitum),* Sichuan pepper *(Xanthoxylum bungeanum)* or prickly ash (southern) *(Xanthoxylum clavia-herculis).* These sanshools include α-, β-, γ- and δ- sanshools and α- and β-hydroxyl sanshools, together with herculin and neoherculin.

Suitably the active agent of use in the present invention will have a molecular weight of less than 1500 Da, such as less than 1000 Da, in particular less than 500 Da.

In certain embodiments, the active agent is not a polypeptide (in particular the active is not a membrane peptide or protein).

Active agents may be conveniently incorporated into the compositions of the present invention by the addition of the active agent to the lipid (and where appropriate to the lipid and co-surfactant) prior to the preparation of an aqueous lipid emulsion, and before the aqueous emulsion and aqueous surfactant solution are mixed. When prepared in this way, the active agent will be incorporated into the macromolecular assemblies.

In the case of formulations prepared in alcohol, the active agent will conveniently be added to the mixture prior to the removal of the alcohol.

In an analogous manner to compositions of the invention, aqueous formulations of the present invention may generally be dried and reconstituted as necessary.

The formulations of the present invention may be in the form of an aqueous solution, especially a clear aqueous solution (e.g. a stable clear aqueous solution), suitably a clear and colourless aqueous solution (e.g. a stable clear and colourless aqueous solution). However, for ease of transportation and handling, once prepared, the formulation may be dried (e.g. by freeze-drying, rotary evaporation or such like) to form a solid which has the benefits of being lower in both volume and weight.

In one embodiment of the present invention the formulation is in the form of an aqueous solution. Aqueous solutions include aqueous semi-solids, such as gels. In a further embodiment of the present invention the formulation is in dried form (for example as a powder, resin or flake). Suitably formulations of the invention in dried form can be reconstituted into aqueous solution to provide aqueous solutions.

Suitably an aqueous solution of the formulations of the present invention will contain at least 60% water by weight, such as at least 70%, especially at least 80%, in particular at least 90% (e.g. at least 95%, or at least 99%).

Suitably dried formulations of the present invention will be substantially free of water, for example containing less than 5% water by weight, especially less than 2.5%, in particular less than 1.0%, such as less than 0.25%.

Suitably an aqueous formulation will comprise the active agent in an amount which exceeds the solubility level of the active agent in aqueous solution with the same amount of surfactant alone (e.g. at least 1.25 times the aqueous solubility, especially at least 1.5 times, such as at least 2 times). Suitably a dried formulation of the present invention will comprise the active agent in an amount which exceeds the solubility level of the active agent in aqueous solution with the same amount of surfactant alone (e.g. at least 1.25 times the aqueous solubility, especially at least 1.5 times, such as at least 2 times) when the formulation is reconstituted into water at a concentration equivalent to at a dry weight in the range of around 0.1-10% (based on the weight of surfactant, lipid and active components), such as about 4%.

In a further aspect of the present invention there is provided a method for the production of a composition comprising lipid, surfactant and active agent wherein the surfactant and lipid are in the form of macromolecular assemblies, comprising the steps of:

(i) Preparing an aqueous solution of surfactant;
(ii) Preparing an aqueous emulsion of lipid and active agent; and
(iii) Mixing the aqueous emulsion of lipid and active agent and the aqueous solution of surfactant;
such that macromolecular assemblies are formed.

Optionally a co-surfactant is included in the aqueous solution of (i) or the aqueous emulsion (ii).

If desirable, a further optional step of removing the water may be performed to provide dried formulations of the present invention.

Alternatively, there is provided a method for the production of a composition comprising lipid, surfactant and active agent, wherein the lipid, surfactant and active are in the form of macromolecular assemblies, comprising the steps of:

(i) Preparing an aqueous solution of surfactant;
(ii) Preparing an aqueous emulsion of lipid;
(iii) Preparing an aqueous emulsion of active agent; and
(iv) Mixing the aqueous aqueous solution of surfactant, the aqueous emulsion of lipid and the aqueous emulsion of

active agent;
such that macromolecular assemblies are formed.

Suitably step (iv) comprises the mixing of the emulsions derived from (ii) and (iii), followed by the addition of the mixture to the solution derived from (i). Optionally step (iv) comprises the addition of the emulsion derived from (ii) to a mixture of the emulsion derived from (iii) and the solution derived from (i). Again, optionally a co-surfactant is included in the aqueous solution of (i) or the aqueous emulsions of (ii) or (iii). If desirable, a further optional step of removing the water may be performed to provide dried formulations of the present invention.

There is also provided a method for the production of a composition comprising lipid, surfactant and an active agent wherein the surfactant, lipid and active agent are in the form of macromolecular assemblies, comprising the steps of mixing the surfactant, lipid and active agent in a short chain alcohol (e.g. ethanol or isopropanol, especially isopropanol), suitability at elevated temperature (around 50 °C), and subsequently removing the alcohol (e.g. by rotary evaporation) such that dried macromolecular assemblies are formed. Aqueous solutions of the formulation may then be prepared by solubilisation in water, suitably with warming. Co-surfactant, if present, will be mixed with the surfactant, lipid and active agent in the alcohol before the composition is dried.

Particular active agents of interest include: TECA, Myristyl ester of L-pyrrolidone, lauric ester of L-pyrrolidone carboxylic acid, Ciclopirox olamine, Econazole nitrate, Red clover extract, Centella extract, Butcher's broom extract, Benzyl nicotinate, Piroctone olamine, acetyl hexapeptide-3, extract of *Ginkgo biloba,* Horse chestnut extract, Nettle extract, Aesculus extract, Yohimbine free base, Hydrocortisone, Salmeterol xinafoate, Progesterone, Devil's claw extract, Gatuline® Expression, extract of *Picea abies,* D-Camphor, Totara-8,11,13-trien-13-ol, extract of *Spilanthes acmella,* Undecylenoyl phenylalanine, extract of *Cimicifuga racemosa,* extract of *Boswellia serrata,* Sichuan pepper extract and Prickly ash extract.

Other active agents of interest include 7-dehydrocholesterol, Apricosal, ascorbyl palmitate, avobenzene, betamethasone 17-valerate, *Boswellia,* camphor, capsaicin, Cha-Plu extract, cholesterol sulphate, cholesterol, clobetasol propionate, clotrimazole, Cosmoperine, diclofenac, *Echinacea angustafolia, Echinacea purpurea,* Edemine, erythromycin sulphate, eserine, Eusolex 4360, Fougere, *Galanga, Ginkgo, Heliopsis* extract, hops tincture, hydrocortisone 17-butyrate, Japanese pepper extract, ketaconazole, ketoprofen, maca, melaleucol, minoxidil, naproxen, NDGA, neomycin sulphate, nystatin, octyl salicylate, PABA, PT-40, P-U, Questice CQ U/A, rosemary extract CG, rosmarinic acid (90%), soy isoflavones CG (50%), *Spilanthes* supercritical $CO_2$ extract, stearyl glycrrhetinate, tarragon extract, tasmanian pepper extract, THC CG, THC Ultra Pure, Unisex Bouquet, Unisol S-22, vitamin C palmitate, vitamin $D_3$, vitamin E.

One skilled in the art will recognise that the active agent(s) in natural extracts may be further purified or isolated, or alternatively prepared by synthetic means.

Suitably a formulation according to the present invention will consist essentially of surfactant, lipid and active agent, optionally together with co-surfactant, (i.e. a dried formulation will suitably comprise less than 10% of other components, suitably less than 5%, especially less than 2% by weight combined of the surfactant, lipid and active agent; an aqueous formulation will suitably comprise less than 10% of other components apart from water, suitably less than 5%, especially less than 2% by combined weight of the water, surfactant, lipid and active agent).

**Preparations**

In general a formulation of the present invention will be incorporated into a cosmetic or pharmaceutical preparation which is tailored to suit a particular purpose, manner of use and mode of administration.

Formulations (or compositions as desired) may be mixed with one or more cosmetic or pharmaceutically acceptable carriers or excipients (anti-oxidants, preservatives, viscosity modifiers, colourants, flavourants, perfumes, buffers, acidity regulators, chelating agents, or other excipients), and optionally with other therapeutic ingredients if desired. Such preparations may be prepared by any of the methods known in the art, and may for example be designed for inhalation, topical, parenteral (including intravenous, intra-articular, intra-muscular, intra-dermal and subcutaneous) administration or oral administration.

Preparations for systemic delivery are suitably made using pharmaceutically acceptable components, especially biodegradable components. Some of the phospholipids described in this application are used for parenteral nutrition and are therefore likely to be broken down fairly readily in the body without causing serious problems. A number of the surfactants described herein are available in pharmaceutical grades. Preparations for parenteral delivery will suitably be sterile.

Compositions of the present invention are believed to be particularly suitable for facilitating the topical delivery of active agents (e.g. topically for local effect, or alternatively topically for systemic effect), in particular topical delivery to a mammal (e.g. a human). Topical delivery may, for example, be via a mucosal surface. Topical delivery will typically be via the dermal surface. Compositions of the present invention are believed to be particularly suitable for the delivery of active agents to (or through) the skin, in particular to (or through) the skin of humans.

When delivering active agents to the skin it is generally important that the particle size be less than that of the lipid interstices found between the corneocytes within the outer layer of the skin, in order for the material to be adequately absorbed into the *stratum corneum.* The inter-corneocyte interstices have relatively small thickness, hence, particles should desirably be sized to be absorbed efficiently. The macromolecular assemblies described in this application may be well suited to penetrating the inter-corneocyte lipid layer and could therefore be used to deliver oily materials such as the active agents already described. Since the macromolecular assemblies may be trapped within the *stratum corneum,* they may act as reservoirs for active agents to enable sustained release into the deeper layers of the skin and thereby provide a distinct therapeutic profile. Advantageously, this could improve product efficacy, reduce the number of applications and quantity of active agent required, and would be more convenient for the consumer or patient.

Although formulations for repeated application to the skin may be slightly acidic, typically being in the pH 5.0-7.5 range, particularly pH 5.5-7.5, formulations for application to other sites, or for internal administration, should typically be maintained around pH 6.5-7.5. Formulations specifically for application to the eye are ideally in the range pH 7.1-7.8, more particularly pH 7.3-7.6 (Carney, LG and Hill, RM Arch. Ophthalmol. 1976 94(5):821-824).

Preparations for topical application may include, for example, anti-oxidants (e.g. alpha-tocopherol, butylated hydroxyanisole (BHA) or butylated hydroxytoluene (BHT)), preservatives (e.g. 2-phenoxyethanol, sorbic acid or parabens), viscosity modifiers (e.g. water soluble gums and resins, such as xanthan gum, carboxymethyl cellulose or lightly cross-linked synthetic polymers such as carbomers, e.g. Carbopols), colourants, flavourants, perfumes, buffers, acidity regulators, chelating agents (e.g. such as EDTA, sodium edetate, disodium edetate or calcium disodium edetate), penetration enhancers and anti-tack agents. Suitable carbomers include Carbopol® 980 and Ultrez® 20. Other suitable gelling agents include carbomers: Carbopol® Ultrez 10, Carbopol® Ultrez 21, Carbopol® Aqua-SF1, Stabileze® QM, Natrasol® 250 and Blanose® 7HF. Other suitable preservatives include Nipaguard PDU (e.g. at around 0.5% by weight), Nipaguard DMDMH (e.g. at around 0.2% by weight), Germaben® II-E (e.g. at around 1% by weight), Suttocide® A (e.g. at around 0.5% by weight) and Euxyl® K500 (e.g. at around 1.5% by weight).

Preparations for topical application may be incorporated into hydrogel patches (i.e. 3-dimensional gels of fixed structure, such as those available from Telic S.A. (Spain)). Other biocompatible hydrogel patches are those supplied by Allmi-Care Limited (Nottingham, UK). Application utilising hydrogels may be advantageous in that: (i) the hydrogel patch may act as a convenient repository for prolonged administration and/or (ii) the hydrogel patch may provide a quantifiable dosage form, such that the quantity of active agent administered can be effectively controlled. Additionally, hydrogel patches may aid absorption by ensuring that skin is fully hydrated.

Delivery of active agents using hydrogel patches may be enhanced by the use of electrical stimulation techniques, such as transcutaneous electrical nerve stimulation (TENS). An alternative electrical stimulation technique is Interferential TENS.

There is provided a composition of the invention (suitably as a formulation which comprises an active agent, or as a preparation which comprises an active agent and a carrier or excipient) which is presented in a hydrogel patch. The hydrogel patch may optionally be adapted for use as an electrode (e.g. being suitable for use in TENS or Interferential TENS).

Thus, there is provided a cosmetic preparation comprising a formulation of the invention and a cosmetically acceptable carrier or excipient.

There is also provided a pharmaceutical preparation comprising a formulation of the invention and a pharmaceutically acceptable carrier or excipient.

Accordingly, there is also provided a formulation of the present invention for use in therapy.

In a further aspect of the present invention there is provided the use of a composition of the invention as a solubilising agent (e.g. a non-irritating solubilising agent), for example in the solubilisation of an active agent (such as those described previously).

Poor bioadbsorption of poorly water soluble active agents through the gastrointestinal tract is a common problem for formulators. Compositions of the present invention, due to their ready ability to be resolubilsed from the dry state, may therefore beneficially improve the absorption (e.g. rate of uptake or absolute bioavailablity) resulting from oral administration of a hydrophobic active agents. Dried formulations of the invention may therefore be utilised in the manufacture of pharmaceutical compositions for oral administration.

Consequently, there is provided a method for improving the absorption of an orally administered active agent comprising preparing a formulation of the invention comprising said active agent. Suitably the formulation of the invention will be in dried form. Suitably the dried formulation will be in a unit dose presentation (e.g. a tablet, capsule or such like).

**Membrane protein/peptide solubilisation**

As mentioned previously, it is believed that the macromolecular assemblies of the present invention are discoidal in shape, mimicking circular fragments of biological membranes. Other potential uses of compositions of the present invention include use as a means of solubilising membrane peptides or proteins for the investigation of their structure

and/or interactions with other species.

A need has been identified for solubilising agents that can be used for solubilising membrane peptides and proteins (including integral, membrane tethered or membrane associated proteins, for example drug receptor proteins), within phospholipid membranes in such a way as to substantially retain their native conformation (e.g. to maintain their natural activities) and thereby to enable their structure to be investigated (e.g. by NMR spectroscopy, but also other suitable techniques which are well known to those skilled in the art including X-ray crystallography, infra-red spectroscopy and circular dichroism).

In addition to structural investigations, it may also be desirable to investigate the interactions of membrane proteins and peptides with other species. Such other species may also be peptides and proteins (e.g. other membrane peptides and proteins). In the case of membrane receptors such other species include ligands and ligand fragments (e.g. agonists and antagonists). In the case of enzymes, such other species may be ligands and ligand fragments (e.g. substrate(s) and inhibitors).

International patent application number PCT/GB2006/050134, publication number WO2006129127, exemplifies the use of compositions comprising a lipid and copolymer of styrene and maleic acid, wherein the ratio of styrene to maleic acid monomer units is greater than 1:1, and wherein the polymer and lipid are in the form of macromolecular assemblies, in the solubilisation of exemplary membrane proteins such as bacteriorhodopsin for the purpose of structural studies.

When a composition of the invention comprises a membrane protein, such compositions are suitably prepared by the dialysis of a solution comprising (i) macromolecular complexes of the invention which are absent a membrane protein and (ii) detergent solubilised membrane protein, such that the membrane protein partitions into the macromolecular complexes of the invention. Alternatively, compositions of the invention comprising a membrane protein can be prepared by the direct solubilisation of a biological membrane to form the macromolecular assemblies, followed by the isolation of macromolecular assemblies containing the desired protein from the other materials present (e.g. by affinity chromatography, such as the use of a nickel chelating column).

Accordingly, there is provided the use of a composition of the invention for the solubilisation of a membrane peptide or protein. Also provided are compositions of the invention (e.g. in dry or aqueous form) which further comprise a membrane peptide or protein.

There is also provided a method for the solubilisation of a membrane peptide or protein which comprises forming a composition of the invention which comprises said membrane peptide or protein (i.e. the membrane peptide or protein is within the macromolecular assemblies).

Further, there is provided a method for the screening of candidate agents for interaction with a membrane protein or peptide comprising the steps of:

(i) solubilising a membrane protein or peptide in a composition of the invention;
(ii) testing a candidate agent to determine whether it interacts with the solubilised membrane protein or peptide.

Determination of interaction between the target protein or peptide and the candidate agent may be performed using any suitable technique known to those skilled in the art, for example by monitoring the environment or location of the candidate agent (e.g. by NMR spectroscopy, radiolabelling) or alternatively by monitoring the environment/activity of the target (e.g. observing structural changes in the target, or measuring changes in activity of an enzyme or activation state of receptors through assays).

Additionally, there is provided a method for the structural investigation of a membrane protein or peptide comprising the steps of:

(i) solubilising a membrane protein or peptide in a composition of the invention;
(ii) investigating the structure of said membrane protein or peptide.

Structural investigation may utilise any suitable technique known to those skilled in the art, for example by NMR spectroscopy, X-ray crystallography, infra-red spectroscopy and circular dichroism).

Candidate agents may be putative ligands or ligand fragments (e.g. agonists, antagonists, inhibitors and such).

By the term membrane peptide is meant a polypeptide of less than 50 residues (such as less than 40 residues or less than 30 residues) and which normally resides partially or fully within a biological membrane. By the term membrane protein is meant a polypeptide of at least 50 residues, for example at least 100 residues, which normally resides partially or fully within a biological membrane.

**Other uses**

It may also be envisaged that the compositions of the present invention may be used to solubilise membrane

peptides or proteins which are immunogenic in nature (e.g. antigens), and which could then be used in vaccines. Alternatively, compositions of the invention may be of use as particulate vaccine adjuvants for enhancing immunogenicity and improving the immune response of antigens in vaccines. Due to the ability of the compositions of the invention to solubilise hydrophobic agents, the compositions may also be used to solubilise active agents which are non-specific immune response enhancers (e.g. lipopolysaccharides, such as monophosphoryl lipid A and 3-de-O-acylated monophosphoryl lipid A; saponins, such as QS-21).

Furthermore, there is a need for treatment of medical conditions affecting mucosal surfaces, e.g. for ophthalmic use in the treatment of the condition known as "dry eye" syndrome, and for lubricating biological (e.g. synovial) membranes. The tear film has a coating of phospholipids, which are necessary for the formation of a stable tear film. Diseases where the tear film is deficient may potentially be treated by the addition of an aqueous phospholipid solution, such as an aqueous solution of the compositions of the present invention. Compositions of the present invention are advantageous in this regard, since they are clear and colourless, unlike conventional aqueous preparations of phospholipids which may be opaque.

Compositions of the invention provide the means for preparing a more ophthalmically acceptable formulation of aqueous insoluble drugs e.g. steroids.

There is also a need for lubricating phospholipids to treat the surfaces of articulated joints in connection with arthritic conditions or to lubricate surfaces of medical devices and prostheses, e.g. artificial joints and contact lenses, that are fitted into or on the body, or to prevent focal adhesions between tissues such as those that may occur during surgical procedures. Compositions of the present invention may be of use in this regard (e.g. by intra-articular injection). In addition to the lubricating potential the compositions may concurrently deliver agents to reduce inflammation or induce analgesia.

The compositions of the invention may also have the ability to deliver active agents locally to the lung or, via the highly permeable membranes lining the deep lung, into the systemic circulation. The similarity between the phospholipid compositions of the invention and the surfactant fluid lining the internal alveolar and bronchial surfaces of the lung may ensure that the compositions of the invention are suited to deliver active agents to the lung, especially the deep lung, or to act as a means of delivering phospholpid to the lung for the treatment of neonatal or adult respiratory distress syndrome, a condition characterised by a insufficient levels of native lung surfactant or phospholipids. Delivery to the lung may be by aerosol or by nebulisation.

**Dimerisation**

As a result of some receptors existing in the form of functional dimers, in particular GPCRs e.g. adrenoreceptors, drug ligands have been shown to have greater efficacy if two molecules simultaneously bind to a receptor dimer or if the active sites of two drug molecules are conjugated as bivalent ligand analogues, separated by a spacer group such that the distance between the active sites optimizes the interaction with the dimerized receptor (e.g. adrenoreceptors in Angers S et al Proceedings of the National Academy of Science USA 2000 97(7):3684-3689; opioid receptors in Portoghese PS et al Journal of Medicinal Chemistry 2001 44 (14):2259-2269).

The disadvantage of this approach to the development of potent new drugs is the requirement to synthesize new chemical entities together with the associated toxicity and regulatory hurdles. However, the macromolecular assemblies described herein offer the potential advantage of acting as platforms to carry drug molecules, especially those agents where the active site is separated from a hydrophobic portion of the molecule which can be used to anchor into the bilayer membrane while exposing its active site. By adjusting the concentration of drug molecules, lipid and surfactant the number of molecules contained in each bilayer can be varied and thereby the average distance between the active sites altered to precisely match the binding sites of the dimeric drug receptors. In this way the potency and/or selectivity of existing drugs in stimulating drug receptors can be greatly enhanced without the need for the development of new chemical entities, greatly reducing the toxicological risks and costs of development.

Suitable dimerised target receptors include adrenoreceptors (AR) particularly the $\beta_2$-AR (Angers 2000), and $\alpha_2$-AR (Lalchandani SH. et al Journal of Pharmacology and Experimental Therapeutics 2002 303(3):979-984), and their appropriate ligands; salmeterol (salmeterol xinafoate) Ph.Eur micronised grade supplied by Natco Pharma Ltd. (India) and yohimbine HCl USP, an extract of *Pausinystalia yohimbe,* supplied by International Lab Inc. (India), respectively. Example formulations of these two agents are given in the Examples, as part of the particles described herein. Selective $\alpha_2$-AR anatagonists would be expected to be particularly suited to the treatment of Raynaud's disease. Such formulations are meant merely to exemplify the application of this technology which could be equally well applied to other amphiphilic drug molecules that act through dimerised receptor ligands Such high potency and/or selective drug lipid/surfactant assemblies could be applied at lower doses than the unassociated drugs and would be expected to degrade in the body, especially if applied through the lung, into individual drug molecules of conventional potency or selectivity, thereby greatly reducing the side effects of the drug assemblies. Other potential agents could include amphiphilic agents acting through dimerized opioid receptors (Portoghese PS et al Journal of Medicinal Chemistry 2001 44 (14):2259-2269) such as

fentanyl (and derivatives such as sufentanyl and remifentanyl), lofentanil and diphenoxylate Yet other receptor subtypes which could be targeted in a similar manner with drug associated lipid:surfactant particles include dopamine, melatonin, and serotonin.

**Miscellaneous**

In certain embodiments the surfactant is not monolauryl lysine.

When the surfactant is laureth-23 (e.g. Brij 35), suitably the composition of the invention does not include phosphatidylethanolamine-N-fluorescein at a surfactant to phosphatildyethanolamine-N-fluorescein ratio of 60:1 by weight. When the surfactant is laureth-23 (e.g. Brij 35), suitably the composition of the invention does not include 3H labelled giberellin A4 at a surfactant to phosphatildyethanolamine-N-fluorescein ratio of 1 mg surfactant to 1kBq. Suitably, the surfactant is not laureth-23.

Suitably the active agent is not phosphatildyethanolamine-N-fluorescein. Suitably the active agent is not 3H labelled giberellin A4 (such as any giberellin). Suitably the active agent is present as more than 1% of the weight of the surfactant and the lipid.

Suitably the surfactant is not oleth-10. Suitably the surfactant is not ceteth-10. Suitably the surfactant is not ceteth-20. Suitably the surfactant is not a PEG-7 ether of decanol. Suitably the surfactant is not PEG-20 stearate. Suitably the surfactant is not octoxynol-9. Suitably the surfactant is not polysorbate 80. Suitably the surfactant is not sodium cholate or sodium deoxycholate. Suitably the surfactant is not octyl glucoside. Suitably the surfactant is not SDS.

Suitably aqueous solutions of the composition of the present invention are substantially free of short chain alcohols (such as ethanol, propanol or glycerol, in particular ethanol), containing less than 10% by weight, especially less than 5%, in particular less than 1% (e.g. less than 0.5% or less than 0.25%).

Suitably aqueous solutions of the composition of the present invention are substantially free of propylene glycol or polyethylene glycol, containing less than 10% by weight, especially less than 5%, in particular less than 3% (e.g. less than 1% or less than 0.25%).

Suitably, the aqueous compositions of the invention comprising membrane proteins are not prepared by the direct solubilisation of a biological membrane using the surfactant.

Suitably, when a composition of the invention comprises a membrane protein, such compositions are prepared by the dialysis of a solution comprising (i) macromolecular complexes of the invention which are absent of a membrane protein and (ii) membrane protein which has been solubilised by conventional detergent, such that the membrane protein partitions into the macromolecular complexes of the invention. Alternatively, compositions of the invention comprising a membrane protein can be prepared by the direct solubilisation of a biological membrane to form the macromolecular assemblies, followed by the isolation of macromolecular assemblies containing the desired protein from the other materials present (e.g. by affinity chromatography, such as the use of a nickel chelating column).

Suitably, aqueous compositions of the invention do not comprise a membrane protein (i.e. a polypeptide which typically resides within a biological membrane and has a molecular weight of at least 2000 Da, for example at least 5000 Da or at least 25000 Da).

Suitably, the surfactant concentration in aqueous solution is at least 0.7% by weight when the compositions of the invention comprise heptethoxyethylenated octoxyphenol, octoxynol-9, octoxynol-9.5, octoxynol-12, octoxynol-12.5, octoxynol-16, octoxynol-30, ceteth-10 or ceteth-20.

Suitably, when the compositions of the invention comprise a membrane protein the surfactant is not a heptethoxyethylenated octoxyphenol. Suitably when the compositions of the invention comprise a membrane protein the surfactant is not octoxynol-9. Suitably when the compositions of the invention comprise a membrane protein the surfactant is not octoxynol-9.5. Suitably when the compositions of the invention comprise a membrane protein the surfactant is not octoxynol-12. Suitably when the compositions of the invention comprise a membrane protein the surfactant is not octoxynol-12.5. Suitably when the compositions of the invention comprise a membrane protein the surfactant is not octoxynol-16. Suitably when the compositions of the invention comprise a membrane protein the surfactant is not octoxynol-30. Suitably when the compositions of the invention comprise a membrane protein the surfactant is not ceteth-10. Suitably when the compositions of the invention comprise a membrane protein the surfactant is not ceteth-20.

Suitably the surfactant is not a polyoxyethylenated octoxyphenol ether having 7.5, 9.5, 12.5, 15 or 30 PEG units.

Suitably when a membrane protein is solubilised in a composition of the present invention, the membrane protein is not rat cytochrome oxidase, rat glycerol phosphate dehydrogenase, rat malic dehydrogenase, rat monoamine oxidase, rat succinic dehydrogenase or a mixture of rat mitochondrial proteins.

In certain embodiments the compositions of the invention are substantially free of polypeptide material, for example, containing less than 10% by dry weight, especially less than 5%, in particular less than 1% (e.g. less than 0.25%, less than 0.1 % or less than 0.01 %, such as less than 0.001 %). When compositions of the invention do contain polypeptide material, suitably the compositions are not prepared by the direct solubilisation of a natural membrane.

Suitably the compositions of the present invention are substantially free of triglycerides containing less than

10% by dry weight, especially less than 5%, in particular less than 1% (e.g. less than 0.5% or less than 0.25%). Suitably the aqueous compositions of the present invention are substantially free of triglycerides containing less than 10% by weight, especially less than 5%, in particular less than 1% (e.g. less than 0.5% or less than 0.25%).

Suitably the surfactant is not sucrose laurate ester. Suitably the surfactant is not PEG-8 laurate.

Suitably the aqueous compositions of the invention do not comprise an oil in water or water in oil emulsion.

Suitably the compositions of the invention are substantially free of oils and fats customary as emulsion oil phases in cosmetics and pharmaceutics, such as the typical oil phases of: e.g. ethers (dicarprylyl ether), triglycerides (caprylic capric triglycerides), alcohols (octyldodecanol), ester oils (cetearyl isononanoate), hydrocarbons (dioctyl cyclohexane), paraffins, silicone oils (cyclomethicone) and mixtures of these oil phases. Suitably the compositions of the invention contain less than 5%, especially less than 2.5%, in particular less than 1.0% (such as less than 0.25%, or less than 0.01 %) of such materials.

Suitably the composition is substantially free of sterol (in particular substantially free of cholesterol), comprising less than 20% sterol, such as less than 10%, for example less than 5% sterol by dry weight (such as less than 2% sterol by dry weight).

Suitably the surfactant is not a polyethoxyethylated lipid, in particular when the composition of the invention comprises cholesterol (e.g. when the composition comprises at least 20%, such as at least 10%, for example at least 5% cholesterol by dry weight). Suitably the surfactant is not PEG(2000)-DSPE, PEG(5000)-DSPE, PEG(2000)-DPPE, PEG(5000)-DPPE, PEG(2000)-DPOE, PEG(5000)-DPOE, PEG(2000)-ceramide or PEG(5000)-ceramide (in particular when the composition comprises at least 20%, such as at least 10%, for example at least 5% cholesterol by dry weight).

Suitably the surfactant is not laureth-8.

Suitably the surfactant is not amphipol A8-35.

Suitably the surfactant is not a homopolymer of ethacrylic acid. Suitably the surfactant is not a hydrolysed alternating copolymer of maleic anhydride and either styrene or an alkyl vinyl ether. Suitably the surfactant is not a hydrolysed copolymer of maleic anhydride and styrene. Suitably the surfactant is not a copolymer of styrene/maleic acid (i.e. including fully and partially hydrolysed co-polymers of styrene/maleic anhydride) or an ester or partially esterified copolymer of styrene/maleic acid.

Suitably the surfactant is not an ethoxylated PPG acyl ether. Suitably the surfactant is not an ethoxylated PPG ether. Suitably the surfactant is not a propoxylated POE ether. Suitably the surfactant is not an ethoxylated glyceride. Suitably the surfactant is not a polyglycerol ester. Suitably the surfactant is not an acylated sorbitan ester. Suitably the surfactant is not a PEG non-sorbitan sugar ester. Suitably the surfactant is not a synthetic phospholipid. Suitably the surfactant is not a fatty acid. Suitably the surfactant is not an ester of an alpha-hydroxycarboxylic acid. Suitably the surfactant is not an anionic phosphate based surfactant. Suitably the surfactant is not cocamidopropyl betaine. Suitably the surfactant is not sodium cholate, sodium deoxycholate, sodium laureth sulphate or sodium lauryl sulphate.

In addition to the surfactants noted here which are the products of commerce and hence may have a variation of structures and corresponding HLB values, monodisperse surfactants of use in the invention can also be applied, this is most suitable for applications related to maintaining membrane proteins within a phospholipid membrane for the purpose of defining the protein structure. In this instance highly purified surfactants such as those available from Anatrace Inc. (Maumee, OH, USA) under the tradenames, Anapoe®-35, Anapoe®-20 and Anapoe®-X-100 are examples.

The invention is further illustrated by the following clauses:

Clause 1. A composition comprising lipid and surfactant, characterised in that the surfactant has an HLB number of less than 20 and in that the lipid and surfactant are in the form of macromolecular assemblies of less than 100 nm in diameter.

Clause 2. A composition according to clause 1 comprising lipid and surfactant, characterised in that the surfactant has an HLB number in the range of about 10.5 to about 17.5 and in that the lipid and surfactant are in the form of macromolecular assemblies of less than 100 nm in diameter.

Clause 3. A composition comprising lipid and surfactant, characterised in that the surfactant is an ether surfactant and in that the lipid and surfactant are in the form of macromolecular assemblies of less than 100 nm in diameter.

Clause 4. A composition comprising lipid and surfactant, characterised in that the surfactant is an ester surfactant and in that the lipid and surfactant are in the form of macromolecular assemblies of less than 100 nm in diameter.

Clause 5. A composition comprising lipid and surfactant, characterised in that the surfactant is an ionic surfactant and in that the lipid and surfactant are in the form of macromolecular assemblies of less than 100 nm in diameter.

Clause 6. A composition according to any one of clauses 1 to 3, wherein the surfactant is an ether surfactant which

is an ethoxylated alcohol surfactant.

Clause 7. A composition according to clause 6, wherein the ethoxylated alcohol is an ethoxylated aromatic alcohol surfactant.

Clause 8. A composition according to clause 7, wherein the ethoxylated aromatic alcohol surfactant is selected from octoxynol-12, nonoxynol-15, octoxynol-16, nonoxynol-20 and mixtures thereof.

Clause 9. A composition according to clause 6, wherein the ethoxylated alcohol is an ethoxylated non-aromatic alcohol surfactant.

Clause 10. A composition according to clause 9, wherein the ethoxylated non-aromatic alcohol surfactant is a member of the laureth series.

Clause 11. A composition according to clause 10, wherein the ethoxylated non-aromatic alcohol surfactant is selected from laureth-8, laureth-10, laureth 23 and mixtures thereof.

Clause 12. A composition according to clause 9, wherein the ethoxylated non-aromatic alcohol surfactant is a member of the ceteth series.

Clause 13. A composition according to clause 12, wherein the ethoxylated non-aromatic alcohol surfactant is selected from ceteth-10, ceteth-15, ceteth-20 and mixtures thereof.

Clause 14. A composition according to clause 9, wherein the ethoxylated non-aromatic alcohol surfactant is a member of the oleth series.

Clause 15. A composition according to clause 14, wherein the ethoxylated non-aromatic alcohol surfactant is selected from oleth-15, oleth-20, oleth-30 and mixtures thereof.

Clause 16. A composition according to clause 9, wherein the ethoxylated non-aromatic alcohol surfactant is a member of the pareth series.

Clause 17. A composition according to clause 16, wherein the ethoxylated non-aromatic alcohol surfactant is selected from C11-15 pareth-12, C11-15 pareth-15, C11-15 pareth-20, C12-C13 pareth-23 and mixtures thereof.

Clause 18. A composition according to clause 9, wherein the ethoxylated non-aromatic alcohol surfactant is a member of the ceteareth series.

Clause 19. A composition according to clause 18, wherein the ethoxylated non-aromatic alcohol surfactant is selected from ceteareth-20, ceteareth-25, ceteareth-30 and mixtures thereof.

Clause 20. A composition according to clause 9, wherein the ethoxylated non-aromatic alcohol surfactant is a member of the isosteareth series.

Clause 21. A composition according to clause 20, wherein the ethoxylated non-aromatic alcohol surfactant is isosteareth-20.

Clause 22. A composition according to clause 9, wherein the ethoxylated non-aromatic alcohol surfactant is a member of the coceth series.

Clause 23. A composition according to clause 20, wherein the ethoxylated non-aromatic alcohol surfactant is selected from coceth-10, coceth-20 and mixtures thereof.

Clause 24. A composition according to any one of clauses 1, 2 and 4, wherein the surfactant is an ester surfactant which is an ethoxylated carboxylic acid surfactant.

Clause 25. A composition according to clause 24, wherein the ethoxylated carboxylic acid is a member of the stearate series.

Clause 26. A composition according to clause 25, wherein the ethoxylated aromatic alcohol surfactant is selected from PEG-20 stearate, PEG-40 stearate and mixtures thereof.

Clause 27. A composition according to any one of clauses 1, 2 and 4, wherein the surfactant is an ester surfactant which is a sugar ester surfactant.

Clause 28. A composition according to clause 27, wherein the sugar ester is a PEG sorbitan ester surfactant.

Clause 29. A composition according to clause 28, wherein the PEG sorbitan ester surfactant is polysorbate 20.

Clause 30. A composition according to clause 27, wherein the sugar ester is a non-sorbitan sugar ester surfactant.

Clause 31. A composition according to clause 30, wherein the non-sorbitan sugar ester surfactant is selected from sucrose laurate, sucrose myristate, decyl glucoside or mixtures thereof.

Clause 32. A composition according to any one of clauses 1, 2 and 5, wherein the surfactant is an ionic surfactant which is a cationic surfactant.

Clause 33. A composition according to clause 32, wherein the cationic surfactant is a PEG alkyl amine surfactant.

Clause 34. A composition according to clause 33, wherein the PEG alkyl amine surfactant is selected from PEG-5 cocamine, PEG-15 cocamine and mixtures thereof.

Clause 35. A composition according to any one of clauses 1, 2 and 5, wherein the surfactant is an ionic surfactant which is an anionic surfactant.

Clause 36. A composition according to clause 35, wherein the cationic surfactant is an amino acid amide surfactant.

Clause 37. A composition according to clause 36, wherein the amino acid amide surfactant is selected from sodium lauroyl glutamate, sodium cocoyl glycinate, sodium cocoyl methyl taurate, sodium cocoyl glutamate, disodium cocoyl glutamate, sodium lauryl wheat amino acids, potassium lauryl wheat amino acids, sodium lauryl oat amino acids, sodium cocoyl apple amino acids and mixtures thereof.

Clause 38. A composition according to clause 35, wherein the anionic surfactant is surfactin.

Clause 39. A composition according to any one of clauses 1 to 38, wherein the HLB of the surfactant is in the range of about 12 to about 17.

Clause 40. A composition according to clause 39, wherein the HLB of the surfactant is in the range of about 13.5 to about 17.

Clause 41. A composition according to clause 39, wherein the HLB of the surfactant is in the range of 12 to less than 13.

Clause 42. A composition according to clause 39, wherein the HLB of the surfactant is in the range of 13 to less than 14.

Clause 43. A composition according to clause 39, wherein the HLB of the surfactant is in the range of 14 to less than 15.

Clause 44. A composition according to clause 39, wherein the HLB of the surfactant is in the range of 15 to less than 16.

Clause 45. A composition according to clause 39, wherein the HLB of the surfactant is in the range of 16 to less than 17.

Clause 46. A composition according to any one of clauses 1 to 45, wherein the surfactant has a molecular weight of less than about 10000 Da.

Clause 47. A composition according to clause 46, wherein the surfactant has a molecular weight of less than about 8000 Da.

Clause 48. A composition according to clause 47, wherein the surfactant has a molecular weight of less than about

5000 Da.

Clause 49. A composition according to clause 48, wherein the surfactant has a molecular weight of less than about 3000 Da.

Clause 50. A composition according to clause 49, wherein the surfactant has a molecular weight of less than about 2500 Da.

Clause 51. A composition according to clause 50, wherein the surfactant has a molecular weight of less than about 1800 Da.

Clause 52. A composition according to clause 46, wherein the surfactant has a molecular weight of between 3000 to 8000 Da.

Clause 53. A composition according to any one of clauses 1 to 52, wherein the surfactant is selected from the list consisting of octoxynol-12, nonoxynol-15, octoxynol-16, nonoxynol-20, laureth-8, laureth-10, laureth 23, ceteth-10, ceteth-15, ceteth-20, oleth-15, oleth-20, C11-15 pareth-12, C11-15 pareth-15, C11-15 pareth-20, C12-C13 pareth-23, ceteareth-20, ceteareth-25, ceteareth-30, isoceteth-20, isosteareth-20, PEG-20 stearate, PEG-40 stearate, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, disodium cocoyl glutamate, potassium lauryl wheat amino acids, sodium lauryl oat amino acids, sodium lauryl wheat amino acids, sodium cocoyl apple amino acids, sodium cocoyl methyl taurate and surfactin.

Clause 54. A composition according to clause 53, wherein the surfactant is selected from the list consisting of octoxynol-12, nonoxynol-15, octoxynol-16, nonoxynol-20, laureth-10, laureth 23, ceteth-10, ceteth-15, ceteth-20, oleth-15, oleth-20, C11-15 pareth-12, C11-15 pareth-15, C11-15 pareth-20, C12-C13 pareth-23, ceteareth-20, ceteareth-25, isoceteth-20, isosteareth-20, PEG-20 stearate, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, disodium cocoyl glutamate, potassium lauryl wheat amino acids, sodium lauryl oat amino acids, sodium lauryl wheat amino acids, sodium cocoyl apple amino acids, sodium cocoyl methyl taurate and surfactin.

Clause 55. A composition according to clause 54, wherein the surfactant is selected from the list consisting of octoxynol-12, nonoxynol-15, octoxynol-16, nonoxynol-20, laureth-10, laureth 23, ceteth-10, ceteth-15, ceteth-20, oleth-15, oleth-20, C11-15 pareth-15, C11-15 pareth-20, C12-C13 pareth-23, ceteareth-25, isoceteth-20, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, potassium lauryl wheat amino acids, sodium lauryl wheat amino acids, sodium lauryl oat amino acids and surfactin.

Clause 56. A composition according to any one of clauses 1 to 55, wherein the lipid is a single pure component.

Clause 57. A composition according to clause 56, wherein the single pure component is a phosphatidyl choline.

Clause 58. A composition according to clause 57, wherein the phosphatidyl choline is DMPC, DLPC, DPPC or DSPC.

Clause 59.. A composition according to clause 58, wherein the phosphatidyl choline is DPPC.

Clause 60. A composition according to clause 58, wherein the phosphatidyl choline is DLPC.

Clause 61. A composition according to any one of clauses 1 to 55, wherein the lipid is a mixture of components.

Clause 62. A composition according to clause 61, wherein the lipid is a lipid mixture having a conserved acyl chain length.

Clause 63. A composition according to clause 62, wherein the conserved acyl chain length is 12, 14, 16, or 18 carbon atoms in length.

Clause 64. A composition according to clause 63, wherein the conserved acyl chain length is 12-16 carbon atoms in length.

Clause 65. A composition according to clause 61, wherein the lipid is a lipid mixture of at least 50% phospholipids having a single headgroup type by weight.

Clause 66. A composition according to clause 65, wherein the lipid is a lipid mixture of at least 75% phospholipids having a single headgroup type by weight.

Clause 67. A composition according to clause 66, wherein the lipid is a lipid mixture of at least 90% phospholipids having a single headgroup type by weight.

Clause 68. A composition according to any one of clauses 65 to 67, wherein the single headgroup type is a phosphatidylcholine.

Clause 69. A composition according to any one of clauses 61 to 68, wherein the lipid is a lipid extract of natural origin.

Clause 70. A composition according to clause 69, wherein the lipid extract is derived from egg.

Clause 71. A composition according to clause 69, wherein the lipid extract is derived from soy.

Clause 72. A composition according to clause 71, wherein the lipid extract comprises at least 92% phosphatidyl cholines, a maximum of 3% lyso-phosphatidyl cholines and a maximum of 2% oils; of which 14-20% of the acyl chains are palmityl, 3-5% stearyl, 8-12% oleic, 62-66% linoleic and 6-8% linolenic by weight.

Clause 73. A composition according to clause 71, wherein the lipid extract comprises: at least 90% hydrogenated phosphatidyl cholines, a maximum of 4% hydrogenated lyso-phosphatidyl cholines and a maximum of 2% oils and triglycerides; of which at least 80% of the acyl chains are stearyl and at least 10% are palmityl by weight.

Clause 74. A composition according to any one of clauses 1 to 73 which is in the form of an aqueous solution.

Clause 75. A composition according to any one of clauses 1 to 73 which is dried.

Clause 76. A composition according to any one of clauses 1 to 75 in which the ratio of surfactant to lipid is at least 0.5:1 on a weight basis.

Clause 77. A composition according to clause 76 in which the ratio of surfactant to lipid is at least 0.75:1 on a weight basis.

Clause 78. A composition according to clause 77 in which the ratio of surfactant to lipid is at least 1:1 on a weight basis.

Clause 79. A composition according to clause 78 in which the ratio of surfactant to lipid is at least 1.25:1 on a weight basis.

Clause 80. A composition according to clause 79 in which the ratio of surfactant to lipid is at least 1.5:1 on a weight basis.

Clause 81. A composition according to clause 80 in which the ratio of surfactant to lipid is at least 2.0:1 on a weight basis.

Clause 82. A composition according to any one of clauses 1 to 81 in which the ratio of surfactant to lipid is 10:1 or lower on a weight basis.

Clause 83. A composition according to clause 82 in which the ratio of surfactant to lipid is 7:1 or lower on a weight basis.

Clause 84. A composition according to clause 83 in which the ratio of surfactant to lipid is 5:1 or lower on a weight basis.

Clause 85. A composition according to clause 84 in which the ratio of surfactant to lipid is 3.5:1 or lower on a weight basis.

Clause 86. A composition according to clause 85 in which the ratio of surfactant to lipid is 3.0:1 or lower on a weight

basis.

Clause 87. A composition according to any one of clauses 1 to 86 which additionally comprises a co-surfactant.

Clause 88. A composition according to clause 87 wherein the co-surfactant has an HLB in the range 18 to 20.

Clause 89. A composition according to clause 87 which comprises a lyso-PC as co-surfactant.

Clause 90. A composition according to clause 89 which comprises a lyso-PC as co-surfactant in an amount equivalent to between 0.1-5% of the weight of lipid in the composition.

Clause 91. A composition according to clause 90 which comprises a lyso-PC as co-surfactant in an amount equivalent to between 0.75-1.5% of the weight of lipid in the composition.

Clause 92. A formulation comprising a composition according to any one of clauses 1 to 91 and an active agent.

Clause 93. A formulation according to clause 92, wherein the active agent is within the macromolecular assemblies.

Clause 94. A formulation according to either clause 92 or 93 wherein the active agent is an oil-soluble vitamin or oil soluble derivative of a water soluble vitamin.

Clause 95. A formulation according to clause 94 wherein the oil-soluble vitamin or oil soluble derivative of a water soluble vitamin is a member of the vitamin A family.

Clause 96. A formulation according to clause 94 wherein the oil-soluble vitamin or oil soluble derivative of a water soluble vitamin is an oil-soluble derivative of vitamin C.

Clause 97. A formulation according to clause 94 wherein the oil-soluble vitamin or oil soluble derivative of a water soluble vitamin is a member of the vitamin D family.

Clause 98. A formulation according to clause 94 wherein the oil-soluble vitamin or oil soluble derivative of a water soluble vitamin is a member of the vitamin E family.

Clause 99. A formulation according to clause 94 wherein the oil-soluble vitamin or oil soluble derivative of a water soluble vitamin is a member of the vitamin K family.

Clause 100. A formulation according to either clause 92 or 93 wherein the active agent is based upon a triterpenoid structure.

Clause 101. A formulation according to either clause 92 or 93 wherein the active agent is based upon a steroidal structure.

Clause 102. A formulation according to either clause 92 or 93 wherein the active agent is an antimicrobial agent.

Clause 103. A formulation according to either clause 92 or 93 wherein the active agent has a peptide structure.

Clause 104. A formulation according to either clause 92 or 93 wherein the active agent is a sunscreen.

Clause 105. A formulation according to either clause 92 or 93 wherein the active agent is flavour or fragrance.

Clause 106. A formulation according to any one of clauses 92 to 105, wherein the active agent is present in an amount of 0.001-50% of the weight of surfactant and lipid.

Clause 107. A formulation according to clause 106, wherein the active agent is present in an amount of 0.001-30% of the weight of surfactant and lipid.

Clause 108. A formulation according to clause 107, wherein the active agent is present in an amount of 1.1-25% of the weight of surfactant and lipid.

Clause 109. A formulation according to clause 108, wherein the active agent is present in an amount of 1.1% to less than 5% of the weight of surfactant and lipid.

Clause 110. A formulation according to clause 108, wherein the active agent is present in an amount of 5% to less than 10% of the weight of surfactant and lipid.

Clause 111. A formulation according to clause 108, wherein the active agent is present in an amount of 10% to less than 20% of the weight of surfactant and lipid.

Clause 112. A formulation according to either clause 106 or 107, wherein the active agent is present in an amount of 20% or more of the weight of surfactant and lipid.

Clause 113. A formulation according to any one of clauses 92 to 112 wherein the active agent is selected from TECA, Myristyl ester of L-pyrrolidone, lauric ester of L-pyrrolidone carboxylic acid, Ciclopirox olamine, Econazole nitrate, Red clover extract, Centella extract, Butcher's broom extract, Benzyl nicotinate, Piroctone olamine, acetyl hexapeptide-3, extract of *Ginkgo biloba,* Horse chestnut extract, Nettle extract, Aesculus extract, Yohimbine free base, Hydrocortisone, Salmeterol xinafoate, Progesterone, Devil's claw extract, Gatuline® Expression, extract of *Picea abies*, D-Camphor, Totara-8,11,13-trien-13-ol, extract of *Spilanthes acmella*, Undecylenoyl phenylalanine, extract of *Cimicifuga racemosa*, extract of *Boswellia serrata*, Sichuan pepper extract and Prickly ash extract.

Clause 114. A formulation according to any one of clauses 92 to 112 wherein the active agent is selected from 7-dehydrocholesterol, apricosal, ascorbyl palmitate, avobenzene, betamethasone 17-valerate, *Boswellia*, camphor, capsaicin, Cha-Plu extract, cholesterol sulphate, cholesterol, clobetasol propionate, clotrimazole, Cosmoperine, diclofenac, *Echinacea angustafolia, Echinacea purpurea,* Edemine, erythromycin sulphate, eserine, Eusolex 4360, Fougere, *Galanga, Ginkgo, Heliopsis* extract, hops tincture, hydrocortisone 17-butyrate, Japanese pepper extract, ketaconazole, ketoprofen, maca, melaleucol, minoxidil, naproxen, NDGA, neomycin sulphate, nystatin, octyl salicylate, PABA, PT-40, P-U, Questice CQ U/A, rosemary extract CG, rosmarinic acid (90%), soy isoflavones CG (50%), *Spilanthes* supercritical $CO_2$ extract, stearyl glycrrhetinate, tarragon extract, tasmanian pepper extract, THC CG, THC Ultra Pure, Unisex Bouquet, Unisol S-22, vitamin C palmitate, vitamin $D_3$, vitamin E.

Clause 115. A formulation according to any one of clauses 92 to 114 which is dried.

Clause 116. A formulation according to any one of clauses 92 to 114 which is in the form of an aqueous solution.

Clause 117. A formulation according to clause 116 wherein the quantity of active agent is in the range 0.001% to 20% of the total weight.

Clause 118. A formulation according to clause 117 wherein the quantity of active agent is in the range 0.001% to 15% of the total weight.

Clause 119. A formulation according to clause 118 wherein the quantity of active agent is in the range 0.05% to less than 0.1 % of the total weight.

Clause 120. A formulation according to clause 117 wherein the quantity of active agent is in the range 0.1 % to 10% of the total weight.

Clause 121. A formulation according to clause 120 wherein the quantity of active agent is in the range 0.1 % to less than 5% of the total weight.

Clause 122. A formulation according to clause 120 wherein the quantity of active agent is in the range 5% to less than 10% of the total weight.

Clause 123. A formulation according to either clause 117 or 118 wherein the quantity of active agent is 10% or more of the total weight.

Clause 124. An aqueous formulation according to any one of clauses 116 to 123 wherein the active agent is present in an amount which exceeds the solubility level of the active agent in aqueous solution with the same amount of surfactant alone.

Clause 125. A composition or formulation according to any one of clauses 1 to 124, wherein the macromolecular assemblies are less than 75 nm in diameter.

Clause 126. A composition or formulation according to clause 125, wherein the macromolecular assemblies are less than 50 nm in diameter.

Clause 127. A composition or formulation according to clause 126, wherein the macromolecular assemblies are less than 30 nm in diameter.

Clause 128. A composition or formulation according to clause 127, wherein the macromolecular assemblies are less than 20 nm in diameter.

Clause 129. A composition or formulation according to any one of clauses 1 to 128, wherein the macromolecular assemblies are at least 5 nm in diameter.

Clause 130. A composition or formulation according to clause 129, wherein the macromolecular assemblies are at least 6 nm in diameter.

Clause 131. A composition or formulation according to clause 130, wherein the macromolecular assemblies are at least 7 nm in diameter.

Clause 132. A composition or formulation according to clause 131, wherein the macromolecular assemblies are at least 8 nm in diameter.

Clause 133. A composition or formulation according to clause 132, wherein the macromolecular assemblies are at least 9 nm in diameter.

Clause 134. A composition or formulation according to clause 133, wherein the macromolecular assemblies are at least 10 nm in diameter.

Clause 135. An aqueous composition according to clause 74 or 76-91, or an aqueous formulation according to any one of clauses 116 to 134 which is substantially clear.

Clause 136. An aqueous composition or formulation according to clause 135 which is substantially clear and is stable.

Clause 137. An aqueous composition or formulation according to clause 135 or 136 which has a turbidity of less than 150 FNU.

Clause 138. An aqueous composition or formulation according to clause 137 which has a turbidity of less than 100 FNU.

Clause 139. An aqueous composition or formulation according to clause 138 which has a turbidity of less than 50 FNU.

Clause 140. An aqueous composition or formulation according to clause 139 which has a turbidity of less than 25 FNU.

Clause 141. An aqueous composition or formulation according to clause 140 which has a turbidity of less than 10 FNU.

Clause 142. A dried composition according to clause 75 or a dried formulation according to clause 115 which when reconstituted into water forms a substantially clear solution.

Clause 143. A dried composition or formulation according to clause 142 which when reconstituted into water forms a substantially clear and stable solution.

Clause 144. A dried composition or formulation according to either clause 142 or 143 which when reconstituted into water has a turbidity of less than 150 FNU.

Clause 145. A dried composition or formulation according to clause 144 which when reconstituted into water has a turbidity of less than 100 FNU.

Clause 146. A dried composition or formulation according to clause 145 which when reconstituted into water has a turbidity of less than 50 FNU.

Clause 147. A dried composition or formulation according to clause 146 which when reconstituted into water has a turbidity of less than 25 FNU.

Clause 148. A dried composition or formulation according to clause 147 which when reconstituted into water has a turbidity of less than 10 FNU.

Clause 149. An aqueous composition or aqueous formulation according to any one of the proceeding clauses which contains at least 60% water by weight.

Clause 150. An aqueous composition or aqueous formulation according to clause 149 which contains at least 70% water by weight.

Clause 151. An aqueous composition or aqueous formulation according to clause 150 which contains at least 80% water by weight.

Clause 152. An aqueous composition or aqueous formulation according to clause 151 which contains at least 90% water by weight.

Clause 153. A cosmetic preparation comprising a formulation according to any one of clauses 92 to 152 and a cosmetically acceptable carrier or excipient.

Clause 154. A pharmaceutical preparation comprising a formulation according to any one of clauses 92 to 152 and a pharmaceutically acceptable carrier or excipient.

Clause 155. A composition, formulation or preparation according to any one of clauses 1 to 154 which is substantially free of short chain alcohols.

Clause 156. A composition, formulation or preparation according to clause 155, which contains less than 10% by weight of short chain alcohols.

Clause 157. A composition, formulation or preparation according to any one of clauses 1 to 156, which is substantially free of sterols.

Clause 158. A composition, formulation or preparation according to clause 157, which contains less than 10% by weight of sterols.

Clause 159. A composition, formulation or preparation according to any one of clauses 1 to 158, which is substantially free of propylene glycol or polyethylene glycol.

Clause 160. A composition, formulation or preparation according to clause 159, which contains less than 10% by weight of propylene glycol or polyethylene glycol.

Clause 161. A composition, formulation or preparation according to any one of clauses 1 to 160, which is substantially free of triglycerides.

Clause 162. A composition, formulation or preparation according to clause 161, which contains less than 10% by weight of triglycerides.

Clause 163. A composition, formulation or preparation according to any one of clauses 1 to 162, which is substantially free of polypeptides.

Clause 164. A composition according to clause 163, which contains less than 10% by weight of polypeptides.

Clause 165. A composition, formulation or preparation according to any one of clauses 1 to 164 which comprises less than 20% cholesterol by dry weight.

Clause 166. A composition, formulation or preparation according to any one of clauses 1 to 166 which is substantially free of propylene glycol or polyethylene glycol.

Clause 167. Use of a composition according to any one of clauses 1 to 91, 125 to 152 or 155 to 166 as a solubilising agent.

Clause 168. Use according to clause 167, in the solubilisation of an active agent.

Clause 169. Use according to clause 167, in the solubilisation of a membrane protein or peptide.

Clause 170. A method for the screening of candidate agents for interaction with a membrane protein or peptide comprising the steps of:

(i) solubilising a membrane protein or peptide in a composition according to any one of clauses 1 to 91, 125 to 152 or 155 to 166;
(ii) testing a candidate agent to determine whether it interacts with the solubilised membrane protein or peptide.

Clause 171. A method for the structural investigation of a membrane protein or peptide comprising the steps of:

(i) solubilising a membrane protein or peptide in a composition according to any one of clauses 1 to 91, 125 to 152 or 155 to 166;
(ii) investigating the structure of said membrane protein or peptide.

Clause 172. A method for the manufacture of a composition according to any one of clauses 1 to 166 comprising the steps of:

(i) Preparing an aqueous solution of a surfactant;
(ii) Preparing an aqueous lipid emulsion; and
(iii) Mixing the aqueous lipid emulsion and aqueous solution of surfactant;
such that macromolecular assemblies are formed.

Clause 173. A method for the production of a composition according to any one of clauses 1 to 166 comprising the steps of:

(i) Preparing an aqueous solution of surfactant;
(ii) Preparing an aqueous emulsion of lipid;
(iii) Preparing an aqueous emulsion of active agent; and
(iv) Mixing the aqueous aqueous solution of surfactant, the aqueous emulsion of lipid and the aqueous emulsion of active agent;
such that macromolecular assemblies are formed.

Clause 174. A method for the delivery of an active agent comprising the topical administration of a formulation or preparation according to any one of clauses.

Clause 175. A hydrogel patch comprising a composition, formulation or preparation according to any one of clauses 1 to 166.

Clause 176. A kit of parts for the preparation of a compositon according to any one of clauses 1 to 75, comprising a lipid and a surfactant.

Clause 177. A method for the production of a composition according to any one of clauses 1 to 75 comprising the steps of:

(i) mixing the surfactant and lipid in a short chain alcohol;
(ii) removing the alcohol;
such that macromolecular assemblies are formed.

The following Examples are non-limiting and are provided to illustrate the preparation and use of compositions

according to the present invention such that a person skilled in the art may more readily appreciate the nature of the invention and put the invention into practical effect.

## COMPARATIVE EXAMPLES

### Comparative Example 1 - The ability of common surfactants to solubilise lipids

The ability of four commonly used surfactants to solubilise a number of lipid mixtures was tested for the purpose of comparison with the solubilising compositions of the present invention.

Method

The appropriate quantity of lipid and surfactant was added to water, which was then warmed to approximately 50 °C and stirred until a uniform emulsion was formed. The mixture was then homogenised for 10 minutes.

Percentage values specified in this experiment refer to the weight of the component in question as a proportion of the total weight of the composition.

Once the mixtures were prepared they were visually examined to determine whether the surfactant component had solubilised the lipid component in the aqueous medium. The clarity of a mixture was categorised as being clear if there was no significant visible opacity to the naked eye, whereas a mixture was categorised as cloudy if there was significant visible disruption to the passage of light.

*Surfactants*

Sodium dodecyl sulphate (CAS Ref 151-21-3), also known as sodium lauryl sulphate and often referred to by the acronym SDS, is one of the most widely used anionic surfactants, for example it is used in many general purpose cleaning agents. SDS was utilised as a laboratory reagent grade powder.

Mackanate DC30 is produced by the McIntyre Group Ltd (USA) (CAS Ref 68784-08-7) and is known by the generic name disodium dimethicone copolyol sulphosuccinate. Mackanate is a mild anioinic surfactant used in personal care cleaning agents. Mackanate was utilised as a clear liquid at 30% concentration.

Lutrol® F127 (CAS Ref 9003-11-6), known by the generic name poloxamer 407, is produced by BASF and is a polyoxyethylene/polyoxypropylene block copolymer surfactant. F127 is a non-ionic polymeric surfactant, possessing 70% polyethylene oxide content, average molecular weight of 12,700 and supplied as a powder. Having a low dermal and ocular irritancy, F127 is of widespread use in personal care applications.

Lyso-phosphatidyl choline (CAS Ref 9008-30-4), is available under the tradename S LPC from Lipoid GmbH. Structurally related to phosphatidylcholines, it differs in that it contains only one fatty acid chain, resulting in a much higher surface activity. S LPC is used as a mild emulsifier in personal care applications. S LPC used herein was at 93.9% purity and supplied as a powder.

*Lipids*

Phospholipon® 90 H, referred to herein by the abbreviation 90H, available from Phospholipid GmbH (Germany), is a hydrogenated soy lecithin extract of at least 90% phosphatidylcholine content and is approved for pharmaceutical and cosmetic use. It is generally used as an emulsifier and is known to form liposomes.

Results

Table 1 below summarises the results of the experiment.

Table 1 - The ability of common surfactants to solubilise lipids

| Surf. | Surf. % w/w | Lipid | Lipid % w/w | Clarity |
|---|---|---|---|---|
| SDS | 5.0 | 90H | 1.0 | Clear |
| | 2.5 | 90H | 1.0 | Cloudy |
| Mackanate | 5.0 | 90H | 1.0 | Cloudy |
| | 2.5 | 90H | 1.0 | Cloudy |

(continued)

| Surf. | Surf. % w/w | Lipid | Lipid % w/w | Clarity |
|---|---|---|---|---|
| F127 | 5.0 | 90H | 1.0 | Cloudy |
| | 2.5 | 90H | 1.0 | Cloudy |
| S LPC | 5.0 | 90H | 1.0 | Cloudy |
| | 2.5 | 90H | 1.0 | Cloudy |

As can be seen from the data in Table 1, conventional surfactants at a concentration of 2.5% w/w are not capable of solubilising lipids at a concentration of 1.0% w/w to form clear and colourless solutions. Even at 5.0% w/w the surfactants are in general unable to solubilise the lipid, the exception being SDS which is well recognised both for its capabilities as a surfactant and its irritant properties.

**Comparative Example 2 - The ability of common surfactants to solubilise active agents**

The ability of five commonly used surfactants to solubilise an exemplary active agent having poor water solubility was tested for the purpose of comparison with the solubilising compositions of the present invention.

Method

The appropriate quantity of surfactant and active agent was added to water, which was then warmed to approximately 50 °C and stirred. The mixture was then homogenised for 10 minutes.

Percentage values specified in this experiment refer to the weight of the component in question as a proportion of the total weight of the composition.

Once the mixtures were prepared they were visually examined to determine whether the surfactant component had solubilised the active agent in the aqueous medium. The clarity of a mixture was categorised as being clear if there was no significant visible opacity to the naked eye, whereas a mixture was categorised as cloudy if there was significant visible disruption to the passage of light.

For a quantitative analysis of the clarity of aqueous solutions of surfactant and active, certain samples were examined using a turbidity meter (Nephla, from Hach-Lange). The turbidity meter was calibrated prior to use, with two known standards (0 and 40 FNU).

*Surfactants*

The four surfactants SDS, Mackanate, F127 and S LPC were as described above in Comparative Example 1.

Brij 35P (Laureth-23) was supplied by Uniqema/ICI (CAS Ref 9002-92-0). Brij 35P is a pharmaceutical grade of polyoxethyleneglycol-23 lauryl ether, which is sold primarily as a solubilising agent.

*Active agent*

Titrated extract of *Centella asiatica*, referred to herein as TECA, is available from Bayer Sant6 Familiale. TECA is a mixture of 60% free genins (asiatic acid and madecassic acid) and 40% asiaticoside, and is of use in regulating collagen synthesis, wound healing, anti-wrinkle, toning and anti-cellulite treatments. Pharmaceutical grade (95% purity) was utilised, supplied as a powder.

Results

Table 2 below summarises the results of the experiment.

Table 2 - The ability of common surfactants to solubilise active agents

| Surf. | Surf. % w/w | Active Agent | Active Agent % w/w | Clarity | Turbidity (FNU) |
|---|---|---|---|---|---|
| SDS | 5 | TECA | 0.5 | Cloudy | - |
| MACKANATE | 5 | TECA | 0.5 | Cloudy | - |
| F127 | 5 | TECA | 0.5 | Cloudy | - |

(continued)

| Surf. | Surf. % w/w | Active Agent | Active Agent % w/w | Clarity | Turbidity (FNU) |
|---|---|---|---|---|---|
| S LPC | 5 | TECA | 0.5 | Cloudy | - |
| Brij 35P | 2.5 | TECA | 0.5 | Cloudy | >150 |
| | 3.5 | TECA | 0.5 | Cloudy | >150 |

Exemplary conventional surfactants, at the tested concentrations, were unable to solubilise an exemplary active agent which has a poor water solubility.

**Comparative Example 3 - The ability of lipids to solubilise active agents**

The ability of lipid compositions to solubilise an exemplary active agent having poor water solubility was tested for the purpose of comparison with the solubilising compositions of the present invention.

Method

The appropriate quantity of lipid and active agent was added to water, which was then warmed to approximately 50 °C and stirred until a uniform emulsion was formed. The emulsion was then homogenised for 10 minutes.

Percentage values specified in this experiment refer to the weight of the component in question as a proportion of the total weight of the composition.

Once the mixtures were prepared they were visually examined to determine whether the lipid component had solubilised the active agent in the aqueous medium. The clarity of a mixture was categorised as being clear if there was no significant visible opacity to the naked eye, whereas a mixture was categorised as cloudy if there was significant visible disruption to the passage of light.

For a quantitative analysis of the clarity of aqueous solutions of lipid and active, certain samples were examined using a turbidity meter (Nephla, from Hach-Lange). The turbidity meter was calibrated prior to use, with two known standards (0 and 40 FNU).

*Lipids*

90H was as described above in Comparative Example 1.

Emulmetik 930 (Em930) is a purified phosphatidylcholine of soyabean origin for the cosmetic industry (containing at least 92% phosphatidylcholine). Em930 is available from Lucas Meyer Cosmetics SA.

*Active agent*

The exemplary active agent, TECA, was as described in Comparative Example 2.

Results

Table 3 below summarises the results of the experiment.

Table 3 - The ability of lipids to solubilise active agents

| Lipid | Lipid % w/w | Active Agent | Active Agent % w/w | Clarity | Turbidity (FNU) |
|---|---|---|---|---|---|
| 90H | 1.0 | TECA | 0.5 | Cloudy | >150 |
| 90H | 2.0 | TECA | 0.5 | Cloudy | - |
| 90H | 3.5 | TECA | 0.5 | Cloudy | >150 |
| Em930 | 1.0 | TECA | 0.5 | Cloudy | - |

As would be expected, the exemplary lipid compositions did not interact with TECA at the tested concentrations to form clear and colourless aqueous solutions.

## EXAMPLES OF THE INVENTION

### Example 1 - The use of surfactants and lipid in the formation of macromolecular complexes of the invention

A range of surfactants were tested for their suitability to be used in the present invention, as indicated by their ability to solubilise a lipid mixture through the formation of macromolecular complexes.

Method

Each surfactant was tested using a standard lipid emulsion containing 1% 90H and ca. 0.01% S LPC cosurfactant (incorporated in the form of 0.05% SL 80-3).

A stock emulsion of lipid was prepared at double the desired final concentration (i.e. containing 2% 90H and 0.1 % SL 80-3). Briefly, to the appropriate volume of warm water (ca. 60 °C), SL 80-3 was added. Heating and stirring was maintained for approximately 15 minutes before the mixture was homogenised for around 1 minute at 13,000 RPM (POLYTRON PT 3100 Homogeniser). 90H was then added gradually, with heating and stirring maintained throughout and for a further 45 minutes after completion. The mixture was then homogenised for around 3 minutes at 15,000 RPM followed by 1 minute at 26,000 RPM.

A stock solution of each surfactant was prepared at double the desired final concentration (i.e. a 5% stock, for a 2.5% final concentration) by mixing of the surfactant with the appropriate volume of water. After mixing the solution was stirred and heated to around 60-70 °C.

The required quantity of warm lipid emulsion was slowly added to the warm surfactant solution while stirring with the temperature maintained.

Samples are then allowed to cool to room temperature before being analysed. For a quantitative analysis of the clarity of aqueous solutions of surfactant and lipid, samples were examined using a turbidity meter (Nephla, from Hach-Lange). The turbidity meter was calibrated prior to use, with two known standards (0 and 40 FNU).

*Surfactants*

The HLB values in the Tables below are based on a combination of the values reported by the manufacturer for the commercial product and those given in the literature (e.g. McCutcheon's Volume 1: Emulsifiers & Detergents, International Edition, MC Publishing Company, Glen Rock, NJ, USA, 2005; Handbook of Industrial Surfactants, M Ash & I Ash, Gower Publishing Company, Aldershot, England, 1993). A rough average of reported values is given. In some cases, details of the HLB are not available.

Surfactants utilised in this experiment are:

- 435473 (Poly(propylene glycol)-block-poly(ethylene glycol)-block-poly(propylene glycol)) was supplied by Sigma-Aldrich Ltd. (UK) CAS: 9003-11-6
- 460141 (Polyethylene glycol 400 monolaurate) was supplied by Sigma-Aldrich Ltd. (UK) 9004-81-3
- 460176 (Polyethylene glycol monooleate) was supplied by Sigma-Aldrich Ltd. (UK) 9004-96-0
- 74680 (Laureth-8) was supplied by Sigma-Aldrich Ltd. (UK) CAS: 3055-98-9
- Akyporox CO 400 (PEG-40 Hydrogenated castor oil) was supplied by Kao Chemicals GmbH. (Germany) CAS: 61788-85-0
- Amilite® GCS-11 (Sodium cocoyl glycinate) was supplied by Ajinamto Co. Inc. (Japan)
- Aminofect® (Surfactin Peptide-amide/ester) was supplied by Showa Denko Co. Ltd. (Japan)
- Aminofoam WOR (Potassium lauryl wheat amino acids) was supplied by Croda Chemicals Ltd. (UK) CAS: 162353-60-8
- Amisoft® CS-11 (F) (Sodium cocoyl glutamate) was supplied by Ajinamto Co. Inc. (Japan)
- Amisoft® GS-11 P(F) (Sodium stearoyl glutamate / Sodium cocoyl glutamate (mix)) was supplied by Ajinamto Co. Inc. (Japan)
- Amisoft® HS-11 P(F) (Sodium stearoyl glutamate) was supplied by Ajinamto Co. Inc. (Japan)
- Amisoft® LS-11(F) (Sodium lauroyl glutamate) was supplied by Ajinamto Co. Inc. (Japan)
- Amisoft® MS-11(F) (Sodium myristoyl glutamate) was supplied by Ajinamto Co. Inc. (Japan)
- Arlasilk™ EFA (Phospholipid EFA) was supplied by Uniqema/ICI (Imperial Chemical Industries PLC)
- Arlasilk™ PTC (Phospholipid PTC) was supplied by Uniqema/ICI (Imperial Chemical Industries PLC)
- Arlasolve™ 200N (Isoceteth-20) was supplied Uniqema/ICI (Imperial Chemical Industries PLC)
- BB-20 (Beheneth-20) was supplied Nikko Chemicals Co. Ltd. (Japan) CAS: 26636-40-8
- BB-30 (Beheneth-30) was supplied Nikko Chemicals Co. Ltd. (Japan) CAS: 26636-40-8
- BC-15TX (Ceteth-15) was supplied Nikko Chemicals Co. Ltd. (Japan) CAS: 9004-95-9

- BC-20TX (Ceteth-20) was supplied Nikko Chemicals Co. Ltd. (Japan) CAS: 9004-95-9
- Benzoic acid was supplied by Sigma-Aldrich Ltd. (UK) CAS: 65-85-0
- BO-15V (Oleth-15) was supplied Nikko Chemicals Co. Ltd. (Japan) CAS: 9004-98-2
- Brij 35 (Laureth-23) was supplied by Sigma-Aldrich Ltd. (UK) CAS: 9002-92-0
- Brij 35P (Laureth-23) was supplied by Uniqema/ICI (Imperial Chemical Industries PLC) CAS: 9002-92-0
- Brij 56 (Ceteth-10) was supplied by Sigma-Aldrich Ltd. (UK) CAS: 9004-95-9
- Brij 58 (Ceteth-20) was supplied by Sigma-Aldrich Ltd. (UK) CAS: 9004-95-9
- Brij 58P (Ceteth-20) was supplied by Uniqema/ICI (Imperial Chemical Industries PLC) CAS: 9002-95-9
- Brij 76 (Steareth-10) was supplied by Uniqema/ICI (Imperial Chemical Industries PLC)
- Capric acid (Decanoic acid) was supplied by A&E Connock (Perfumery & Cosmetics) Ltd. (UK) CAS: 334-48-5
- Caproic acid (Hexanoic acid) was supplied by A&E Connock (Perfumery & Cosmetics) Ltd. (UK) CAS: 142-62-1
- Caprylic acid (Octanoic acid) was supplied by A&E Connock (Perfumery & Cosmetics) Ltd. (UK) CAS: 124-07-2
- Cithrol 10MS (PEG-20 stearate) was supplied by Croda Chemicals Ltd. (UK) CAS: 9004-99-3
- Crodafos MCA (Cetyl phosphate) was supplied Croda Chemicals Ltd. (UK) CAS: 3539-43-3
- Crodafos SG (PPG-5 Ceteth-10) was supplied by Croda Chemicals Ltd. (UK) CAS: 73361-29-2
- Crodet S40LD (PEG-40 stearate) was supplied by Croda Chemicals Ltd. (UK) CAS: 9004-99-3
- Cromul EM1207 (Steareth-21) was supplied by Croda Chemicals Ltd. (UK) CAS: 9005-00-9
- Decaglyn 1-L (Polyglyceryl-10 laurate) was supplied Nikko Chemicals Co. Ltd. (Japan)
- Decanoic acid (Capric acid) was supplied by Sigma-Aldrich Ltd. (UK) CAS: 334-48-5
- Dermofeel G 10L (Polyglyceryl-10 laurate) was supplied by Gemro Products Ltd (UK)
- Dermofeel G 6CY (Polyglyceryl-6 caprylate) was supplied by Gemro Products Ltd (UK)
- DHC-30 (Dihydrocholeth-30) was supplied Nikko Chemicals Co. Ltd. (Japan)
- Dodecanoic acid (Lauric acid) was supplied by Sigma-Aldrich Ltd. (UK) CAS: 143-07-7
- Empilan® NP20 (Nonoxynol-20) was supplied by Uniqema/ICI (Imperial Chemical Industries PLC)
- Empilan® NP30 (Nonoxynol-30) was supplied by Uniqema/ICI (Imperial Chemical Industries PLC)
- Emulgin BA 25 (Beheneth-25) was supplied by Cognis Iberia s.l. (Spain)
- Emulgin CS-50 (Ceteareth-50) was supplied by Cognis Iberia s.l. (Spain) CAS: 68439-49-6
- Genapol C100 (Coceth-10) was supplied Clariant International Ltd. CAS: 61791-13-7
- Genapol C200(Coceth-20) was supplied Clariant International Ltd. CAS: 61791-13-7
- Genapol LA030 (Laureth-3) was supplied Clariant International Ltd. CAS: 68551-12-2
- Genapol LA070 (Laureth-7) was supplied Clariant International Ltd. CAS: 68551-12-2
- Genapol T800 (Ceteareth-80)was supplied Clariant International Ltd. CAS: 68439-49-6
- Glucamate™ DOE-120 (PEG-120 methyl glucose dioleate) was supplied by Chemron Corp. (Belgium)
- Glucamate™ SSE-20 (PEG-20 methyl glucose sesquistearate) was supplied by Chemron Corp. (Belgium) CAS: 68389-70-8
- Hexanoic acid (Caproic acid) was supplied by Sigma-Aldrich Ltd. (UK) CAS: 142-62-1
- Hostacerin DGMS (Polyglyceryl-2-stearate) was supplied Clariant International Ltd. CAS: 12694-22-3
- Igepal CA-720 (Octoxynol-12.) was supplied by Sigma-Aldrich Ltd. (UK) CAS: 9002-93-1
- Igepal CO-890 (Octoxynol-40) was supplied by Sigma-Aldrich Ltd. (UK) CAS: 9002-93-1
- Incronam 30 (Cocamidopropyl betaine) was supplied by Croda Chemicals Ltd. (UK)
- L.A.S. (PEG-8 caprylic/capric glycerides) was supplied by Gattefosse SAS (France)
- Lincol ORH 40/S (PEG-40 Hydrogenated castor oil) was supplied by Eigenmann & Veronelli SPA (Italy)
- Lutrol® F127 (Poloxamer 407) was supplied by BASF (Germany) CAS: 106392-12-5
- Mackanate DC-50 (Dimethicone copolyol sulfosuccinate) was supplied by McIntyre Group Ltd. (USA)
- Mandelic acid was supplied by Sigma-Aldrich Ltd. (UK) CAS: 90-64-2
- Marilpal 1618/11 (Ceteareth-11) was supplied by Sasol UK Ltd. CAS: 68439-49-6
- Monasil PCA (Polysiloxy carboxylic acid) was supplied by Mona Industries (USA)
- Monasil PLN (Polysiloxy linoleyl phospholipid) was supplied by Mona Industries (USA)
- Myrj 52S (PEG-40 stearate) was supplied by Uniqema/ICI (Imperial Chemical Industries PLC) CAS: 9004-99-3
- Octanoic acid (Caprylic acid) was supplied by Sigma-Aldrich Ltd. (UK) CAS: 124-07-2
- Oleic acid (octadecenoic acid) was supplied by Sigma-Aldrich Ltd. (UK) CAS: 112-80-1
- Oramix CG 110 (Caprylyl/Capryl glucoside) was supplied by Seppic S.A. (France)
- Oramix NS-10 (Decyl glucoside) was supplied by Seppic S.A. (France)
- P2393 (Trideceth-10) was supplied by Sigma-Aldrich Ltd. (UK) CAS: 24938-91-8
- P9641 (Laureth-9) was supplied by Sigma-Aldrich Ltd. (UK) CAS: 3055-99-0
- P9769 (Laureth-10) was supplied by Sigma-Aldrich Ltd. (UK) CAS: 6540-99-4
- Palmitic acid (Hexadecanoic acid) was supplied by Sigma-Aldrich Ltd. (UK) CAS: 57-10-3
- Pationic 138C (Sodium lauryl lactylate) was supplied by Rita Corporation (USA) CAS: 13557-75-0

- PBC-34 (PPG-4 ceteth-20) was supplied Nikko Chemicals Co. Ltd. (Japan) CAS: 9087-53-0
- Pecosil® PS-100 (Dimethicone PEG-7 phosphate) was supplied by Phoenix Chemical Inc. (USA) CAS: 132207-31-9
- Plantacare® 1200UP (C8-16 mainly C12 glucoside/Lauryl glucoside) was supplied by Cognis Iberia s.l. (Spain) CAS: 110615-47-9
- Plantacare® 2000UP (C8-16 mainly C8 glucoside/Decyl glucoside) was supplied by Cognis Iberia s.l. (Spain) CAS: 68515-73-1
- Plantacare® 810UP (C8-16 mainly C8 glucoside/Capryly glucoside) was supplied by Cognis Iberia s.l. (Spain) CAS: 68515-73-1
- Plantacare® 818UP (C8-16 mainly C12 glucoside/Coco glucoside) was supplied by Cognis Iberia s.l. (Spain) CAS: 141464-42-8
- Plantapon® ACG 35 (Disodium cocoyl glutamate) was supplied by Cognis Iberia s.l. (Spain) CAS: 68187-30-4
- Plantapon® ACG 50 (Sodium cocoyl glutamate) was supplied by Cognis Iberia s.l. (Spain) CAS: 68187-32-6
- Plantapon® S (Sodium cocoyl hydrolyzed wheat protein glutamate) was supplied by Cognis Iberia s.l. (Spain) CAS: 68188-38-5
- Potassium oleate was supplied A&E Connock (UK) CAS: 143-18-0
- Procol CS-20 (Ceteareth-20) was supplied by Protameen Chemicals Inc. (USA) CAS: 68439-49-6
- Procol CS-30 (Ceteareth-30) was supplied by Protameen Chemicals Inc. (USA) CAS: 9004-95-9
- Procol IS-20 (Isosteareth-20) was supplied by Protameen Chemicals Inc. (USA) CAS: 52292-17-8
- Procol LA-4 (Laureth-4) was supplied by Protameen Chemicals Inc. (USA) CAS: 5274-68-0
- Procol OA-20 (Oleth-20) was supplied by Protameen Chemicals Inc. (USA) CAS: 9004-98-2
- Procol OA-20SP (Oleth-20 Special) was supplied by Protameen Chemicals Inc. (USA) CAS: 9004-98-2
- Procol OA-5 (Oleth-5) was supplied by Protameen Chemicals Inc. (USA) CAS: 9004-98-2
- Procol OA-5SP (Oleth-5 Special) was supplied by Protameen Chemicals Inc. (USA) CAS: 9004-98-2
- Procol SA-20 (Steareth-20) was supplied by Protameen Chemicals Inc. (USA) CAS: 9005-00-9
- Protachem AWS-100 (PPG-5 ceteth-20) was supplied by Protameen Chemicals Inc. (USA) CAS: 9087-53-0
- Protachem DGS (PEG-2 stearate) was supplied by Protameen Chemicals Inc. (USA) CAS: 111-60-4
- Protachem SMO (Sorbitan oleate) was supplied by Protameen Chemicals Inc. (USA) CAS: 1338-43-8
- Protachem SMP (Sorbitan palmitate) was supplied by Protameen Chemicals Inc. (USA) CAS:26266-57-9
- Protachem SMS (Sorbitan stearate) was supplied by Protameen Chemicals Inc. (USA) CAS: 1338-41-6
- Protamate 1000 DPS (PEG-20 stearate) was supplied by Protameen Chemicals Inc. (USA) CAS: 9004-99-3
- Protamate 1540-DPS (PEG-40 stearate) was supplied by Protameen Chemicals Inc. (USA) CAS: 9004-99-3
- Protamate 200 DPS (PEG-4 stearate) was supplied by Protameen Chemicals Inc. (USA) CAS: 9004-99-3
- Protamate 200-OC (PEG-4 oleate) was supplied by Protameen Chemicals Inc. (USA) CAS: 9004-96-0
- Protamate 300 DPS (PEG-6 stearate) was supplied by Protameen Chemicals Inc. (USA)
- Protamate 400-DO (PEG-8 dioleate) was supplied by Protameen Chemicals Inc. (USA) CAS: 9005-07-6
- Protamate 4400-DPS (PEG-100 stearate) was supplied by Protameen Chemicals Inc. (USA) CAS: 9004-97-3
- Protamate 6000-DS (PEG-150 distearate) was supplied by Protameen Chemicals Inc. (USA) CAS: 9005-08-7
- Protasorb L-20 (Polysorbate-20) was supplied by Protameen Chemicals Inc. (USA) CAS: 68154-33-6
- Protasorb O-20 (Polysorbate-80) was supplied by Protameen Chemicals Inc. (USA) CAS: 9005-65-6
- Protasorb P-20 (Polysorbate-40) was supplied by Protameen Chemicals Inc. (USA) CAS: 9005-66-7
- Protasorb S-20 (Polysorbate 60) was supplied by Protameen Chemicals Inc. (USA) CAS: 9005-67-8
- Proteol™ LW 30 (Sodium lauryl wheat amino acids) was supplied by Seppic S.A. (France)
- Proteol™ O.A.T. (Sodium lauryl oat amino acids) was supplied by Seppic S.A. (France)
- Proteol™ APL (Sodium cocoyl apple amino acids) was supplied by Seppic S.A. (France)
- Protox C-15 (PEG-15 cocamine) was supplied by Protameen Chemicals Inc. (USA) CAS: 61791-14-8
- Protox C-5 (PEG-5 cocamine) was supplied by Protameen Chemicals Inc. (USA) CAS: 68439-72-5
- Salicylic acid was supplied by Sigma-Aldrich Ltd. (UK) CAS: 69-72-7
- Sisterna SP01-C (Sucrose distearate) was supplied by Sisterna C.V. (Netherlands) CAS: 27195-16-0
- Sisterna SP30-C (Sucrose distearate) was supplied by Sisterna C.V. (Netherlands) CAS: 27195-16-0
- Sisterna SP50-C (Sucrose stearate) was supplied by Sisterna C.V. (Netherlands) CAS: 25168-73-4
- Sisterna SP70-C (Sucrose stearate) was supplied by Sisterna C.V. (Netherlands) CAS: 25168-73-4
- Sodium cholate was supplied by Sigma-Aldrich Ltd. (UK)
- Sodium deoxycholate was supplied by Sigma-Aldrich Ltd. (UK)
- Sodium dodecyl sulphate (SDS) was as described in Comparative Example 1.
- Sodium laureth sulphate (EMAL® 228 D/JM) was supplied by Kao Chemicals GmbH, Emmerich (Germany)
- Solan E50 (PEG-75 lanolin) was supplied by Croda Chemicals Ltd. (UK) CAS: 61790-81-6
- Somepan T25 (Sodium cocoyl methyl taurate) was supplied by Seppic S.A. (France)
- Surfac OP5 (Octoxynol-5) was supplied by Surfachem Ltd. (UK) CAS: 9002-93-1

- Surfac OP30 (Octoxynol-30) was supplied by Surfachem Ltd. (UK) CAS: 9002-93-1
- Surfhope C-1215L (Sucrose laurate) was supplied by Mitsubishi Corporation (Japan) CAS: 37266-93-6
- Surfhope C-1216 (Sucrose myristate) was supplied by Mitsubishi Corporation (Japan) CAS: 37266-93-6
- Surfhope C-1416 (Sucrose myristate) was supplied by Mitsubishi Corporation (Japan) CAS: 9042-71-1
- Surfhope C-1615 (Sucrose palmitate) was supplied by Mitsubishi Corporation (Japan) CAS: 39300-95-3
- Surfhope C-1616 (Sucrose palmitate) was supplied by Mitsubishi Corporation (Japan) CAS: 39300-95-3
- Surfhope C-1715 (Sucrose oleate) was supplied by Mitsubishi Corporation (Japan) CAS: 52683-61-1
- Surfhope C-1715L (Sucrose oleate) was supplied by Mitsubishi Corporation (Japan) CAS: 52683-61-1
- Surfhope C-1815 (Sucrose stearate) was supplied by Mitsubishi Corporation (Japan) CAS: 37318-31-3
- Surfhope C-1816 (Sucrose stearate) was supplied by Mitsubishi Corporation (Japan) CAS: 37318-31-3
- Sympatens-AIC/200 (Isoceteth-20) was supplied by Kolb Distribution Ltd. (Switzerland) CAS: 9004-95-9
- Sympatens-AS/1000G (Steareth-100) was supplied by Kolb Distribution Ltd. (Switzerland) CAS: 9005-00-9
- Sympatens-BS/300G (PEG-30 stearate) was supplied by Kolb Distribution Ltd. (Switzerland) CAS: 9004-99-3
- Sympatens-BS/500G (PEG-50 stearate) was supplied by Kolb Distribution Ltd. (Switzerland) CAS: 9004-99-3
- Sympatens-NP/090 (Nonoxynol-9) was supplied by Kolb Distribution Ltd. (Switzerland) CAS: 9016-45-9
- Sympatens-NP/150 (Nonoxynol-15) was supplied by Kolb Distribution Ltd. (Switzeralnd) CAS: 9016-45-9
- Symperonic PE/L44 (Poloxamer 124) was supplied by Uniqema/ICI (Imperial Chemical Industries PLC)
- Synperonic PE/F68 (Poloxamer 188) was supplied by Uniqema/ICI (Imperial Chemical Industries PLC)
- Synperonic PE/F87 (Poloxamer 237) was supplied by Uniqema/ICI (Imperial Chemical Industries PLC)
- Synperonic PE/L64 (Poloxamer 184) was supplied by Uniqema/ICI (Imperial Chemical Industries PLC)
- Tergitol® 15-S-5 (C11-15 Pareth-5) was supplied by Surfachem Ltd. (UK) CAS: 068131-40-8
- Tergitol® 15-S-7 (C11-15 Pareth-7) was supplied by Surfachem Ltd. (UK) CAS: 068131-40-8
- Tergitol® 15-S-12 (C11-15 pareth-12) was supplied by The Dow Chemical Company (US) CAS: 84133-50-6
- Tergitol® 15-S-15 (C11-15 pareth-15) was supplied by The Dow Chemical Company (US) CAS: 84133-50-6
- Tergitol® 15-S-20 (C11-15 pareth-20) was supplied by The Dow Chemical Company (US) CAS: 84133-50-6
- Tergitol® 15-S-20(80%) (C11-15 pareth-20) was supplied by The Dow Chemical Company (US) CAS: 84133-50-6
- Tergitol® 15-S-40 (C11-15 pareth-40) was supplied by The Dow Chemical Company (US) CAS: 68131-40-8
- Tergitol® NP-10 (Nonoxynol-10) was supplied by Sigma-Aldrich Ltd. (UK) CAS: 127087-87-0
- Triton X-100 (Octoxynol-9) was supplied by Sigma-Aldrich Ltd. (UK) CAS: 9002-93-1
- Triton X-165 (Octoxynol-16) was supplied by Sigma-Aldrich Ltd. (UK) CAS: 9002-93-1
- Triton X-405 (Octoxynol-40) was supplied by Sigma-Aldrich Ltd. (UK) CAS: 9002-93-1
- Volpo CS20 (Ceteareth-20) was supplied by Croda Chemicals Ltd. (UK) CAS: 68439-49-6
- Volpo CS25 (Ceteareth-25) was supplied by Croda Chemicals Ltd. (UK) CAS: 68439-49-6
- Volpo L23 (C12-13 pareth-23) was supplied by Croda Chemicals Ltd. (UK) CAS: 66455-14-9
- Volpo N10 (Oleth-10) was supplied by Croda Chemicals Ltd. (UK) CAS: 9004-98-2

*Lipids*

90H was as described in Comparative Example 1.

SL 80-3 is a purified soy extract containing 54% phosphatidyl choline, though it may be noted for its relatively high content of lyso-phosphatidyl choline (S LPC). It is available from Lipoid GmbH.

Results

Tables 4a-4v below summarise the results of the experiment.

Table 4a - The use of ether surfactants (ethoxylated non-aromatic alcohols) in the formation of macromolecular complexes

| Surf. | Tradename | Supplier | Average HLB | Number of samples | Average Turbidity (FNU) |
|---|---|---|---|---|---|
| Laureth-4 | Procol LA4 | Protameen | 9.4 | 3 | >150 |
| Oleth-5 | Procol OA-5 | Protameen | 12.0 | 3 | >150 |
| | Procol OA-5SP | Protameen | 12.0 | 3 | >150 |
| Steareth-10 | Brij 76 | Unigema | 12.4 | 3 | >150 |
| Oleth-10 | Volpo N10 | Croda | 12.4 | 3 | >150 |

(continued)

| Surf. | Tradename | Supplier | Average HLB | Number of samples | Average Turbidity (FNU) |
|---|---|---|---|---|---|
| Ceteth-10 | Brij 56 | Sigma | 12.9 | 3 | >150 |
| Laureth-8 | 74680 | Sigma | 13.1 | 2 | 131 |
| Laureth-9 | P9641 | Sigma | 13.3 | 2 | >150 |
| Laureth-10 | P9769 | Sigma | 13.5 | 3 | 14.02 |
| Trideceth-10 | P2393 | Sigma | 13.7 | 3 | >150 |
| Oleth-15 | BO-15V | Nikko | 14.2 | 3 | 41.07 |
| C11-15 pareth-12 | Tergitol 15-S-12 | Dow | 14.7 | 2 | 91.9 |
| Isosteareth-20 | Procol IS-20 | Protameen | 15.0 | 3 | 88.7 |
| Steareth-20 | Procol SA-20 | Protameen | 15.2 | 3 | >150 |
| Oleth-20 | Procol OA-20 | Protameen | 15.3 | 2 | >150 |
| | Procol OA-20SP | Protameen | 15.3 | 3 | 37.77* |
| Ceteth-15 | BC-15TX | Nikko | 15.5 | 2 | 101.9 |
| Steareth-21 | Cromul EM1207 | Croda | 15.5 | 3 | >150 |
| C11-15 pareth-15 | Tergitol 15-S-15 | Dow | 15.6 | 1 | 23.6 |
| Ceteareth-20 | Volpo CS20 | Croda | 15.7 | 2 | >150 |
| | Procol CS-20 | Protameen | 15.7 | 1 | 76.8 |
| Ceteth-20 | BC-20TX | Nikko | 15.7 | 3 | 36.75 |
| | Brij 58 | Sigma | 15.7 | 2 | 47.25 |
| | Brij 58P | Uniqema | 15.7 | 3 | 17.87 |
| Isoceteth-20 | Sympatens-AIC/ 200 | Kolb | 15.7 | 3 | 9.63 |
| | Arlasolve 200N | Uniqema | 15.7 | 3 | 8.06 |
| Ceteareth-25 | Volpo CS25 | Croda | 16.2 | 3 | 48.5 |
| C11-15 pareth-20 | Tergitol 15-S-20 | Dow | 16.4 | 1 | 8.19 |
| | Tergitol 15-S-20 (80%) | Dow | 16.4 | 1 | 13.42 |
| Beheneth-20 | BB-20 | Nikko | 16.5 | 3 | >150 |
| C12-C13 pareth-23 | Volpo L23 | Croda | 16.7 | 3 | 10.8 |
| Ceteareth-30 | Procol CS-30 | Protameen | 16.7 | 3 | 141 |
| Laureth-23 | Brij 35 | Sigma | 16.9 | 3 | 4.39 |
| | Brij 35P | Uniqema | 16.9 | 3 | 8.92 |
| Beheneth-25 | Eumulgin BA 25 | Cognis | 17.0 | 3 | >150 |
| Dihydrocholeth-30 | DHC-30 | Nikko | 17.0 | 1 | >150 |
| Beheneth-30 | BB-30 | Nikko | 18.0 | 3 | >150 |

(continued)

| Surf. | Tradename | Supplier | Average HLB | Number of samples | Average Turbidity (FNU) |
|---|---|---|---|---|---|
| C11-15 pareth-40 | Tergitol 15-S-40 | Dow | 18.0 | 3 | >150 |
| Steareth-100 | Sympatens-AS/ 1000G | Kolb | 18.8 | 2 | >150 |
| * Procol OA-20SP contains a higher quantity of unsaturated material than Procol OA-20, and thereby may be expected to be a purer form of Oleth-20. | | | | | |

Table 4a (supplemental) - The use of ether surfactants (ethoxylated non-aromatic alcohols) in the formation of macromolecular complexes

| Surf. | Tradename | Supplier | Average HLB | Number of samples | Average Turbidity (FNU) |
|---|---|---|---|---|---|
| Laureth-3 | Genapol LA030 | Clariant | 8.1 | 1 | >150 |
| C11-15 Pareth-5 | Tergitol 15-S-5 | Surfachem | 10.6 | 1 | >150 |
| Laureth-7 | Genapol LA070 | Clariant | 12.3 | 1 | >150 |
| C11-15 Pareth-7 | Tergitol 15-S-7 | Surfachem | 12.4 | 1 | >150 |
| Ceteareth-11 | Marilpal 1618/11 | Sasol | 13.1 | 1 | >150 |
| Coceth-10 | Genapol C100 | Clariant | 14 | 1 | 14.40 |
| Coceth-20 | Genapol C200 | Clariant | 16 | 1 | 7.50 |
| Ceteareth-80 | Genapol T800 | Clariant | 17.5 | 1 | >150 |
| Ceteareth-50 | Emulgin CS-50 | Cognis | 17.9 | 1 | >150 |

Table 4b - The use of ether surfactants (ethoxylated aromatic alcohols) in the formation of macromolecular complexes

| Surf. | Tradename | Supplier | Average HLB | Number of samples | Average Turbidity (FNU) |
|---|---|---|---|---|---|
| Nonoxynol-9 | Sympatens NP/ 090 | Kolb | 12.9 | 3 | >150 |
| Octoxynol-9 | Triton X-100 | Sigma | 13.5 | 3 | >150 |
| Nonoxynol-10 | Tergitol NP-10 | Sigma | 13.6 | 3 | >150 |
| Octoxynol-12 | Igepal CA-720 | Sigma | 14.5 | 1 | 21.50 |
| Nonoxynol-15 | Sympatens NP/ 150 | Kolb | 15.0 | 3 | 6.46 |
| Octoxynol-16 | Triton X-165 | Sigma | 15.8 | 3 | 13.73 |
| Nonoxynol-20 | Empilan NP20 | Uniqema | 16.0 | 1 | 21.50 |
| Nonoxynol-30 | Empilan NP30 | Uniqema | 17.1 | 1 | >150 |
| Nonoxynol-40 | Igepal CO-890 | Sigma | 17.8 | 3 | >150 |
| Octoxinol-40 | Triton X-405 | Sigma | 17.9 | 3 | >150 |

Table 4b (supplemental) - The use of ether surfactants (ethoxylated aromatic alcohols) in the formation of macromolecular complexes

| Surf. | Tradename | Supplier | Average HLB | Number of samples | Average Turbidity (FNU) |
|---|---|---|---|---|---|
| Octoxynol-5 | Surfac OP5 | Surfachem | 10.4 | 1 | >150 |
| Octoxynol-30 | Surfac OP30 | Surfachem | 17.3 | 1 | >150 |

Table 4c - The use of ether surfactants (ethoxylated PPG acyl ethers) in the formation of macromolecular complexes

| Surf. | Tradename | Supplier | Average HLB | Number of samples | Average Turbidity (FNU) |
|---|---|---|---|---|---|
| PPG-5 ceteth-20 | Protachem AWS-100 | Protameen | 14.4 | 3 | >150 |
| PPG-4 ceteth-20 | PBC-34 | Nikko | 16.5 | 3 | >150 |

Table 4d - The use of ether surfactants (ethoxylated PPG ethers) in the formation of macromolecular complexes

| Surf. | Tradename | Supplier | Average HLB | Number of samples | Average Turbidity (FNU) |
|---|---|---|---|---|---|
| Poloxamer 124 | Synperonic PE/L44 | Uniqema | 15 | 1 | >150 |
| Poloxamer 184 | Synperonic PE/L64 | Uniqema | 20 | 1 | >150 |
| Poloxamer 407 | Lutrol® F127 | BASF | 20 | 1 | >150 |
| Poloxamer 188 | Synperonic PE/F68 | Uniqema | 24 | 1 | >150 |
| Poloxamer 237 | Synperonic PE/F87 | Uniqema | 24 | 1 | >150 |

Table 4e - The use of ether surfactants (propoxylated POE ethers) in the formation of macromolecular complexes

| Surf. | Tradename | Supplier | Average HLB | Number of samples | Average Turbidity (FNU) |
|---|---|---|---|---|---|
| PO-EO-PO | 435473 | Sigma | 15 | 1 | >150 |

Table 4f - The use of ester surfactants (ethoxylated carboxylic acids) in the formation of macromolecular complexes

| Surf. | Tradename | Supplier | Average HLB | Number of samples | Average Turbidity (FNU) |
|---|---|---|---|---|---|
| PEG-2 stearate | Protachem DGS | Protameen | 4.5 | 1 | >150 |
| PEG-4 stearate | Protamate 200 DPS | Protameen | 7.5 | 1 | >150 |
| PEG-4 oleate | Protamate 200-OC | Protameen | 8.0 | 1 | >150 |

(continued)

| Surf. | Tradename | Supplier | Average HLB | Number of samples | Average Turbidity (FNU) |
|---|---|---|---|---|---|
| PEG-8 dioleate | Protamate 400-DO | Protameen | 8.3 | 1 | >150 |
| PEG-6 stearate | Protamate 300 DPS | Protameen | 10.0 | 3 | >150 |
| PEG-9 laurate | P460141 | Sigma | 13.1 | 3 | >150 |
| PEG-14 oleate | P460176 | Sigma | 13.5 | 3 | >150 |
| PEG-20 stearate | Protamate 1000 DPS | Protameen | 16.0 | 1 | >150 |
| | Cithrol 10MS | Croda | 16.0 | 3 | 75.06 |
| PEG-75 lanolin | Solan E50 | Croda | 16.2 | 1 | >150 |
| PEG-150 distearate | Protamate 6000-DS | Protameen | 16.5 | 3 | >150 |
| PEG-30 stearate | Sympatens-BS/ 300G | Kolb | 16.5 | 1 | >150 |
| PEG-40 stearate | Crodet S40LD | Croda | 16.9 | 3 | 118.93 |
| | Protamate 1540-DPS | Protameen | 16.9 | 3 | >150 |
| | Myrj 52S | Uniqema | 16.9 | 1 | >150 |
| PEG-50 stearate | Sympatens-BS/ 500G | Kolb | 18.8 | 1 | >150 |
| PEG-100 stearate | Protamate 4400-DPS | Protameen | 18.8 | 1 | >150 |

Table 4g - The use of ester surfactants (ethoxylated glycerides) in the formation of macromolecular complexes

| Surf. | Tradename | Supplier | Average HLB | Number of samples | Average Turbidity (FNU) |
|---|---|---|---|---|---|
| PEG-40 hydrogenated castor oil | Lincol ORH 40/S | Eigenmann & V | 12.9 | 1 | >150 |
| PEG-40 hydrogenated castor oil | Akyporox CO400 | Kao | 12.9 | 1 | >150 |
| PEG-8 glyceryl-caprylate/ caprate | L.A.S. | Gattefosse | 14.0 | 1 | >150 |

Table 4h - The use of ester surfactants (polyglycerol esters) in the formation of macromolecular complexes

| Surf. | Tradename | Supplier | Average HLB | Number of samples | Average Turbidity (FNU) |
|---|---|---|---|---|---|
| Polyglyceryl-6-caprylate | Dermofeel G 6CY | Gemro/Dr Streat | 15.0 | 1 | >150 |

(continued)

| Surf. | Tradename | Supplier | Average HLB | Number of samples | Average Turbidity (FNU) |
|---|---|---|---|---|---|
| Polyglyceryl-10-laurate | Dermofeel G 10L | Gemro/Dr Streat | 16.0 | 1 | >150 |
| Polyglyceryl-10 laurate | Decaglyn 1-L | Nikko | 16.0 | 1 | >150 |

Table 4h (supplemental) - The use of ester surfactants (polyglycerol esters) in the formation of macromolecular complexes

| Surf. | Tradename | Supplier | Average HLB | Number of samples | Average Turbidity (FNU) |
|---|---|---|---|---|---|
| Polyglyceryl-2-stearate | Hostacerin DGMS | Clariant | 5** | 1 | >150 |
| ** No published HLB data available, estimated based on other HLB values for related surfactants | | | | | |

Table 4i - The use of sorbitan ester surfactants (acylated sorbitan esters) in the formation of macromolecular complexes

| Surf. | Tradename | Supplier | Average HLB | Number of samples | Average Turbidity (FNU) |
|---|---|---|---|---|---|
| Sorbiatan oleate | Protachem SMO | Protameen | 4.3 | 1 | >150 |
| Sorbitan stearate | Protachem SMS | Protameen | 4.7 | 1 | >150 |
| Sorbitan palmitate | Protachem SMP | Protameen | 6.7 | 1 | >150 |

Table 4j - The use of sorbitan ester surfactants (PEG sorbitan esters) in the formation of macromolecular complexes

| Surf. | Tradename | Supplier | Average HLB | Number of samples | Average Turbidity (FNU) |
|---|---|---|---|---|---|
| Polysorbate 60 | Protasorb S-20 | Protameen | 14.9 | 1 | >150 |
| Polysorbate 80 | Protasorb O-20 | Protameen | 15.0 | 1 | >150 |
| Polysorbate 40 | Protasorb P-20 | Protameen | 15.6 | 1 | >150 |
| Polysorbate 20 | Protasorb L-20 | Protameen | 16.7 | 1 | 3.25 |

Table 4k - The use of sugar ester surfactants (non-sorbitan sugar esters) in the formation of macromolecular complexes

| Surf. | Tradename | Supplier | Average HLB | Number of samples | Average Turbidity (FNU) |
|---|---|---|---|---|---|
| Sucrose distearate | Sisterna SP01-C | Sisterna | 1.0 | 1 | >150 |
| Sucrose distearate | Sisterna SP30-C | Sisterna | 6.0 | 1 | >150 |
| Sucrose stearate | Sisterna SP50-C | Sisterna | 11.0 | 1 | >150 |

(continued)

| Surf. | Tradename | Supplier | Average HLB | Number of samples | Average Turbidity (FNU) |
|---|---|---|---|---|---|
| Sucrose laurate | Surfhope C-1215L | Mitsubishi | 15.0 | 1 | 14.5 |
| Sucrose palmitate | Surfhope C-1615 | Mitsubishi | 15.0 | 1 | >150 |
| Sucrose oleate | Surfhope C-1715 | Mitsubishi | 15.0 | 1 | >150 |
| Sucrose oleate | Surfhope C-1715L | Mitsubishi | 15.0 | 1 | >150 |
| Sucrose stearate | Surfhope C-1815 | Mitsubishi | 15.0 | 1 | >150 |
| Sucrose stearate | Sisterna SP70-C | Sisterna | 15.0 | 1 | >150 |
| Sucrose myristate | Surfhope C-1216 | Mitsubishi | 16.0 | 1 | 35.0 |
| Sucrose myristate | Surfhope C-1416 | Mitsubishi | 16.0 | 1 | 45.2 |
| Sucrose palmitate | Surfhope C-1616 | Mitsubishi | 16.0 | 1 | >150 |
| Sucrose stearate | Surfhope C-1816 | Mitsubishi | 16.0 | 1 | >150 |
| Lauryl glucoside (C8-C16, mainly C12) | Plantacare® 1200UP | Cognis | - | 1 | >150 |
| Decyl glucoside (C8-C16, mainly C8) | Plantacare® 2000UP | Cognis | - | 1 | 100.6 |
| Decyl glucoside | Oramix NS-10 | Seppic | - | 1 | 26.4 |
| Caprylyl/Capryl glucoside (C8-C16) | Plantacare® 810UP | Cognis | - | 1 | >150 |
| Caprylyl/Capryl glucoside | Oramix CG 110 | Seppic | - | 1 | >150 |
| Coco-glucoside (C8-C16, mainly C12) | Plantacare® 818UP | Cognis | - | 1 | >150 |

Table 41- The use of sugar ester surfactants (PEG non-sorbitan sugar esters) in the formation of macromolecular complexes

| Surf. | Tradename | Supplier | Average HLB | Number of samples | Average Turbidity (FNU) |
|---|---|---|---|---|---|
| PEG-20 methyl glucose sesquistearate | Glucamate SSE-20 | Uniqema | 15.0 | 3 | >150 |

(continued)

| Surf. | Tradename | Supplier | Average HLB | Number of samples | Average Turbidity (FNU) |
|---|---|---|---|---|---|
| PEG-120 methyl glucose dioleate | Glucamate™ DOE-120 | Uniqema | - | 1 | >150 |

Table 4m - The use of cationic surfactants (synthetic phospholipids) in the formation of macromolecular complexes

| Surf. | Tradename | Supplier | Average HLB | Number of samples | Average Turbidity (FNU) |
|---|---|---|---|---|---|
| Linoleamidopropy l PG-dimonium chloride phosphate | Arlasilk™ EFA | Uniqema | 18.0 | 1 | >150 |
| Cocamidopropyl PG-dimonium chloride phosphate | Arlasilk™ PTC | Uniqema | 18.0 | 1 | >150 |

Table 4n - The use of cationic surfactants (PEG alkyl amines) in the formation of macromolecular complexes

| Surf. | Tradename | Supplier | Average HLB | Number of samples | Average Turbidity (FNU) |
|---|---|---|---|---|---|
| PEG-5 cocamine | Protox C-5 | Protameen | 11.0 | 3 | 5.26 |
| PEG-15 cocamine | Protox C-15 | Protameen | 15.4 | 1 | 4.42 |

Table 4o - The use of anionic surfactants (fatty acids) in the formation of macromolecular complexes

| Surf. | Common Name | Supplier | Average HLB | Number of samples | Average Turbidity (FNU) |
|---|---|---|---|---|---|
| Decanoic acid | Decanoic Acid | Sigma | - | 1 | >150 |
| Hexanoic acid | Hexanoic Acid | Sigma | - | 1 | >150 |
| Dodecanoic acid | Lauric Acid | Sigma | - | 1 | >150 |
| Octanoic acid | Octanoic Acid | Sigma | - | 1 | >150 |
| 2-Hydroxybenzoic acid | Salicylic Acid | Sigma | - | 1 | >150 |
| Octadecanoic acid | Oleic Acid | Breckland | - | 1 | >150 |
| Hexadecanoic acid | Palmitic Acid | Sigma | - | 1 | >150 |
| DL-Mandelic acid | Mandelic Acid | Sigma | - | 1 | >150 |
| Benzoic acid | Benzoic Acid | Sigma | - | 1 | >150 |
| Decanoic acid | Capric Acid | A&E Connock | - | 1 | >150 |

(continued)

| Surf. | Common Name | Supplier | Average HLB | Number of samples | Average Turbidity (FNU) |
|---|---|---|---|---|---|
| Octanoic acid | Caprylic Acid | A&E Connock | - | 1 | >150 |
| Hexanoic acid | Caproic Acid | A&E Connock | - | 1 | >150 |
| Potassium oleate | Potassium Oleate | A&E Connock | - | 1 | >150 |

Table 4p - The use of anionic surfactants (amino acid amides) in the formation of macromolecular complexes

| Surf. | Tradename | Supplier | Average HLB | Number of samples | Average Turbidity (FNU) |
|---|---|---|---|---|---|
| Sodium stearoyl glutamate/ sodium cocoyl glutamate (mixture) | Amisoft® GS-11P (F) | Ajinamoto | - | 2 | >150 |
| Sodium stearoyl glutamate | Amisoft® HS-11P (F) | Ajinamoto | - | 2 | >150 |
| Sodium lauroyl glutamate | Amisoft® LS-11 (F) | Ajinamoto | - | 2 | 26.10 |
| Sodium myristoyl glutamate | Amisoft® MS-11 (F) | Ajinamoto | - | 2 | >150 |
| Sodium cocoyl glycinate | Amilite® GCS-11 | Ajinamoto | - | 2 | 40.50 |
| Sodium cocoyl glutamate | Amisoft® CS-11 (F) | Ajinamoto | - | 2 | 28.50 |
| Sodium cocoyl methyl taurate | Somepan T25 | Seppic | - | 1 | 50.9 |
| Potassium lauryl wheat amino acids | Aminofoam® WOR | Croda | - | 3 | 11.45 |
| Sodium lauryl wheat amino acids | Proteol® LW 30 | Seppic | - | 1 | 49.1 |
| Sodium lauryl oat amino acids | Proteol® OAT | Seppic | - | 3 | 15.48 |
| Disodium cocoyl glutamate | Plantapon® ACG 35 | Cognis | - | 1 | 54.50 |
| Sodium cocoyl glutamate | Plantapon® ACG 50 | Cognis | - | 1 | 16.63 |
| Sodium cocoyl hydrolyzed wheat protein glutamate | Plantapon® S | Cognis | - | 1 | >150 |
| Sodium cocoyl apple amino acids | Proteol™ APL | Seppic | - | 1 | 82.3 |

Table 4q - The use of anionic surfactants (surfactin) in the formation of macromolecular complexes

| Surf. | Tradename | Supplier | Average HLB | Number of samples | Average Turbidity (FNU) |
|---|---|---|---|---|---|
| Surfactin | Aminofect | Showa Denko | - | 1 | 13.16 |

Table 4r- The use of anionic surfactants (esters of alpha-hydroxycarboxylic acids) in the formation of macromolecular complexes

| Surf. | Tradename | Supplier | Average HLB | Number of samples | Average Turbidity (FNU) |
|---|---|---|---|---|---|
| Sodium lauryl lactate | Pationic 138C | Rita | 14.4 | 1 | >150 |

Table 4s - The use of anionic surfactants (phosphate based) in the formation of macromolecular complexes

| Surf. | Tradename | Supplier | Average HLB | Number of samples | Average Turbidity (FNU) |
|---|---|---|---|---|---|
| Cetyl phosphate | Crodafos MCA | Croda | - | 1 | >150 |
| PPG-5-ceteth-10 phosphate | Crodafos SG | Croda | - | 1 | >150 |

Table 4t - The use of amphoteric surfactants in the formation of macromolecular complexes

| Surf. | Tradename | Supplier | Average HLB | Number of samples | Average Turbidity (FNU) |
|---|---|---|---|---|---|
| Cocamidopropyl betaine | Incronam 30 | Croda | - | 1 | >150 |

Table 4u - The use of silicone surfactants in the formation of macromolecular complexes

| Surf. | Tradename | Supplier | Average HLB | Number of samples | Average Turbidity (FNU) |
|---|---|---|---|---|---|
| Polysiloxy linoleyl phospholipid | Monasil PLN | Mona | - | 1 | >150 |
| Polysiloxy carboxylic acid | Monasil PCA | Mona | - | 1 | >150 |
| Dimethicone copolyol phosphate | Pecosil PS-100 | Phoenix | - | 1 | >150 |
| Dimethicone copolyol sulfosuccinate | Mackanate DC-50 | McIntyre | - | 1 | >150 |

Table 4v - The use of non-amino acid based anionic surfactants in the formation of macromolecular complexes

| Surf. | Tradename | Supplier | Average HLB | Number of samples | Average Turbidity (FNU) |
|---|---|---|---|---|---|
| Sodium cholate | - | Sigma | - | 1 | 23.3 |
| Sodium deoxycholate | - | Sigma | 24 | 1 | 32.6 |
| Sodium laureth sulphate | EMAL® 228 D/JM | Kao | 18 | 1 | >150 |
| Sodium lauryl sulphate | - | Sigma | 40 | 1 | 26.2 |

Discussion

Compositions with a low turbidity can be deduced to have formed macromolecular assemblies which are sufficiently small not to disrupt the passage of light (i.e. being less than 100 nm in size).

Figure 1 shows a plot of HLB numbers of less than 20 against sample clarity. As can be seen from the plot, a distinct band of surfactants having HLB numbers from around 12.5 to around 17.5 is of use in production of the macromolecular complexes of the present invention.

Only one surfactant falling outside the HLB range from around 12.5 to around 17.5 was found to form macromolecular assemblies (PEG-5 cocamine, HLB reported as 11.0). However, it should be noted that this is a mixture of cationic components and has an unusual structure (the PEG units being divided between two groups attached to the tertiary amine), it is therefore not ideally suited to being categorised by the HLB system and the HLB numbers reported for this surfactant may be lower than the actual hydrophobic/lipophilic balance. The major acyl chain component of PEG-5 cocamine is PEG-5 lauryl amine, which should have an HLB of around 12.4 (following the HLB determination method described in Aulton ME Pharmaceutics- The Science of Dosage Form Design, Churchill Livingstone, 2002). Applying the same calculation method to PEG-15 cocamine provides an HLB value of 15.7 (reported HLB 15.4).

A proportion of surfactants within the HLB range from around 12.5 to around 17.5 did not form macromolecular complexes under the conditions tested. Firstly, as each surfactant in this experiment was only tested with a single lipid component, these materials may be able to form macromolecular complexes with other lipids. Secondly, the numbers assigned by the HLB system are estimative of a surfactant's properties and the system is best suited for comparison between surfactants of similar structure. The principle behind the present invention is best illustrated by reference to individual groups of surfactants (i.e. those having a similar chemical structure). In this regard Figure 2 plots the HLB of the alkyl phenol ethoxylates surfactants against sample clarity and Figure 3 plots the HLB of the PEG pareth ether surfactants against sample clarity. Figure 4 provides an illustration of the turbidity of samples prepared using ethoxyalkylated PEG oleth ether surfactants. Figures 2 to 4 demonstrate the existence of a clearly defined HLB range for each specific surfactant class over which macromolecular complexes can be prepared.

In summary, contrary to the expectations of one skilled in the art, it may be concluded that it is in principle possible to make macromolecular complexes with diverse types of surfactant in the absence of additional stabilising components such as cholesterol. Suitable surfactants within a given appropriate class may be readily identified by testing exemplary surfactants from that class having a number of different HLB values.

**Example 2 - The use of the exemplary surfactants with a range of natural lipid mixtures in the formation of macromolecular complexes of the invention**

In light of the results of Example 1, and the knowledge that certain surfactants are capable of forming macromolecular complexes of the invention, the suitability of a range of natural lipid extracts for use in the present invention was tested. A number of commercially available lipid compositions derived from soyabean were analysed.

Method

Samples were prepared in water by an analogous procedure to that described in Example 1. A range of aqueous solutions containing 2.5% surfactant and 1% lipid were produced (note the absence of co-surfactant in this case). A further sample was prepared using a mixture of 1.25% of each of two surfactants (i.e. total surfactant 2.5%) together with 1% lipid.

For a quantitative analysis of the clarity of aqueous solutions of surfactant and lipid mixtures, samples were

examined using a turbidity meter (Nephla, from Hach-Lange). The turbidity meter was calibrated prior to use, with two known standards (0 and 40 FNU).

*Surfactant*

The surfactants were as described previously in Example 1.

*Lipids*

90H was as described in Example 1.
Phospholipon® 80 H, referred to herein by the abbreviation 80H, available from Phospholipid GmbH (Germany), is a hydrogenated soy lecithin extract of at least 60% phosphatidyl choline content and is used as an emulsifier and forms liposomes. It is sold for use in cosmetics.
SL 80 is a purified soy extract containing 69% phosphatidyl choline. It is available from Lipoid GmbH.
Emulmetik 900 (Em900) is a de-oiled purified soy extract enriched with phosphatidyl choline to at least 45% purity. It is used as an emulsifier and forms liposomes. Em900 is available from Lucas Meyer Cosmetics SA.
Emulmetik 300 (Em300) is a de-oiled purified soy extract containing at least 97% phospholipids and glycolipids. It is used as a coemulsifier. Em300 is available from Lucas Meyer Cosmetics SA.
Epikuron 130P (Ep130P) is a de-oiled soy lecithin fraction enriched with phosphatidyl choline to at least 30% purity. It is used as an emulsifier, and is approved for pharmaceutical use. Ep130P is available from Degussa Texturant Systems UK Ltd.
Emulmetik 950 (Em950) is a purified, hydrogenated soy extract containing at least 94% phosphatidyl cholines. It is used as an emulsifier and forms liposomes. Em950 is available from Lucas Meyer Cosmetics SA.
EMULTOP® IP (EMT IP) is a deoiled, enzymatically hydrolysed, soybean lecithin which is enriched with lysophospholipids for use in the food industry. It is available from Lucas Meyer (Degussa Texturant Systems UK Ltd). The lipid mixture contains >95% acetone insolubles, less than 3% oil, less than 5% lyso-PC and greater than 12% phosphatidylcholine.
EMULPUR® IP (EMP IP) is a deoiled, powdered soybean lecithin for use in the food industry. It is available from Lucas Meyer (Degussa Texturant Systems UK Ltd). The lipid mixture contains >96.5% acetone insolubles, less than 2% oil and is mainly phospholipids and glycolipids.
Phospholipon® 90 NG, referred to herein by the abbreviation 90NG, available from Phospholipid GmbH (Germany), is a soy lecithin extract of at least 90% phosphatidyl choline content. It is used as an emulsifier and forms liposomes, and is sold for use in pharmaceuticals and cosmetics.
S 75 is a purified soy extract containing 68-73% phosphatidyl choline. It is available from Lipoid GmbH.
S 100 is a purified soy extract containing at least 94% phosphatidyl choline. It is available from Lipoid GmbH.
S PC is a purified soy extract containing 98% phosphatidyl choline. It is available from Lipoid GmbH.
Epikuron 145V (Ep145V) is a de-oiled soy lecithin fraction enriched with phosphatidyl choline to at least 45% purity. Ep145V is available from Degussa Texturant Systems UK Ltd.

Results

Table 5 below summarises the composition of the lipid extracts on a dry weight basis (where available).

Table 5 - Composition of exemplary soya lipid extracts

| Lipid Component | Lipid Composition % | | | | |
|---|---|---|---|---|---|
| | PC | LPC | PE | Other PL | Free Fatty Acids |
| Ep145V | >45 | <4 | >10 | PI <3 | UNKNOWN |
| Em950 † | >94 | <1 | UNKNOWN | <3 | UNKNOWN |
| Em930 | >92 | <3 | UNKNOWN | <2 | UNKNOWN |
| Em900 | >45 | UNKNOWN | <10 | PA<3.0 | UNKNOWN |
| Em300 | PL+GL>97 | | | | |
| Ep130P | 30-33 | UNKNOWN | 16-19 | PI 9-12 | UNKNOWN |
| S 75 | 68-73 | <3.0 | 7-10 | UNKNOWN | |

(continued)

| Lipid Component | Lipid Composition % | | | | |
|---|---|---|---|---|---|
| | PC | LPC | PE | Other PL | Free Fatty Acids |
| S 100 | >94 | <3.0 | <0.1 | PI<0.1 | NON-PLR <3.0 |
| S PC | 98 | 0.20 | <0.1 | <0.1 | <0.05 |
| SL 80 | 69 | 15.60 | UNKNOWN | | |
| SL 80-3 | 54 | 21.70 | UNKNOWN | | |
| 90H † | >90 | <4.0 | UNKNOWN | | |
| 80H † | >60 | <10 | UNKNOWN | | |
| 90 NG | >90% | <6.0 | UNKNOWN | | |

Key:
PC = phosphatidylcholine
LPC = lyso-phosphatidylcholine
PE = phosphatidylethanolamine
PI = phosphatidylinositol
PL = phospholipid
PA = phosphatidic acid
GL = glycolipid
PLR = polar lipid
† = hydrogenated

Table 6 below summarises the results of the experiment.

Table 6 - The use of various lipid extracts in the manufacture of macromolecular complexes

| Row | Lipid | Surfactant | | | |
|---|---|---|---|---|---|
| | | Brij 56 (HLB=12.9) | Symp. AIC 200 (HLB=15.7) | Triton X-165 (HLB=16) | Brij 35P (HLB=16.9) |
| 1 | 90H | >150 | 9.63 | 14.37 | 9.36 |
| 2 | 80H | 30.85 | 10.02 | 10.59 | 50.55 |
| 3 | SL 80 | 139.70 | 13.59 | 10.63 | 18.38 |
| 4 | Em900 | >150 | 78.30 | 34.75 | 118.20 |
| 5 | Em300 | >150 | 10.67 | 10.47 | 76.08 |
| 6 | Ep130P | >150 | 13.05 | 3.67 | 108.10 |
| 7 | Em950 | >150 | 38.90 | 92.00 | >150 |
| 8 | EMT IP | >150 | 21.40 | 13.88 | >150 |
| 9 | EMP IP | >150 | 74.10 | 93.00 | >150 |
| 10 | 90 NG | >150 | >150 | 3.48 | 6.04 |
| 11 | S 75 | >150 | >150 | 5.76 | 65.40 |
| 12 | S 100 | >150 | >150 | 9.00 | 17.13 |
| 13 | S PC | >150 | >150 | 3.63 | 42.30 |
| 14 | Ep200 | >150 | >150 | 4.60 | 15.92 |
| 15 | Ep145V | >150 | >150 | 88.45 | 125.2 |
| 16 | S 75 | - | 4.37 | | - |

Discussion

The results of Example 2 confirm that surfactants previously determined to form macromolecular assemblies with the lipid 90H (i.e. Sympatens-AIC/200, Triton X-165 and Brij 35P) can also be used with a range of other lipid extracts to form the macromolecular assemblies of the present invention.

The lipids used in rows 1-6 of Table 6 were determined to be suitable with all three of the Sympatens-AIC/200, Triton X-165 and Brij 35P surfactants (HLB 15.7, 16.0 and 16.9). The lipids used in rows 7-9 of Table 6 were suitable for use with Sympatens-AIC/200 and Triton X-165 (HLB 15.7 and 16.0). The lipids used in rows 10-15 of Table 6 were found to be suitable for use with Triton X-165 and Brij 35P (HLB 16.0 and 16.9). As such, it may be concluded that each lipid composition has a specific range of surfactant HLB values with which it will form the macromolecular complexes of the present invention.

Also of note is the fact that Brij 56, which did not form macromolecular complexes with 90H lipid, was found to form macromolecular complexes with other lipid extracts.

A mixture of surfactants (1.25% Sympatens-AIC/200 and 1.25% Triton X-165) was able to solubilise 1% S 75 lipid, whereas of these two surfactants only Triton X-165 was able to solubilised 1% S 75 lipid on its own.

The lipid extracts shown above are extremely complex natural products whose contents vary both in the nature of the phospholipid headgroups present and in their associated acyl chains (chain length and degree of unsaturation). This experiment therefore demonstrates the versatility of the present invention in the solubilisation a broad range of lipid mixtures to form substantially clear and colourless aqueous solutions.

**Example 3 - The use of a co-surfactant in compositions of the invention**

As demonstrated above, surfactants are capable of interacting with lipids to form macromolecular surfactant/ lipid complexes. Although there will generally be an insubstantial level of disruption to the passage of light in compositions of the invention, the addition of a co-surfactant was tested as a means of ensuring that solutions were completely clear.

Method

Samples containing co-surfactant were prepared according to the general procedure described in Example 1.

A stock emulsion of lipid was prepared at double the desired final concentration (i.e. containing 2% 90H and 0.1% SL 80-3). Briefly, to the appropriate volume of warm water (ca. 60 °C), SL 80-3 was added. Heating and stirring was maintained for approximately 15 minutes before the mixture was homogenised for around 1 minute at 13,000 RPM (POLYTRON PT 3100 Homogeniser). 90H was then added gradually, with heating and stirring maintained throughout and for a further 45 minutes after completion. The mixture was then homogenised for around 3 minutes at 15,000 RPM followed by 1 minute at 26,000 RPM.

A stock solution of surfactant was prepared at double the desired final concentration (i.e. a 5% stock, for a 2.5% final concentration) by mixing of the surfactant with the appropriate volume of water. After mixing the solution was stirred and heated to around 60-70 °C.

The required quantity of warm lipid emulsion was slowly added to the warm surfactant solution while stirring with the temperature maintained.

Samples were then allowed to cool to room temperature before being analysed.

Samples which did not contain any co-surfactant were prepared by an analogous procedure, based on a standard lipid emulsion containing 1% 90H. A stock emulsion of lipid was prepared at double the desired final concentration (i.e. containing 2% 90H). Briefly, to the appropriate volume of warm water (ca. 60 °C), 90H was added gradually, with heating and stirring maintained throughout and for a further 45 minutes after completion. The mixture was then homogenised for around 3 minutes at 15,000 RPM followed by 1 minute at 26,000 RPM.

A stock solution of surfactant was prepared at double the desired final concentration (i.e. a 5% stock, for a 2.5% final concentration) by mixing of the surfactant with the appropriate volume of water. After mixing the solution was stirred and heated to around 60-70 °C.

The required quantity of warm lipid emulsion was slowly added to the warm surfactant solution while stirring with the temperature maintained.

Samples were then allowed to cool to room temperature before being analysed.

For a quantitative analysis of the clarity of aqueous solutions of surfactant, lipid and co-surfactant, samples were examined using a turbidity meter (Nephla, from Hach-Lange). The turbidity meter was calibrated prior to use, with two known standards (0 and 40 FNU).

*Surfactant*

Brij 35P was as described in Example 1.

*Lipid*

90H was as described in Example 1.
S 75 was as described in Example 2.

*Co-surfactant*

S LPC was added as a component of SL 80-3 (which is described in Example 2).

Results

Table 7 below summarises the results of the experiment.

Table 7 - The use of a co-surfactant in compositions of the invention

| Surf. | Surf. % w/w | Lipid | Lipid % w/w | Co-surf. | Co-surf. % w/w | Clarity | Turbidity (FNU) |
|---|---|---|---|---|---|---|---|
| Brij 35P | 2.5 | 90H | 1.0 | - | - | Clear | 17.36 |
| Brij 35P | 2.5 | 90H | 1.0 | S LPC (as SL 80-3) | ca. 0.01 (0.05%) | Clear | 9.36 |
| Brij 35P | 2.5 | S 75 | 1.0 | - | - | Clear | 65.40 |
| Brij 35P | 2.5 | S 75 | 1.0 | S LPC | ca. 0.01 | Clear | 13.64 |
| | | | | (as SL 80-3) | (0.05%) | | |

Discussion

The use of a small quantity of co-surfactant (equivalent to around only 1% of the total lipid component) provides a notable improvement in the clarity of solutions.

**Example 4 - The use of compositions of the invention to solubilise exemplary active agents**

As demonstrated above, certain surfactants are capable of interacting with a range of lipids to form macromolecular complexes. Such macromolecular complexes may be expected to be of use in the solubilisation of active agents which have a poor aqueous solubility. Compositions according to the present invention were therefore tested with a range of exemplary active agents with poor aqueous solubility to illustrate the potential application of the compositions in the fields of cosmetics and pharmaceuticals.

Method

A stock emulsion of co-surfactant, lipid and active agent was prepared at double the desired final concentration. Co-surfactant was dissolved in water while heating (approximately 60°C) and stirring, with the conditions maintained for approximately 15 further minutes before the mixture was homogenised (approximately 1 minute at 13,000 RPM, POLYTRON PT 3100 Homogeniser). Lipid was then added gradually, followed by continued stirring and heating until a uniform emulsion is formed. After approximately 45 minutes the mixture was then homogenised (approximately 3 minute at 15,000 RPM). The temperature was then brought to around 50°C before the active component was added slowly under stirring until a uniform emulsion was present. The final emulsion was then homogenised (approximately 1 minute, ULTRA-TURRAX® - Janke & Kunkel).
A stock solution of surfactant was prepared at double (i.e. 5%) the desired final concentration of 2.5%, by mixing of the surfactant with the appropriate volume of water.
Macromolecular complexes incorporating active agents were then prepared by the dropwise addition of the warm lipid containing emulsion to an equal volume of surfactant solution while stirring and heating 50°C.

Samples were then allowed to cool to room temperature before being analysed.

For a quantitative analysis of the clarity of aqueous solutions of surfactant, lipid and active, samples were examined using a turbidity meter (Nephla, from Hach-Lange). The turbidity meter was calibrated prior to use, with two known standards (0 and 40 FNU).

*Surfactants*

Tergitol 15-S-20 was as described in Example 1.

Aminofoam WOR was as described in Example 1.

Protasorb L-20 was as described in Example 1.

Amisoft MS11-F was as described in Example 1.

Amisoft CS11-F was as described in Example 1.

Amisoft LS11-F was as described in Example 1.

Surfhope C-1216 was as described in Example 1.

Volpo L23 was as described in Example 1.

Brij 35P was as described in Example 1.

Proteol OAT was as described in Example 1.

Control compositions of sodium cholate, sodium deoxycholate, sodium lauryl sulphate were as described in example 1.

*Lipids*

The lipid 90H was as described in Example 1.

The lipid 80H was as described in Example 2.

The lipid S75 was as described in Example 2.

The lipid SL 80-3 was as described in Example 1.

*Active Agents*

- TECA (Titrated extract of *Centella asiatica)* supplied by Bayer Santé Familiale (France) CAS: 84696-21-9
- D&C Red No. 27 supplied by Sun Chemical Corporation (USA). CAS: 84473-86-9
- Myristidone® (Myristyl ester of L-pyrrolidone) supplied by UCIB, Solabia Group (France) CAS: 37673-27-1
- Laurydone® (Lauric ester of L-pyrrolidone carboxylic acid) supplied by UCIB, Solabia Group (France) CAS: 22794-26-9
- Ciclopirox olamine supplied by Sigma-Aldrich Ltd. (UK) CAS: 41621-49-2
- Econazole nitrate supplied by Sigma-Aldrich Ltd. (UK) CAS: 24169-02-6
- Bromocresol Green (tetrabromo-m-cresolsulfonphthalein sulfone) supplied by Merck KGaA. (Germany) CAS: 76-60-8
- Herbalia® Red Clover is an extract of *Trifolium pretense* supplied by Cognis Iberia s.l. (Spain)
- Herbalia® Centella is an extract of *Centella asiatica* containing 10% active constituents (madecassic acid, asiatic acid, asiaticoside) supplied by Cognis Iberia s.l. (Spain)
- Herbailia® Butcher's Broom is an extract of *Ruscus aculeatus* supplied by Cognis Iberia s.l. (Spain)
- Benzyl nicotinate supplied by Fluka Chemie GmbH. (Germany) CAS: 94-44-0
- Octopirox® (Piroctone olamine) supplied by Clariant UK Ltd. (UK) CAS: 68890-66-4
- Plantactiv® Centella is an extract of *Centella asiatica* containing 100% active constituents (madecassic acid, asiatic acid, asiaticoside) supplied by Cognis Iberia s.l. (Spain)
- Argireline® (acetyl hexapeptide-3) supplied by Lipotec S.A. (Spain)
- Ginkgo (Herbalia Ginkgo CG) is an extract of *Ginkgo biloba* supplied by Cognis Iberia s.l. (Spain)
- Ginkgo G38 (Ginkgo Biloba Extract G328) is an extract of *Ginkgo biloba* supplied by Linnea S.A. (Switzerland)
- Ginkgo GSF (Ginkgo Biloba Extract G320) is an extract of *Ginkgo biloba* supplied by Linnea S.A. (Switzerland)
- Herbalia® Horse Chestnut is an extract of *Aesculus hippocastanum* supplied by Cognis Iberia s.l. (Spain)
- Herbalia® Nettle is an extract of *Urtica dioica* supplied by Cognis Iberia s.l. (Spain)
- Plantactiv® Aesculus is an extract of *Aesculus hippocastanum* containing 98% active constituents (β-escin) supplied by Cognis Iberia s.l. (Spain)
- Yohimbine HCl Crystalline is an extract of *Pausinystalia yohimbe* supplied by International Laboratory Inc. (USA), it was used in free base form

- Yohimbine HCI USP is an extract of *Pausinystalia yohimbe* supplied by Chemical Resources Ltd. (India), it was used in free base form
- Yohimbine 10% Extract is an extract of *Pausinystalia yohimbe* supplied by Chemical Resources Ltd. (India)
- Yohimbine HCI USP H.O. #031 is an extract of *Pausinystalia yohimbe* supplied by Alchem International (India), it was used in free base form
- Hydrocortisone Ph.Eur./USP/JP grade supplied by Sanofi Aventis Pharma S.A. (France) CAS: 50-23-7
- Salmeterol (as salmeterol xinafoate) Ph.Eur Micronised grade supplied by Natco Pharma Ltd. (India)
- Progesterone USP grade supplied by Sigma-Aldrich Ltd. (UK)
- Devil's Claw Extract is an extract of *Harpagophytum procumbens* supplied by Advanced Phyto-Trading (South Africa)
- Gatuline® Expression contains an extract of *Acmella oleracea* supplied by Gattefossé SAS (France), the agent was used after removal of the alcohol vehicle in which it is supplied
- Extract of *Picea abies*
- Camphor (D-Camphor) Ph Eur/BP/USP grade supplied by Merck KGaA. (Germany)
- Totarol® (Totara-8,11,13-trien-13-ol) is an extract of *Podocarpus totara* supplied by Mende-Biotech Ltd. (New Zealand)
- Jambu - Jambu oleoresine extract of *Spilanthes acmella* containing 30% spilanthol supplied by Robertet S.A. (France)
- SepiWhite™ MSH (Undecylenoyl phenylalanine) supplied by Seppic S.A. (France)
- Phyto-Age™ is an extract of *Cimicifuga racemosa* supplied by Seppic S.A. (France)
- Boswellin® CG is an extract of *Boswellia serrata* (β-boswellic acids) supplied by Sabinsa Corp. (USA) CAS: 97952-72-2
- Sichuan Pepper Extract - prepared from Sichuan pepper supplied by Incense Magic Ltd. (UK)
- Prickly Ash Tincture is an extract of *Zanthoxylum clava herculi* supplied by G.Baldwin & Co. (UK)
- 7-dehydro-cholesterol, pro-vitamin D3, supplied by MMP Inc. (USA) CAS 000434-16-2
- Apricosal (AFL-3607/E) supplied by Arriva Fragrances (UK)
- Ascorbyl Palmitate, is a vitamin C monopalmitate derivative supplied by DSM N.V. (Netherlands) CAS 137-66-6
- Avobenzene supplied by Unifect (UK). CAS 70356-09-1
- Boswellin CG extract of Boswellia *serrata* (β-boswellic acids) supplied by Sabinsa Corp. (USA) CAS 97952-72-2
- Cholesterol was used at >95% Ph Eur, BP grade supplied by Merck KGaA (Germany) CAS 57-88-5
- Cholesterol sulphate supplied by MMP Inc. (USA) CAS 6614-96-6
- Clobetasol Propionate Micronized BP/USP grade supplied by Farmabios SpA (Italy) CAS 25122-46-7
- Clotrimazole USP/Ph.Eur grade supplied by Farchemia Srl (Italy) CAS 23593-75-1
- Cosmoperine® extract of *Piper nigrum* (Tetrahydropiperine) supplied by Sabinsa Corp. (USA)
- Erythromycin sulphate supplied by SM Biomed Sdn. Bhd. (Malaysia) CAS 114-07-8
- Eusolex 4360 (Benzophenone-3) supplied by Merck KGaA (Germany) CAS 131-57-7
- Fougere (AFL-3607/D) supplied by Arriva Fragrances (UK)
- Galanga extract of *Kaempferia galanga* (Ethyl-p-methoxycinnamate 98%) supplied by Sabinsa Corp. (USA) CAS 99880-64-5
- Hydrocortisone 17-butyrate supplied by Sigma-Aldrich Ltd (UK) CAS 13609-67-1
- Ketaconazole Ph. Eur grade supplied by Nicholas Piramal India Ltd. (India) CAS 65277-42-1
- Melaleucol (Terpinen-4-ol) supplied by SNP Natural Products Pty Ltd. (Australia) CAS 562-74-3
- Minoxidil supplied by Flamma SpA (Italy) CAS 38304-91-5
- NDGA (Nordihydroguaiaretic acid) supplied by Whyte Chemicals Ltd. (UK) CAS 500-38-9
- Neomycin sulphate was supplied by Leshan Sanjiu-LongMarch Pharmarceuticals Co., Ltd. (China) CAS 1405-10-3
- Nystatin Eur.Ph., BP grade was supplied by Antibiotice SA (Romania) CAS 1400-61-9
- PABA (4-Aminobenzoic acid extra pure) USP was supplied by Merck KGaA (Germany) CAS 150-13-0
- PT-40 Polyol soluble liquorice extract of *Glycyrrhiza glabra* was supplied by Maruzen Pharmaceuticals Co. Ltd. (Japan) CAS 84775-66-6.
- P-U Polyol soluble liquorice extract of *Glycyrrhiza inflata* was supplied by Maruzen Pharmaceuticals Co. Ltd. (Japan)
- Questice CQ U/A (Menthyl PCA) was supplied by Quest International (UK) CAS 68127-22-0
- Rosmarinic acid (90%) extract of *Melissa officinalis* was supplied by Sabinsa Corp. (USA) CAS 84604-14-8
- Soy isoflavones CG (50%) extract of *Glycine soja* was supplied by Sabinsa Corp. (USA)
- Unisex Bouquet (AFL-3607/A) was supplied by Arriva Fragrances (UK)
- Unisol S-22 (3-Benzylidene camphor) was supplied by Induchem AG (Switzerland) CAS 15087-24-8
- Vitamin D3 (cholecalciferol) cryst. Ph. Eur., BP, USP was supplied by Merck KGaA (Germany) CAS 67-97-0
- V-CP vitamin C dipalmitate derivative was supplied by Nikko Chemicals Co. Ltd. (Japan) CAS 28474-90-0
- Ketoprofen was supplied by Sigma-Aldrich Ltd. (UK) CAS 22071-15-4
- Diclofenac was supplied by Sigma-Aldrich Ltd. (UK) CAS 15307-79-6
- Naproxen was supplied by Sigma-Aldrich Ltd. (UK) CAS 22204-53-1

- Betamethasone 17-valerate Ph. Eur., USP was supplied by Farmabios Spa (Italy) CAS 2152-44-5
- Rosemary Extract CG (extract of *Rosmarinus Officinalis longa)* was supplied by Sabinsa Corp. (USA)
- Stearyl glycrrhetinate extract of *Glycyrrhiza glabra* was supplied by Maruzen Pharmaceuticals Co. Ltd. (Japan)
- THC CG (Tetrahydrocurcuminoids) extract of *Curcuma longa* was supplied by Sabinsa Corp. (USA) CAS 36062-04-1
- THC Ultra Pure (Tetrahydrocurcuminoids) extract of *Curcuma longa* supplied by Sabinsa Corp. (USA) CAS 36062-04-1
- Edemine (lecithin and escin (triterpenic saponin from *Aesculus hippocastanum))* was supplied by Vama FarmaCosmetica Srl (Italy) CAS 6805-41-0
- Capsaicin (Sigma) 8-Methyl-N-vanillyl-trans-6-nonenamide (minimum 65% capsaicin) extract of *capsicum* supplied by Sigma-Aldrich Ltd. (UK) CAS 404-86-4
- Capsaicin (Sabinsa) Capsaicinoids USP (minimum 55% capsaicin) extract of *capsicum annum* supplied by Sabinsa Corp. (USA) CAS 404-86-4
- Camphor D(+)-Camphor Ph. Eur., BP, USP was supplied by Merck KGaA CAS 464-49-3
- Spilanthes CO2 Extract (Supercritical CO2 extract of *Spilanthes oleracea)* raw material was supplied by Aayurmed Biotech Pvt. Ltd. (India)
- Vitamin E (DL-Alpha tocopherol Ph. Eur., BP, USP, E307) was supplied by Merck KGaA (Germany) CAS 10191-41-0
- Cha-Plu MeOH Extract (methanol soxhlet extraction of *Piper sarmentosum* leaves) Leaves supplied by Mr.Green Company (Thailand)
- Maca IPA Extract (isopropyl alcohol soxhlet extraction of Maca (*Lepidium meyenii*)-Maca was supplied by Plant Spirit Ltd. (UK)
- Tas. Pepper (Oshadhi) Tasmanian pepper *(Tasmannia lanceolata)* was supplied by Oshadhi Ltd. (UK)
- Hops Tincture (tincture of *Humulus lupus)* was supplied by Hambleden Herbs (UK)
- Octyl salicylate was supplied by Surfachem Ltd. (UK) CAS 118-60-5
- Ginkgo (EUK) (Dry extract of *Ginkgo biloba)* was supplied by E.U.K. Ltd. (UK) CAS 90045-36-6
- Echinacea (A.Vogel) tincture of *Echinacea pupurea* herb and root was supplied by Bioforce AG (Switzerland)
- Echinacea Purpurea IPA Extract. (isopropyl alcohol soxhlet extraction of *Echinacea purpurea)* raw material was supplied by G. Baldwin & Co. (UK)
- Tarragon Extract. ethanol:water (50:50) soxhlet extraction of *Artemisia dracunculus.* Raw material was supplied by McCormick (UK) Ltd.
- Echinacea Angust. IPA soxhlet extract of *Echinacea angustafolia.* Raw material was supplied by Naturally Green Ltd. (UK)
- Boswellia (EUK) Dry extract of *Boswellia serrata* was supplied by E.U.K. Ltd. (UK) CAS 97952-72-2
- Japanese Pepper Extract. Hexane soxhlet extraction of *Zanthoxylum piperitum.* Raw was material supplied by S&B Foods Inc. (Japan)
- Heliopsis Extract. Hexane soxhlet extraction of roots of *Heliopsis helianthoides var. scabra.*
- Eserine. (-)-phytostigmine was supplied by Sigma-Aldrich Ltd. (UK) CAS 57-47-6

Results

Table 8 below summarises the results of the experiment.

Table 8 - The ability of compositions of the invention to solubilise active agents

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Tergitol 15-S-20 | 2.5 | 90H | 1.0 | S LPC (as SL 80-3) | ca. 0.01 (0.05) | TECA | 0.40 | 10.1 | 14.17 |
| Aminofoam WOR | 2.5 | 90H | 1.0 | S LPC (as SL 80-3) | ca. 0.01 (0.05) | TECA | 0.80 | 18.4 | 10.53 |
| Protasorb L-20 | 2.5 | 90H | 1.0 | S LPC (as SL 80-3) | ca. 0.01 (0.05) | TECA | 0.80 | 18.4 | 19.71 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Amisoft MS11-F | 2.5 | 90H | 1.0 | S LPC (as SL 80-3) | ca. 0.01 (0.05) | TECA | 0.80 | 18.4 | 40.50 |
| Volpo L23 | 2.5 | 90H | 1.0 | S LPC (as SL 80-3) | ca. 0.01 (0.05) | D&C Red No. 27 | 0.10 | 2.7 | 21.85 |
| Volpo L23 | 2.5 | 90H | 1.0 | S LPC (as SL 80-3) | ca. 0.01 (0.05) | Myristidone® | 0.25 | 6.6 | 58.60 |
| Volpo L23 | 2.5 | 90H | 1.0 | S LPC (as SL 80-3) | ca. 0.01 (0.05) | Laurydone® | 0.25 | 6.6 | 61.70 |
| Volpo L23 | 2.5 | 90H | 1.0 | S LPC (as SL 80-3) | ca. 0.01 (0.05) | Ciclopirox olamine | 0.25 | 6.6 | 10.85 |
| Volpo L23 | 2.5 | 90H | 1.0 | S LPC (as SL 80-3) | ca. 0.01 (0.05) | Econazole nitrate | 0.25 | 6.6 | 71.50 |
| Volpo L23 | 2.5 | 90H | 1.0 | S LPC (as SL 80-3) | ca. 0.01 (0.05) | Bromocresol Green | 0.25 | 6.6 | 19.40 |
| Volpo L23 | 2.5 | 90H | 1.0 | S LPC (as SL 80-3) | ca. 0.01 (0.05) | Red Clover | 0.25 | 6.6 | 21.80 |
| Brij 35P | 2.5 | 90H | 1.0 | S LPC (as SL 80-3) | ca. 0.01 (0.05) | TECA | 0.50 | 12.3 | 12.56 |
| Brij 35P | 2.5 | 90H | 1.0 | S LPC (as SL 80-3) | ca. 0.01 (0.05) | TECA | 0.80 | 18.4 | 26.16# |
| Brij 35P | 2.5 | S75 | 1.0 | S LPC (as SL 80-3) | ca. 0.01 (0.05) | TECA | 0.5 | 12.3 | 12.56 |
| Brij 35P | 2.5 | 80H | 1.0 | S LPC (as SL 80-3) | ca. 0.01 (0.05) | TECA | 0.5 | 12.3 | 34.10 |
| Brij 35P | 2.5 | 90H | 1.0 | S LPC (as SL 80-3) | ca. 0.01 (0.05) | D&C Red No. 27 | 0.10 | 2.7 | 16.44 |
| Brij 35P | 2.5 | 90H | 1.0 | S LPC (as SL 80-3) | ca. 0.01 (0.05) | Centella (Herballia) | 0.25 | 6.6 | 44.40 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1.0 | S LPC (as SL 80-3) | ca. 0.01 (0.05) | Herbalia Butchers Broom | 0.25 | 6.6 | 78.60 |
| Brij 35P | 2.5 | 90H | 1.0 | S LPC (as SL 80-3) | ca. 0.01 (0.05) | Benzyl nicotinate | 0.25 | 6.6 | 18.84 |
| Brij 35P | 2.50 | 90H | 1.0 | S LPC (as SL 80-3) | ca. 0.01 (0.05) | Octopirox® | 0.25 | 6.6 | 9.10 |
| Brij 35P | 2.50 | 90H | 1.0 | S LPC (as SL 80-3) | ca. 0.01 (0.05) | Octopirox® | 0.80 | 18.4 | 6.91 |
| Brij 35P | 2.5 | 90H | 1.0 | S LPC (as SL 80-3) | ca. 0.01 (0.05) | Centella (Plantactiv) | 0.50 | 12.3 | 33.60 |
| Brij 35P | 2.5 | 90H | 1.0 | S LPC (as SL 80-3) | ca. 0.01 (0.05) | Argireline® | 0.10 | 2.7 | 7.82 |
| Brij 35P | 2.5 | 90H | 1.0 | S LPC (as SL 80-3) | ca. 0.01 (0.05) | Ginkgo | 0.25 | 6.6 | 33.80 |
| Brij 35P | 2.5 | 90H | 1.0 | S LPC (as SL 80-3) | ca. 0.01 (0.05) | Ginkgo G38 | 0.25 | 6.6 | 47.40 |
| Brij 35P | 2.5 | 90H | 1.0 | S LPC (as SL 80-3) | ca. 0.01 (0.05) | Ginkgo GSF | 0.25 | 6.6 | 59.60 |
| Brij 35P | 2.5 | 90H | 1.0 | S LPC (as SL 80-3) | ca. 0.01 (0.05) | Horse Chestnut | 0.25 | 6.6 | 31.40 |
| Brij 35P | 2.5 | 90H | 1.0 | S LPC (as SL 80-3) | ca. 0.01 (0.05) | Herballia Nettle | 0.25 | 6.6 | 18.54 |
| Brij 35P | 2.5 | 90H | 1.0 | S LPC (as SL 80-3) | ca. 0.01 (0.05) | Plantactiv Aesculus | 0.25 | 6.6 | 26.80 |
| Brij 35P | 2.5 | 90H | 1.0 | S LPC (as SL 80-3) | ca. 0.01 (0.05) | Yohimbine HCl Crystalline | 0.25 | 6.6 | 9.85 |
| Brij 35P | 2.5 | 90H | 1.0 | S LPC (as SL 80-3) | ca. 0.01 (0.05) | Yohimbine HCl USP | 0.25 | 6.6 | 13.42 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1.0 | S LPC (as SL 80-3) | ca. 0.01 (0.05) | Yohimbine 10% Extract | 0.25 | 6.6 | 68.90 |
| Brij 35P | 2.5 | 90H | 1.0 | S LPC (as SL 80-3) | ca. 0.01 (0.05) | Yohimbne HCl USP HO | 0.20 | 5.3 | 22.78 |
| Brij 35P | 2.5 | 90H | 1.0 | S LPC (as SL 80-3) | ca. 0.01 (0.05) | Hydrocortisone | 0.25 | 6.6 | 10.00 |
| Brij 35P | 2.5 | 90H | 1.0 | S LPC (as SL 80-3) | ca. 0.01 (0.05) | Salmeterol xinafoate | 0.25 | 6.6 | 5.49 |
| Brij 35P | 2.5 | 90H | 1.0 | S LPC (as SL 80-3) | ca. 0.01 (0.05) | Progesterone | 0.10 | 2.7 | 34.20† |
| Brij 35P | 2.5 | 90H | 1.0 | S LPC (as SL 80-3) | ca. 0.01 (0.05) | Devils Claw Extract | 0.25 | 6.6 | 10.49 |
| Brij 35P | 2.5 | 90H | 1.0 | S LPC (as SL 80-3) | ca. 0.01 (0.05) | Gatulne® Expression | 0.25 | 6.6 | 15.11 |
| Brij 35P | 2.5 | 90H | 1.0 | S LPC (as SL 80-3) | ca. 0.01 (0.05) | Extract of *Picea abies* | 0.80 | * | * |
| Brij 35P | 2.5 | 90H | 1.0 | S LPC (as SL 80-3) | ca. 0.01 (0.05) | Red Clover | 0.40 | 10.1 | 59.20 |
| Brij 35P | 2.5 | 90H | 1.0 | S LPC (as SL 80-3) | ca. 0.01 (0.05) | Camphor | 0.25 | 6.6 | 26.80 |
| Brij 35P | 2.5 | 90H | 1.0 | S LPC (as SL 80-3) | ca. 0.01 (0.05) | Totarol® | 0.25 | 6.6 | 12.89 |
| Brij 35P | 2.5 | 90H | 1.0 | S LPC (as SL 80-3) | ca. 0.01 (0.05) | Jambu Extract | 0.25 | 6.6 | 45.00 |
| Brij 35P | 2.5 | 90H | 1.0 | S LPC (as SL 80-3) | ca. 0.01 (0.05) | SepiWhite MSH | 0.25 | 6.6 | 10.43 |
| Brij 35P | 2.5 | 90H | 1.0 | S LPC (as SL 80-3) | ca. 0.01 (0.05) | Phyto-Age | 0.25 | 6.6 | 15.48 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1.0 | S LPC (as SL 80-3) | ca. 0.01 (0.05) | Prickly Ash Tincture | 0.25 | 6.6 | 15.46 |
| Brij 35P | 2.5 | 90H | 1.0 | S LPC (as SL 80-3) | ca. 0.01 (0.05) | Prickly Ash Tincture | 1.00 | 22.0 | 60.95 |
| Brij 35P | 2.5 | 90H | 1.0 | S LPC (as SL 80-3) | ca. 0.01 (0.05) | Boswellin® CG | 0.20 | 5.3 | 54.00 |
| Proteol OAT | 2.5 | 90H | 1.0 | S LPC (as SL 80-3) | ca. 0.01 (0.05) | Sichuan Pepper Extract | 0.25 | 6.6 | 21.65 |
| Proteol OAT | 2.5 | 90H | 1.0 | S LPC (as SL 80-3) | ca. 0.01 (0.05) | Prickly Ash Tincture | 0.25 | 6.6 | 18.76 |
| | | | | | | | | | |
| Sodium lauryl sulphate | 2.5 | 90H | 1.0 | S LPC (as SL 80-3) | ca. 0.01 (0.05) | TECA | 0.2 | 5.3 | 18.07 |
| Sodium lauryl sulphate | 2.5 | 90H | 1.0 | S LPC (as SL 80-3) | ca. 0.01 (0.05) | TECA | 0.5 | 12.3 | 24.2 |
| Sodium lauryl sulphate | 2.5 | 90H | 1.0 | S LPC (as SL 80-3) | ca. 0.01 (0.05) | TECA | 0.8 | 18.4 | 84.9 |
| Sodium cholate | 2.5 | 90H | 1.0 | S LPC (as SL 80-3) | ca. 0.01 (0.05) | TECA | 0.2 | 5.3 | 49.9 |
| Sodium cholate | 2.5 | 90H | 1.0 | S LPC (as SL 80-3) | ca. 0.01 (0.05) | TECA | 0.5 | 12.3 | 47.8 |
| Sodium cholate | 2.5 | 90H | 1.0 | S LPC (as SL 80-3) | ca. 0.01 (0.05) | TECA | 0.8 | 18.4 | >150 |
| Sodium deoxycholate | 2.5 | 90H | 1.0 | S LPC (as SL 80-3) | ca. 0.01 (0.05) | TECA | 0.2 | 5.3 | 49.8 |
| Sodium deoxycholate | 2.5 | 90H | 1.0 | S LPC (as SL 80-3) | ca. 0.01 (0.05) | TECA | 0.5 | 12.3 | >150 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Sodium deoxycholate | 2.5 | 90H | 1.0 | S LPC (as SL 80-3) | ca. 0.01 (0.05) | TECA | 0.8 | 18.4 | >150 |
| # This formulation was subsequently freeze-dried.<br>† This formulation was subsequently dried by rotary evaporation.<br>* Undisclosed. | | | | | | | | | |

Table 8 (supplemental) - The ability of compositions of the invention to solubilise active agents

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1.0 | S LPC (as SL 80-3) | ca. 0.01 (0.05) | Capsaicin (Sigma) | 0.1 | 2.7 | 14.55 |
| Brij 35P | 2.5 | 90H | 1.0 | S LPC (as SL 80-3) | ca. 0.01 (0.05) | Capsaicin (Sabinsa) | 0.1 | 2.7 | 8.41 |
| Brij 35P | 2.5 | 90H | 1 | S LPC (as SL 80-3) | ca. 0.01 (0.05) | 7-dehydro -cholesterol | 0.10 | 2.7 | 58.4 |
| Brij 35P | 2.5 | 90H | 1 | S LPC (as SL 80-3) | ca. 0.01 (0.05) | Apricosal | 0.25 | 6.6 | 11.49 |
| Brij 35P | 2.5 | 90H | 1 | S LPC (as SL 80-3) | ca. 0.01 (0.05) | Ascorbyl palmitate | 0.10 | 2.7 | 72.4 |
| Brij 35P | 2.5 | 90H | 1 | S LPC (as SL 80-3) | ca. 0.01 (0.05) | Avobenzene | 0.10 | 2.7 | 52.9 |
| Brij 35P | 2.5 | 90H | 1 | S LPC (as SL 80-3) | ca. 0.01 (0.05) | Boswellin CG | 0.10 | 2.7 | 54.0 |
| Brij 35P | 2.5 | 90H | 1 | S LPC (as SL 80-3) | ca. 0.01 (0.05) | Caffeine | 0.25 | 6.6 | 14.96 |
| Brij 35P | 2.5 | 90H | 1 | S LPC (as SL 80-3) | ca. 0.01 (0.05) | Cholesterol | 0.10 | 2.7 | 10.91 |
| Brij 35P | 2.5 | 90H | 1 | S LPC (as SL 80-3) | ca. 0.01 (0.05) | Cholesterol sulphate | 0.10 | 2.7 | 9.48 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1 | S LPC (as SL 80-3) | ca. 0.01 (0.05) | Clobetasol Propionate | 0.10 | 2.7 | 23.3 |
| Brij 35P | 2.5 | 90H | 1 | S LPC (as SL 80-3) | ca. 0.01 (0.05) | Clotrimazole | 0.10 | 2.7 | 10.4 |
| Brij 35P | 2.5 | 90H | 1 | S LPC (as SL 80-3) | ca. 0.01 (0.05) | Cosmoperine | 0.10 | 2.7 | 6.38 |
| Brij 35P | 2.5 | 90H | 1 | S LPC (as SL 80-3) | ca. 0.01 (0.05) | Erythromycin sulphate | 0.10 | 2.7 | 8.56 |
| Brij 35P | 2.5 | 90H | 1 | S LPC (as SL 80-3) | ca. 0.01 (0.05) | Eusolex 4360 | 0.10 | 2.7 | 10.02 |
| Brij 35P | 2.5 | 90H | 1 | S LPC (as SL 80-3) | ca. 0.01 (0.05) | Fougere | 0.25 | 6.6 | 5.44 |
| Brij 35P | 2.5 | 90H | 1 | S LPC (as SL 80-3) | ca. 0.01 (0.05) | Galanga extract | 0.10 | 2.7 | 8.18 |
| Brij 35P | 2.5 | 90H | 1 | S LPC (as SL 80-3) | ca. 0.01 (0.05) | Hydrocortisone -17 butyrate | 0.10 | 2.7 | 55.9 |
| Brij 35P | 2.5 | 90H | 1 | S LPC (as SL 80-3) | ca. 0.01 (0.05) | Ketaconazole | 0.40 | 10.1 | 11.12 |
| Brij 35P | 2.5 | 90H | 1 | S LPC (as SL 80-3) | ca. 0.01 (0.05) | Melaleucol | 0.10 | 2.7 | 19.4 |
| Brij 35P | 2.5 | 90H | 1 | S LPC (as SL 80-3) | ca. 0.01 (0.05) | Minoxidil | 0.10 | 2.7 | 13.67 |
| Brij 35P | 2.5 | 90H | 1 | S LPC (as SL 80-3) | ca. 0.01 (0.05) | NDGA | 0.10 | 2.7 | 40.5 |
| Brij 35P | 2.5 | 90H | 1 | S LPC (as SL 80-3) | ca. 0.01 (0.05) | Neomycin sulphate | 0.10 | 2.7 | 19.76 |
| Brij 35P | 2.5 | 90H | 1 | S LPC (as SL 80-3) | ca. 0.01 (0.05) | Nystatin | 0.10 | 2.7 | 21.3 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1 | S LPC (as SL 80-3) | ca. 0.01 (0.05) | PABA | 0.10 | 2.7 | 8.81 |
| Brij 35P | 2.5 | 90H | 1 | S LPC (as SL 80-3) | ca. 0.01 (0.05) | PT-40 | 0.25 | 6.6 | 21.5 |
| Brij 35P | 2.5 | 90H | 1 | S LPC (as SL 80-3) | ca. 0.01 (0.05) | P-U | 0.10 | 2.7 | 18.45 |
| Brij 35P | 2.5 | 90H | 1 | S LPC (as SL 80-3) | ca. 0.01 (0.05) | Questice CQ U/A | 0.25 | 6.6 | 46.81 |
| Brij 35P | 2.5 | 90H | 1 | S LPC (as SL 80-3) | ca. 0.01 (0.05) | Rosmarinic acid (90%) | 0.10 | 2.7 | 31.5 |
| Brij 35P | 2.5 | 90H | 1 | S LPC (as SL 80-3) | ca. 0.01 (0.05) | Soy isoflavones (50%) CG | 0.10 | 2.7 | 7.01 |
| Brij 35P | 2.5 | 90H | 1 | S LPC (as SL 80-3) | ca. 0.01 (0.05) | Unisex Bouquet | 0.10 | 2.7 | 56.6 |
| Brij 35P | 2.5 | 90H | 1 | S LPC (as SL 80-3) | ca. 0.01 (0.05) | Unisol S-22 | 0.15 | 4.05 | 14.59 |
| Brij 35P | 2.5 | 90H | 1 | S LPC (as SL 80-3) | ca. 0.01 (0.05) | Vitamin $D_3$ | 0.25 | 6.6 | 11.62 |
| Brij 35P | 2.5 | 90H | 1 | S LPC (as SL 80-3) | ca. 0.01 (0.05) | V-CP | 0.10 | 2.7 | 29.8 |
| Brij 35P | 2.5 | 90H | 1 | S LPC (as SL 80-3) | ca. 0.01 (0.05) | Ketoprofen | 0.10 | 2.7 | 8.05 |
| Brij 35P | 2.5 | 90H | 1 | S LPC (as SL 80-3) | ca. 0.01 (0.05) | Diclofenac | 0.10 | 2.7 | 14.39 |
| Brij 35P | 2.5 | 90H | 1 | S LPC (as SL 80-3) | ca. 0.01 (0.05) | Naproxen | 0.10 | 2.7 | 14.99 |
| Brij 35P | 2.5 | 90H | 1 | S LPC (as SL 80-3) | ca. 0.01 (0.05) | Betamethasone 17-valerate | 0.10 | 2.7 | 19.24 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1 | S LPC (as SL 80-3) | ca. 0.01 (0.05) | Rosemary Extract CG | 0.10 | 2.7 | 29.1 |
| Brij 35P | 2.5 | 90H | 1 | S LPC (as SL 80-3) | ca. 0.01 (0.05) | Stearyl glycrrhetinate | 0.10 | 2.7 | 88.9 |
| Brij 35P | 2.5 | 90H | 1 | S LPC (as SL 80-3) | ca. 0.01 (0.05) | THC CG | 0.10 | 2.7 | 16.42 |
| Brij 35P | 2.5 | 90H | 1 | S LPC (as SL 80-3) | ca. 0.01 (0.05) | THC Ultra Pure | 0.10 | 2.7 | 14.89 |
| Brij 35P | 2.5 | - | - | - | - | Edemine◊ | 2.0 | 44.44 | 10.30 |
| Brij 35P | 2.5 | 90H | 1.0 | S LPC (as SL 80-3) | ca. 0.01 (0.05) | Capsaicin (Sigma) | 0.10 | 2.7 | 14.55 |
| Brij 35P | 2.5 | 90H | 1.0 | S LPC (as SL 80-3) | ca. 0.01 (0.05) | Capsaicin (Sabinsa) | 0.10 | 2.7 | 8.41 |
| Brij 35P | 2.5 | 90H | 1.0 | S LPC (as SL 80-3) | ca. 0.01 (0.05) | Camphor (S-Black) | 0.10 | 2.7 | 29.70 |
| Protasorb L-20 | 2.5 | 90H | 1.0 | S LPC (as SL 80-3) | ca. 0.01 (0.05) | Spilanthes $CO_2$ Extract | 0.25 | 6.6 | 27.1 |
| Protasorb L-20 | 2.5 | 90H | 1.0 | S LPC (as SL 80-3) | ca. 0.01 (0.05) | Spilanthes $CO_2$ Extract | 0.50 | 12.3 | 49.3 |
| Amisoft LS-11 F | 2.5 | 90H | 1.0 | S LPC (as SL 80-3) | ca. 0.01 (0.05) | Spilanthes $CO_2$ Extract | 0.25 | 6.6 | 16.77 |
| Surmope C-1218 | 2.5 | 90H | 1.0 | S LPC (as SL 80-3) | ca. 0.01 (0.05) | Spilanthes $CO_2$ Extract | 0.25 | 6.6 | 66.7 |
| Protasorb L-20 | 2.5 | 90H | 1.0 | S LPC (as SL 80-3) | ca. 0.01 (0.05) | Vitamin E | 0.10 | 2.7 | 29.60 |
| Amisoft CS-11F | 2.5 | 90H | 1.0 | S LPC (as SL 80-3) | ca. 0.01 (0.05) | Cha-Plu MeOH Extract | 0.20 | 5.3 | >150* |
| Amisoft CS-11F | 2.5 | 90H | 1.0 | S LPC (as SL 80-3) | ca. 0.01 (0.05) | Maca IPA Extract | 0.25 | 6.6 | 58.2 |

(continued)

| Surf. | Surf. % | Lipid | Lipid % | Co-surf. | Co-surf. % | Active Agent | Active Agent % | Active Agent % by dry weight | Turbidity (FNU) |
|---|---|---|---|---|---|---|---|---|---|
| Brij 35P | 2.5 | 90H | 1.0 | S LPC (as SL 80-3) | ca. 0.01 (0.05) | Tas. Pepper (Oshandi) | 0.25 | 6.6 | 107.1 |
| Brij 35P | 2.5 | 90H | 1.0 | S LPC (as SL 80-3) | ca. 0.01 (0.05) | Hops Tincture | (0.60) eqv 0.30 | 8.1 | 23.5 |
| Brij 35P | 2.5 | 90H | 1.0 | S LPC (as SL 80-3) | ca. 0.01 (0.05) | Octyl Salicylate | 0.10 | 2.7 | 65.6 |
| Brij 35P | 2.5 | 90H | 1.0 | S LPC (as SL 80-3) | ca. 0.01 (0.05) | Ginkgo (EUK) | 0.25 | 6.6 | 10.79 |
| Brij 35P | 2.5 | 90H | 1.0 | S LPC (as SL 80-3) | ca. 0.01 (0.05) | Echinacea (A.Vogel) | 0.25 | 6.6 | 9.53 |
| Brij 35P | 2.5 | 90H | 1.0 | S LPC (as SL 80-3) | ca. 0.01 (0.05) | *Echinacea purpurea* IPA Extract | 0.25 | 6.6 | 32.4 |
| Brij 35P | 2.5 | 90H | 1.0 | S LPC (as SL 80-3) | ca. 0.01 (0.05) | Tarragon Extract (EtOH: $H_2O$) | 0.25 | 6.6 | 18.65 |
| Brij 35P | 2.5 | 90H | 1.0 | S LPC (as SL 80-3) | ca. 0.01 (0.05) | *Echinacea angust.* IPA Extract | 0.25 | 6.6 | 75.0 |
| Brij 35P | 2.5 | 90H | 1.0 | S LPC (as SL 80-3) | ca. 0.01 (0.05) | Boswellia (EUK) | 0.10 | 2.7 | 17.66 |
| Brij 35P | 2.5 | 90H | 1.0 | S LPC (as SL 80-3) | ca. 0.01 (0.05) | Japanese Pepper Extract | 0.25 | 6.6 | 59.9 |
| Brij 35P | 2.5 | 90H | 1.0 | S LPC (as SL 80-3) | ca. 0.01 (0.05) | *Heliopsis helianthoides var. scabra* | 0.20 | 5.3 | 37.4 |
| Brij 35P | 2.5 | 90H | 1.0 | S LPC (as SL 80-3) | ca. 0.01 (0.05) | Esenne* | 0.10 | 2.7 | 120.54** |

◊ Edemine is a combined lecithin and escin product, containing a minimum escin concentration 12%.
* Cha-Plu sample was noted as being of high clarity by visual inspection, due to intense green colour of the solution turbidity measurement registers higher than would normally be associated with such clarity levels.
** Eserine sample was of very high clarity by visual inspection, due to intense red colour of the solution turbidity measurement registers higher than would normally be associated with such clarity levels.

Compositions of the present invention, based on a range of surfactant and lipid components, demonstrate a potent ability to solubilise active agents with known poor water solubility to form clear and colourless aqueous solutions. For example, the exemplary active agent TECA was solubilised at 0.8%, equivalent to approximately 18.5% of the total

dry weight (80% of lipid weight). Comparative Example 2 indicated that none of the four common surfactants SDS, Mackanate, F127, S LPC were capable of solubilising TECA at 9.1 % of total dry weight irrespective of any other potential problems these surfactants may have.

Comparative Example 2 also shows that Brij 35P (3.5%) is unable to solubilise TECA at 12.5% dry weight, while Comparative Example 3 shows that the lipid 90H (3.5%) is unable to solubilise TECA at 12.5% dry weight. As such, it is evident that a synergistic interaction between the surfactant and lipid component results in the formation of a macromolecular complex having a solubilising capability well in excess of that for the equivalent quantity of either individual component in isolation. It is extremely surprising that the aqueous solubility of an active agent with poor water solubility may actually be *increased* by the addition of further material which has poor water solubility (i.e. the lipid).

Of those samples which were dried, all were easily reconstituted into water at the same concentration as prior to drying. This stability on processing is of value in commercial applications, where the transfer of dried formulations may significantly reduce transportation and handling costs.

The solubilisation levels described above may not be limiting and are merely illustrative of the surprising potency of the present invention in solubilisation of hydrophobic agents in aqueous media without the need for undesirable excipients. Therefore the possibility exists that active agents may be solubilised by the compositions of the invention at higher levels than those indicated.

Reconstitution of the freeze-dried composition containing Brij 35P (2.5%), 90H (1%), SL 80-3 (0.05%) and TECA (0.8%), indicated in the table above by #, was successful at a concentration of 32.4% by dry weight (i.e. an overall concentration of 6% active in the final aqueous solution). This finding indicates that formulations of varying concentration may be prepared from a single freeze-dried stock and demonstrates the high quantity of compositions of the invention which may be present in a solution.

The rotary evaporated composition containing Brij 35P (2.5%), 90H (1%), SL 80-3 (0.05%) and progesterone (0.1 %), indicated in the table above by †, was reconstituted in water to provide a clear and colourless solution with a progesterone concentration of 0.25% (i.e. 2500 ug/ml, 2.7% progesterone by dry weight). This may be contrasted, for example, with the delivery system described in WO00/50007 (Lipocine Inc), where a maximum progesterone concentration of 1760 ug/ml was obtained at notably higher carrier concentration (see Example 47 in WO00/50007).

It may be noted that the compositions of the present invention provide comparable solubilisation potential to corresponding compositions comprising the potent and irritant surfactant sodium lauryl sulphate. Compositions of the present invention exceed the solubilisation potential of corresponding compositions comprising the potent and irritant surfactants sodium cholate and sodium deoxycholate, neither of which were capable of producing clear solutions of TECA at a dry weight of 18.4% (sodium deoxycholate also failing to do so at a dry weight of 12.3%).

**Example 5 - Optimum surfactant/lipid ratios**

The effect of variation in the relative amount of surfactant present in compositions of the invention was investigated.

<u>Method</u>

A range of aqueous solutions of compositions of the invention containing lipid (90H), surfactant (Brij 35P), co-surfactant (indirectly, in the form of SL 80-3) and active agent (TECA) were prepared in water analogously to previously described methods.

For a quantitative analysis of the clarity of aqueous solutions of surfactant and lipid, samples were examined using a turbidity meter (Nephla, from Hach-Lange). The turbidity meter was calibrated prior to use, with two known standards (0 and 40 FNU).

<u>Results</u>

The results of the experiment are summarised in Table 9.

Table 9 - The effect of variation in surfactant/lipid ratio on turbidity

| Active (TECA) % w/w | Surfactant (Brij 35P) % w/w | Lipid | | | Surfactant/ Lipid Ratio | Turbidity FNU |
|---|---|---|---|---|---|---|
| | | 90H % w/w | SL 80-3 % w/w | Total % w/w | | |
| **Active (TECA)** | **Surfactant** | **Lipid** | | | **Surfactant/ Lipid** | **Turbidity** |
| 0.8 | 2.5 | 0 | 0 | 0 | - | >150 |
| 0.8 | 7.5 | 0.95 | 0.05 | 1.0 | 7.5:1.0 | 30.7 |
| 0.8 | 5.0 | 0.95 | 0.05 | 1.0 | 5.0:1.0 | 35.8 |
| 0.8 | 3.0 | 0.95 | 0.05 | 1.0 | 3.0:1.0 | 38.9 |
| 0.8 | 2.5 | 0.95 | 0.05 | 1.0 | 2.5:1.0 | 32.0 |
| 0.8 | 2.0 | 0.95 | 0.05 | 1.0 | 2.0:1.0 | 40.0 |
| 0.8 | 1.8 | 0.95 | 0.05 | 1.0 | 1.8:1.0 | 47.8 |
| 0.8 | 1.5 | 0.95 | 0.05 | 1.0 | 1.5:1.0 | 42.3 |
| 0.8 | 1.25 | 0.95 | 0.05 | 1.0 | 1.25:1.0 | 88.1 |
| 0.8 | 1.0 | 0.95 | 0.05 | 1.0 | 1.0:1.0 | 88.6 |
| 0.8 | 0.75 | 0.95 | 0.05 | 1.0 | 0.75:1.0 | >150 |
| 0.8 | 2.5 | 0.570 | 0.030 | 0.6 | 2.5:0.6 | 38.0 |
| 0.8 | 2.5 | 0.665 | 0.035 | 0.7 | 2.5:0.7 | 38.6 |
| 0.8 | 2.5 | 0.760 | 0.040 | 0.8 | 2.5:0.8 | 32.4 |
| 0.8 | 2.5 | 0.855 | 0.045 | 0.9 | 2.5:0.9 | 41.4 |
| 0.8 | 2.5 | 0.95 | 0.050 | 1.0 | 2.5:1.0 | 32.0 |
| 0.8 | 2.5 | 1.045 | 0.055 | 1.1 | 2.5:1.1 | 40.9 |
| 0.8 | 2.5 | 1.140 | 0.060 | 1.2 | 2.5:1.2 | 38.0 |
| 0.8 | 2.5 | 1.425 | 0.075 | 1.2 | 2.5:1.5 | 38.5 |
| 0.8 | 2.5 | 2.85 | 0.15 | 1.2 | 2.5:3.0 | >150 |
| 0.9 | 2.5 | 0.57 | 0.03 | 0.6 | 2.5:0.6 | 36.2 |
| 0.9 | 2.0 | 0.57 | 0.03 | 0.6 | 2:0.6 | 41.0 |
| 0.9 | 1.875 | 0.57 | 0.03 | 0.6 | 1.875:0.6 | 105.2 |
| 0.9 | 1.75 | 0.57 | 0.03 | 0.6 | 1.75:0.6 | >150 |

Discussion

As can be seen from the data in Table 9, the clarity of a solution containing the compositions of the invention is improved by increasing the quantity of surfactant relative to the quantity of lipid which is present.

In the case of solutions fixed with 0.8% TECA and 1.0% lipid, solutions with good clarity levels (i.e. <100 FNU) are obtained with a surfactant/lipid ratio of 1:1, with high clarity levels (i.e. <50 FNU) being obtained for a surfactant/lipid ratio of 1.5:1. No notable improvement results from the presence of additional surfactant beyond a level 1.5:1 level.

In the case of solutions fixed with 0.8% TECA and 2.5% surfactant, high clarity levels (i.e. <50 FNU) are obtained for a surfactant/lipid ratio of 1.25:1, with no notable improvement resulting from the presence of additional surfactant beyond this level.

For solutions fixed with 0.9% TECA and 0.6% lipid, high clarity levels are obtained for a surfactant/lipid ratio of 3.3:1 with little improvement resulting from the presence of additional surfactant beyond this level. It may be noted that such compositions contain ca. 25% TECA by dry weight, approaching the solubility limit of TECA in the compositions of the invention, which may explain why an increased proportion of surfactant is required to form the macromolecular assemblies relative to samples containing lower amounts of TECA.

**Example 6 - Formation of macromolecular complexes of the invention in alcohol**

For commercial applications where it is desirable to prepare large quantities of dried composition, for convenient transport or storage, it is desirable to utilise a solvent which can more easily be removed than water.

Method

Co-surfactant in the form of SL 80-3 (0.05 g) was added to ethanol (30 g) and heated under stirring to approximately 30°C for around 5 minutes. Lipid (90H, 1.0 g) was then added, with heating and stirring maintained for a further 5 minutes. Subsequently, the exemplary active agent TECA (0.5 g) was added, with heating and stirring maintained for an additional 5 minutes. Finally, surfactant (Brij 35P, 2.5 g) was added to the Lipid/TECA alcohol solution with the solution stirred under heating for a further 5 minutes.
Ethanol was removed from the sample via rotary evaporation (BUCHI Rotavapor RE111).

Results

The dried material was re-solubilised into water at the desired concentration.

Discussion

The modified manufacturing process is beneficial for the production of dry samples. Furthermore, all components may be added to the solvent together if desired.
To ensure that the macromolecular complexes are stable when reconstituted in water, it is necessary to use component ratios suitable for the preparation of aqueous solutions of the macromolecular assemblies of the invention rather than component ratios which may be soluble in the chosen solvent system.

**Example 7 - Solubilisation of Octopirox**® (piroctone olamine)

In order to demonstrate the potential of the compositions of the present invention in the preparation of aqueous solutions of hydrophobic active agents, a preparation using piroctone olamine was made.

Method

Macromolecular assemblies of the present invention which incorporated piroctone olamine were prepared in isopropanol using a method analogous to that described in Example 6 (in that case using ethanol solvent), before being dried by rotary evaporation. The dried material having the composition:

Surfactant - 15 g Brij 35P
Lipid-6g 90H
Co-surfactant (indirectly) - 0.3 g SL 80-3
Active agent - 4.8 g piroctone olamine
i.e. total piroctone olamine by weight = 18.4%

The dried material was then re-solubilised into 50 g of water to provide a concentrated solution (i.e. using the present invention enables aqueous formulations containing at least 9.6% piroctone olamine by weight to be prepared).
The concentrated solution was subsequently diluted to provide a final solution containing 15% surfactant by weight (i.e. 4.8% piroctone olamine by weight).

Discussion

Piroctone olamine is available under the tradename Octopirox® from Clariant. In a promotional brochure 'Taking Care of Your Customers' Hair', published in 2005, the manufacturer summarises the solubility of piroctone olamine in a number of solvent systems:

| | |
|---|---|
| Water | 0.05% |
| Glycerol | 1.7% |
| PEG-400 | 1.9% |

(continued)

| | |
|---|---|
| Isopropanol | 5.0% |
| Ethanol | 10.0% |
| 1,2-Propyleneglycol | 16.0% |

A comparison of the published solubilities with that enabled by the present invention illustrates the potential of the inventive compositions. A similar concentration of piroctone olamine can be obtained in an aqueous solution using the inventive system as is obtained using pure ethanol as solvent.

Octopirox® manufacturer's brochure also describes the aqueous solubility of piroctone olamine using a number of surfactants. For example, at a concentration of 15% SDS, at pH 7 and room temperature, piroctone olamine is soluble at approximately 2.8%. At an equivalent surfactant concentration of 15% the compositions of the invention enable the preparation of clear aqueous solutions with at least 4.8% piroctone olamine by weight.

## Example 8 - Long-term stability of compositions of the invention

Bilayer discs described in the prior art suffer from instability or require the presence of specific materials to ensure stability. The long-term stability of an aqueous composition of the present invention was determined for comparative purposes.

Method

The sample utilised in this experiment was prepared during the experiments described in previous examples. Following the initial turbidity testing, the sample was stored in the dark at room temperature until final turbidity testing.

The sample was examined using a turbidity meter (Nephla, from Hach-Lange). The turbidity meter was calibrated prior to use, with two known standards (0 and 40 FNU).

Results

Long term Stability data is shown in Table 10 below.

Table 10 - Long term stability of aqueous solutions of the macromolecular assemblies of the present invention

| Surf. | Surf. % | Lipid | Lipid % | Co-surf. | Co-surf. % | Active Agent | Active Agent % | Initial Turbidity (FNU) | Final Turbidity (FNU) | Storage Time |
|---|---|---|---|---|---|---|---|---|---|---|
| Volpo L23 | 2.5 | 90H | 1.0 | S LPC (as SL 80-3) | ca. 0.01 (0.05) | Ciclopirox olamine | 0.25 | 10.86 | 11.58 | >8 months |

Discussion

Surprisingly and in contrast with the expectations of one skilled in the art, aqueous formulations of the compositions of the present invention demonstrate good long term stability, with no notable change in sample turbidity over a period of at least 8 months.

Although not shown here, an extensive range of samples containing other active agents were also stored. The other samples did not contain any preservative agents and there was notable growth within these other samples which could alter the significance of long term turbidity results. Ciclopirox olamine is an antimycotic/antibacterial and no growth was observed in this sample.

This finding is of significance for many practical applications, since cosmetic or pharmaceutical products desirably have a long shelf-life.

## Example 9 - Particle size analysis

A range of aqueous compositions of the invention were tested to determine the size of the macromolecular

assemblies which are present.

Method

All test samples were prepared in water as described in Examples 1-5.

Particle sizing measurements were carried out using a 10mm disposable sizing cuvette and a Malvern Zetasizer Nano ZS.

The instrument was set up to seek the optimum attenuator and measurement position. The measurement duration was set to automatic and five repeat measurements were taken at 25°C.

The sample viscosity used was 0.8896 cP (equivalent to water); the dispersant refractive index used was 1.330 (equivalent to water).

*Principal Particle Size*

The principal particle size is the intensity mean of the peak corresponding to the predominant particle size detected.

*Polydispersity index*

The polydispersity index (PDI) is calculated from the cumulants analysis as described in ISO 13321. It is a dimensionless estimate of the width of the distribution and is scaled such that values smaller than 0.05 are rarely seen other than in latex standards. Values greater than 0.7 indicates that the sample has a very broad size distribution and is probably not suitable for the technique. The maximum value is arbitrarily limited to 1.0.

Results

The results of particle size experiments are shown in the following tables.

Table 11a - Exemplary aqueous compositions containing surfactant, lipid and co-surfactant, where the identity of the surfactant and lipid are varied

| Surf. | Surf. % | Lipid | Lipid % | Co-surf. | Co-surf. % | HLB | Turbidity (FNU) | Particle Size (nm) | PDI |
|---|---|---|---|---|---|---|---|---|---|
| Brij 58 | 2.5 | 90H | 0.95 | S LPC (as SL 80-3) | ca. 0.01 (0.05) | 15.7 | 19.45 | 10.59 | 0.359 |
| Symp.AIC 200N | 2.5 | 90H | 0.95 | S LPC (as SL 80-3) | ca. 0.01 (0.05) | 15.7 | 6.58 | 13.44 | 0.211 |
| Brij 35P | 2.5 | 90H | 0.95 | S LPC (as SL 80-3) | ca. 0.01 (0.05) | 16.9 | 14.74 | 11.0 | 0.452 |
| Protasorb O-20 | 2.5 | 90H | 0.95 | S LPC (as SL 80-3) | ca. 0.01 (0.05) | 15.0 | >150 | 1107 | 0.853 |
| Protasorb L-20 | 2.5 | 90H | 0.95 | S LPC (as SL 80-3) | ca. 0.01 (0.05) | 16.7 | 21.2 | 16.98*1 | 0.363 |
| Triton X-165 | 2.5 | 90H | 0.95 | S LPC (as SL 80-3) | ca. 0.01 (0.05) | 15.8 | 15.15 | 12.23 | 0.301 |
| Protachem DGS | 2.5 | 90H | 0.95 | S LPC (as SL 80-3) | ca. 0.01 (0.05) | 4.5 | 115 | 471.9 | 1.0 |

(continued)

| Surf. | Surf. % | Lipid | Lipid % | Co-surf. | Co-surf. % | HLB | Turbidity (FNU) | Particle Size (nm) | PDI |
|---|---|---|---|---|---|---|---|---|---|
| Symp.AIC 200N | 2.5 | S-75 | 0.95 | S LPC (as SL 80-3) | ca. 0.01 (0.05) | 15.7 | >150 | 644.6 | 0.616 |
| Protasorb L-20 | 2.5 | S-75 | 0.95 | S LPC (as SL 80-3) | ca. 0.01 (0.05) | 16.7 | >150 | 804.8 | 0.809 |
| Brij 35P | 2.5 | S-75 | 0.95 | S LPC (as SL 80-3) | ca. 0.01 (0.05) | 16.9 | 6.47 | 54.38 | 0.319 |
| Protasorb L-20 | 2.5 | SL80 | 0.95 | S LPC (as SL 80-3) | ca. 0.01 (0.05) | 16.7 | >150 | 90.26 | 0.808 |
| Brij 35P | 2.5 | SL80 | 0.95 | S LPC (as SL 80-3) | ca. 0.01 (0.05) | 16.9 | 10.37 | 7.86 | 0.533 |
| Symp.AIC 200N | 2.5 | EM 970 | 0.95 | S LPC (as SL 80-3) | ca. 0.01 (0.05) | 15.7 | >150 | 195.7 | 0.466 |
| Triton X-165 | 2.5 | EM 970 | 0.95 | S LPC (as SL 80-3) | ca. 0.01 (0.05) | 15.8 | >150 | 150.3 | 0.387 |
| Protasorb L-20 | 2.5 | EM 970 | 0.95 | S LPC (as SL 80-3) | ca. 0.01 (0.05) | 16.7 | >150 | 349 | 0.333 |
| Brij 35P | 2.5 | EM 970 | 0.95 | S LPC (as SL 80-3) | ca. 0.01 (0.05) | 16.9 | >150 | 279 | 0.606 |
| Symp.AIC 200N | 2.5 | EP 145V | 0.95 | S LPC (as SL 80-3) | ca. 0.01 (0.05) | 15.7 | 79.5 | 9.105 | 0.718 |
| Triton X-165 | 2.5 | EP 145V | 0.95 | S LPC (as SL 80-3) | ca. 0.01 (0.05) | 15.8 | 71.4 | 31.01 | 0.557 |
| Protasorb L-20 | 2.5 | EP 145V | 0.95 | S LPC (as SL 80-3) | ca. 0.01 (0.05) | 16.7 | >150 | 307 | 0.677 |
| Brij 35P | 2.5 | EP 145V | 0.95 | S LPC (as SL 80-3) | ca. 0.01 (0.05) | 16.9 | 88.1 | 813.1 | 0.713 |

Table 11b - Exemplary aqueous compositions containing surfactant, lipid and co-surfactant, where the ratio of surfactant to lipid is varied

| Surf. | Surf. % | Lipid | Lipid % | Co-surf. | Co-surf. % | Ratio | Turbidity (FNU) | Particle Size (nm) | PDI |
|---|---|---|---|---|---|---|---|---|---|
| Brij 35P | 3.5 | 90H | 0.95 | S LPC (as SL 80-3) | ca. 0.01 (0.05) | 3.5 | 7.08 | 9.99 | 0.320 |
| Brij 35P | 2.5 | 90H | 0.76 | S LPC (as SL 80-3) | ca. 0.008 (0.04) | 3.125 | 7.15 | 10.62 | 0.261 |
| Brij 35P | 2.5 | 90H | 0.95 | S LPC (as SL 80-3) | ca. 0.01 (0.05) | 2.5 | 14.74 | 11.0 | 0.452 |
| Brij 35P | 1.0 | 90H | 0.95 | S LPC (as SL 80-3) | ca. 0.01 (0.05) | 1 | 42.2 | 20.53 | 0.324 |
| Brij 35P | 0.5 | 90H | 0.95 | S LPC (as SL 80-3) | ca. 0.01 (0.05) | 0.5 | >150 | 1734 | 0.876 |

Table 11c- Exemplary aqueous compositions containing surfactant, lipid, co-surfactant and a range of active agents

| Surf. | Surf. % | Lipid | Lipid % | Co-surf. | Co-surf. % | Active | Active % | Turbidity (FNU) | Particle Size (nm) | PDI |
|---|---|---|---|---|---|---|---|---|---|---|
| Brij 35P | 2.5 | 90H | 0.85 | S LPC (as SL 80-3) | ca. 0.01 (0.05) | TECA | 0.1 | 62.0 | 57.11 | 0.337 |
| Brij 35P | 2.5 | 90H | 0.65 | S LPC (as SL 80-3) | ca. 0.01 (0.05) | TECA | 0.3 | 32.4 | 49.47 | 0.260 |
| Brij 35P | 2.5 | 90H | 0.45 | S LPC (as SL 80-3) | ca. 0.01 (0.05) | TECA | 1.0 | 42.4 | 46.76 | 0.216 |
| Brij 35P | 2.5 | 90H | 0.7 | S LPC (as SL 80-3) | ca. 0.01 (0.05) | Hydrocortisone | 0.5 | 8.41 | 10.36 | 0.356 |

Discussion

As an indication of approximate boundaries, analysis of turbidity versus particle size data suggests that turbidity values of less than 75 FNU are generally characterised by particle sizes of less than 50 nm, while turbidity values of less than 25 FNU are generally characterised by a particle size of less than 17 nm.

**Example 10 - An aqueous gel preparation for collagen stimulation**

Method

Solution A:

A stock solution of surfactant, co-surfactant, lipid and active agent was initially prepared in water at double the

desired final concentration. Briefly, co-surfactant, lipid and active agent were dissolved in ethanol, while heating (approximately 30 °C) and stirring. Surfactant material was then added whilst maintaining heat and stir conditions. Ethanol was subsequently removed via rotary evaporation and the resulting dry material was then resolubilised in water with warming (ca. 50 °C) at double the desired final concentration. The resulting solution is referred to as Solution A and its composition is summarised in Table 12.

Table 12 - Solution A Composition

| Component | Mass g | Concentration % |
|---|---|---|
| Brij 35P | 1.5625 | 6.25 |
| 90H | 0.59375 | 2.375 |
| SL 80-3 | 0.03125 | 0.1325 |
| TECA | 0.5 | 2 |
| Water | 22.3125 | 89.25 |

Solution B:

A gel solution containing preservative was prepared at double the desired final concentration. Nipaguard PDU was dissolved into water while heating (approximately 30°C) and stirring. Blanose® 7HF was then added until a uniform gel was formed. The resulting solution is referred to as Solution B and its composition is summarised in Table 13.

Table 13 - Solution B Composition

| Component | Mass g | Concentration % |
|---|---|---|
| Blanose® 7HF | 1.0 | 4 |
| Nipaguard PDU | 0.5 | 2 |
| Water | 23.5 | 94 |

Solution A and Solution B were then mixed in equal volumes to produce the final preparation.

*Surfactants*

Brij 35P was as described in Example 1.

*Lipid*

90H was as described in Comparative Example 1.

*Co-surfactants*

SL 80-3 was as described in Example 1.

*Active Agent*

TECA was as described in Comparative Example 2.

*Preservative*

Nipaguard PDU (diazolidinyl urea, methylparaben, propylparaben) supplied by NIPA Biocides, Clariant UK Ltd. Leeds (UK).

*Viscosity Modifiers*

Blanose® 7HF supplied by Aqualon (USA).

Results

A cosmetic preparation of the active agent TECA, which has poor water solubility, was successfully prepared in a clear and colourless aqueous gel.

**Example 11 - An aqueous gel preparation for collagen stimulation and antioxidant/skin whitener**

Method

Solution A:

A stock solution of surfactant, co-surfactant, lipid and active agent was initially prepared. Briefly, co-surfactant, lipid and active agent were dissolved in ethanol, while heating (approximately 30 °C) and stirring. Surfactant material was then added whilst maintaining heat and stir conditions. Ethanol was subsequently removed via rotary evaporation and the resulting dry material was then resolubilised in water with warming (ca. 50 °C) at four times the desired final concentration. The resulting solution is referred to as Solution A and its composition is summarised in Table 14.

Table 14 - Solution A Composition final weight 12.5 g

| Component | Mass g | Concentration % |
|---|---|---|
| Brij 35P | 1.5625 | 12.5 |
| 90H | 0.59375 | 4.75 |
| SL80-3 | 0.03125 | 0.25 |
| TECA | 0.5 | 4 |
| Water | 9.8125 | 78.5 |

Solution B:

A stock solution of surfactant, co-surfactant, lipid and active agent was initially prepared. Briefly, co-surfactant, lipid and active agent were dissolved in ethanol, while heating (approximately 30 °C) and stirring. Surfactant material was then added whilst maintaining heat and stir conditions. Ethanol was subsequently removed via rotary evaporation and the resulting dry material was then resolubilised in water with warming (ca. 50 °C) at the four times the desired final concentration. The resulting solution is referred to as Solution B and its composition is summarised in Table 15.

Table 15 - Solution B Composition final weight 12.5 g

| Component | Mass g | Concentration % |
|---|---|---|
| Brij 35P | 1.5625 | 12.5 |
| 90H | 0.59375 | 4.75 |
| SL 80-3 | 0.03125 | 0.25 |
| VC-P | 0.3125 | 2.5 |
| Water | 10.0 | 80 |

Solution C:

A gel solution containing preservative was prepared at double the desired final concentration. Nipaguard DMDMH was dissolved into water while heating (approximately 30 °C) and stirring. Blanose® 7HF was then added until a uniform gel was formed. The resulting solution is referred to as Solution C and its composition is summarised in Table 16.

Table 16 - Solution C Composition final weight 25 g

| Component | Mass g | Concentration % |
|---|---|---|
| Blanose® 7HF | 1.0 | 4 |

(continued)

| Component | Mass g | Concentration % |
|---|---|---|
| Nipaguard DMDMH | 0.2 | 0.8 |
| Water | 23.8 | 95.2 |

Solution A (12.5 g), Solution B (12.5 g) and Solution C (25 g) were then mixed.

*Surfacatants*

Brij 35P was as described in Example 1.

*Lipid*

90H was as described in Comparative Example 1.

*Co-surfactants*

SL 80-3 was as described in Example 1.

*Active Agent*

TECA was as described in Comparative Example 2.

VC-P is vitamin C dipalmitate, available from Nikko Chemicals Co. Ltd. (Japan), CAS 28474-90-0.

*Preservative*

Nipaguard DMDMH (DMDM hydantoin) supplied by NIPA Biocides, Clariant UK Ltd. Leeds (UK).

*Viscosity Modifiers*

Blanose® 7HF supplied by Aqualon (USA)

Results

A cosmetic preparation of the active agents TECA (collagen stimulator) and VC-P (antioxidant/skin whitener), which have poor water solubility, was successfully prepared in a clear and colourless aqueous gel.

**Example 12 - Quantification of skin penetration**

1 ml of a solution of the invention containing surfactant (Brij 35P, 2.5%), lipid (90H, 1 %), co-surfactant (SL 80-30, 0.05%) and a dye having poor water solubility (D&C Red 27, 0.25%) was dosed on a 25x25 mm cross-linked adhesive hydrogel patch before being allowed to absorb for 1 hr.

Gels were contacted with forearm skin for 30 minutes. The skin surface was then briefly washed.

After washing, layers of the *stratum corneum* were removed by firmly applying a 2 cm wide section of Scotch™ Pressure Sensitive adhesive tape to the test area, and subsequently removing the tape section from the skin.

The tape section was then placed onto a transparent acetate sheet and examined using a white-light light box to assess the presence of colouration resulting from dye penetration. Application of tape sections was then repeated until no further dye penetration was noted.

**Example 13 - Quantification of skin penetration using TENS**

1 ml of a solution of the invention containing surfactant (Brij 35P, 2.5%), lipid (90H, 1%), co-surfactant (SL 80-30, 0.05%) and a dye having poor water solubility (D&C Red 27, 0.25%) was dosed on a 25x25 mm cross-linked adhesive hydrogel electrode patch before being allowed to absorb for 1 hr.

Gels were contacted with forearm skin for 30 minutes, during which time the gel was connected to a TENS stimulator set at 10 mA, 35 Hz and a pulse width of 300 us. The skin surface was then briefly washed.

After washing, layers of the *stratum corneum* were removed by firmly applying a 2 cm wide section of Scotch™ Pressure Sensitive adhesive tape to the test area, and subsequently removing the tape section from the skin.

The tape section was then placed onto a transparent acetate sheet and examined using a white-light light box or a Mexameter MX18 (Courage+Khazaka Electronic UK Ltd.) to assess the presence of colouration resulting from dye penetration. Application of tape sections was then repeated until no further dye penetration was noted.

**Example 14 - A hydrogel patch preparation with active agent**

1 ml of an aqueous solution of the compositions of the invention containing surfactant (Protasorb L20, 2.5% w/w), lipid (90H, 1 % w/w), co-surfactant (lysoPC 0.01%, provided in the form of SL 80-3 at 0.05% w/w) and active agent (spilanthes $CO_2$ extract, 0.25% w/w) was pipetted onto the surface of a hydrogel patch (Allmi-Care Ltd., product reference: S0242) which had been trimmed to approximately 40 mm by 90 mm in size. The solution was then allowed to absorb into the gel over the course of one hour.

The patch was worn on the facial skin of a volunteer, adjacent to eye, for a period of 30 minutes.

On removal of the patch sensations of numbness were reported in the area treated.

All references referred to in this application, including patent and patent applications, are incorporated herein by reference to the fullest extent possible.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

Unless specifically stated otherwise, all ratios and proportions are given on a weight to weight basis.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

**Claims**

**1.** A composition comprising lipid and surfactant, **characterised in that** the surfactant:

a) has an HLB number of less than 20;
b) is an ether surfactant;
c) is an ester surfactant; or
d) is an ionic surfactant

and the lipid and surfactant are in the form of macromolecular assemblies of less than 100 nm in diameter.

**2.** A composition according to claim 1, wherein the HLB of the surfactant is in the range of about 13.5 to about 17.

**3.** A composition according to any one of claims 1 to 2, wherein the surfactant has a molecular weight of less than about 10000 Da.

**4.** A composition according to any one of claims 1 to 3, wherein the surfactant is selected from the list consisting of octoxynol-12, nonoxynol-15, octoxynol-16, nonoxynol-20, laureth-8, laureth-10, laureth 23, ceteth-10, ceteth-15, ceteth-20, oleth-15, oleth-20, C11-15 pareth-12, C11-15 pareth-15, C11-15 pareth-20, C12-C13 pareth-23, ceteareth-20, ceteareth-25, ceteareth-30, isoceteth-20, isosteareth-20, PEG-20 stearate, PEG-40 stearate, polysorbate 20, sucrose laurate, sucrose myristate, decyl glucoside, PEG-5 cocamine, PEG-15 cocamine, sodium lauroyl glutamate, sodium cocoyl glycinate, sodium cocoyl glutamate, disodium cocoyl glutamate, potassium lauryl wheat amino acids, sodium lauryl oat amino acids, sodium lauryl wheat amino acids, sodium cocoyl apple amino acids, sodium cocoyl methyl taurate and surfactin.

**5.** A composition according to any one of claims 1 to 4, wherein the lipid is a single pure component.

**6.** A composition according to any one of claims 1 to 5, wherein the lipid is a lipid mixture of at least 50% phospholipids having a single headgroup type by weight.

7. A composition according to any one of claims 1 to 6 in which the ratio of surfactant to lipid is at least 0.5:1 on a weight basis.

8. A composition according to any one of claims 1 to 7 in which the ratio of surfactant to lipid is 10:1 or lower on a weight basis.

9. A formulation comprising a composition according to any one of claims 1 to 8 and an active agent.

10. A formulation according to claim 9, wherein the active agent is present in an amount of 0.001-50% of the weight of surfactant and lipid.

11. A formulation according to either of claims 9 or 10 wherein the active agent is selected from TECA, Myristyl ester of L-pyrrolidone, lauric ester of L-pyrrolidone carboxylic acid, Ciclopirox olamine, Econazole nitrate, Red clover extract, Centella extract, Butcher's broom extract, Benzyl nicotinate, Piroctone olamine, acetyl hexapeptide-3, extract of *Ginkgo biloba,* Horse chestnut extract, Nettle extract, Aesculus extract, Yohimbine free base, Hydrocortisone, Salmeterol xinafoate, Progesterone, Devil's claw extract, Gatuline® Expression, extract of *Picea abies,* D-Camphor, Totara-8,11,13-trien-13-ol, extract of *Spilanthes acmella,* Undecylenoyl phenylalanine, extract of *Cimicifuga racemosa,* extract of *Boswellia serrata,* Sichuan pepper extract and Prickly ash extract.

12. A formulation according to either of claims 9 or 10 wherein the active agent is selected from 7-dehydrocholesterol, apricosal, ascorbyl palmitate, avobenzene, betamethasone 17-valerate, *Boswellia,* camphor, capsaicin, Cha-Plu extract, cholesterol sulphate, cholesterol, clobetasol propionate, clotrimazole, Cosmoperine, diclofenac, *Echinacea angustafolia, Echinacea purpurea,* Edemine, erythromycin sulphate, eserine, Eusolex 4360, Fougere, *Galanga, Ginkgo, Heliopsis* extract, hops tincture, hydrocortisone 17-butyrate, Japanese pepper extract, ketaconazole, ketoprofen, maca, melaleucol, minoxidil, naproxen, NDGA, neomycin sulphate, nystatin, octyl salicylate, PABA, PT-40, P-U, Questice CQ U/A, rosemary extract CG, rosmarinic acid (90%), soy isoflavones CG (50%), *Spilanthes* supercritical $CO_2$ extract, stearyl glycrrhetinate, tarragon extract, tasmanian pepper extract, THC CG, THC Ultra Pure, Unisex Bouquet, Unisol S-22, vitamin C palmitate, vitamin $D_3$, vitamin E.

13. A formulation according to any one of claims 9 to 12 which is in the form of an aqueous solution and wherein the quantity of active agent is in the range 0.001 % to 20% of the total weight.

14. An aqueous formulation according to any one of claims 9 to 12 wherein the active agent is present in an amount which exceeds the solubility level of the active agent in aqueous solution with the same amount of surfactant alone.

15. A composition or formulation according to any one of claims 1 to 15, wherein the macromolecular assemblies are less than 50 nm in diameter.

Figure 1

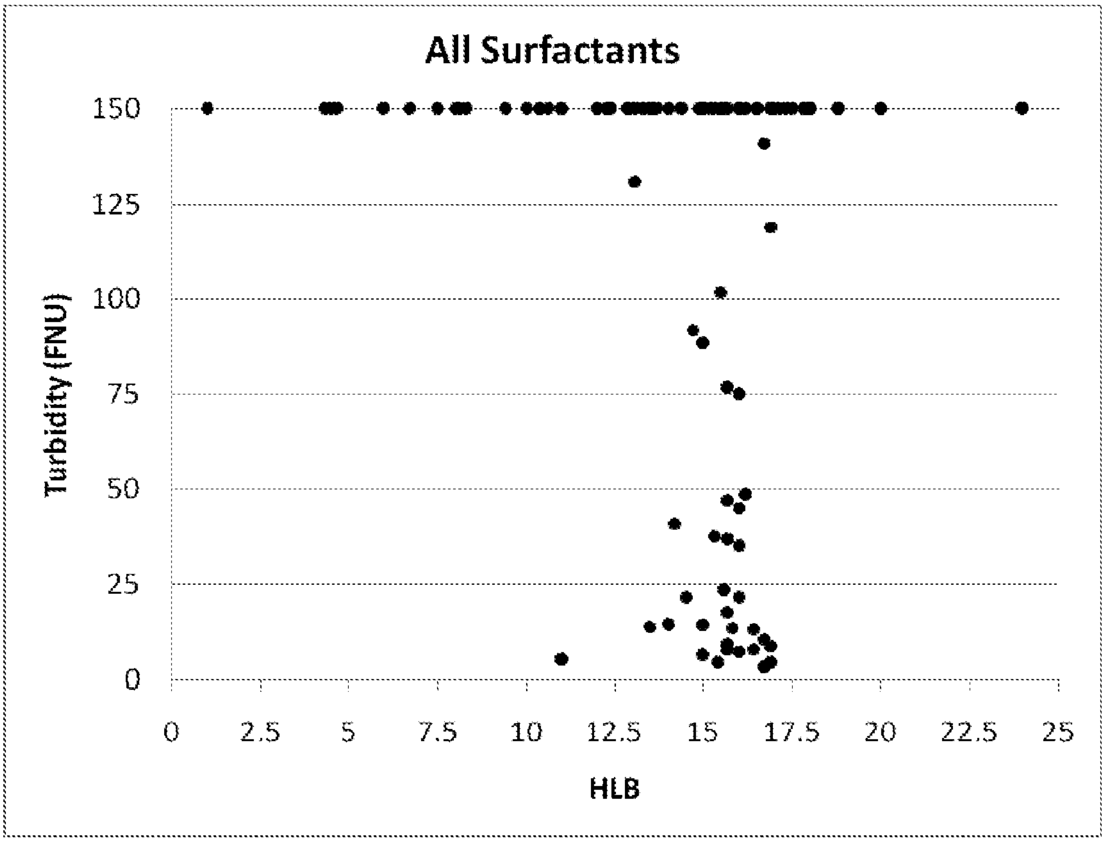


Figure 2

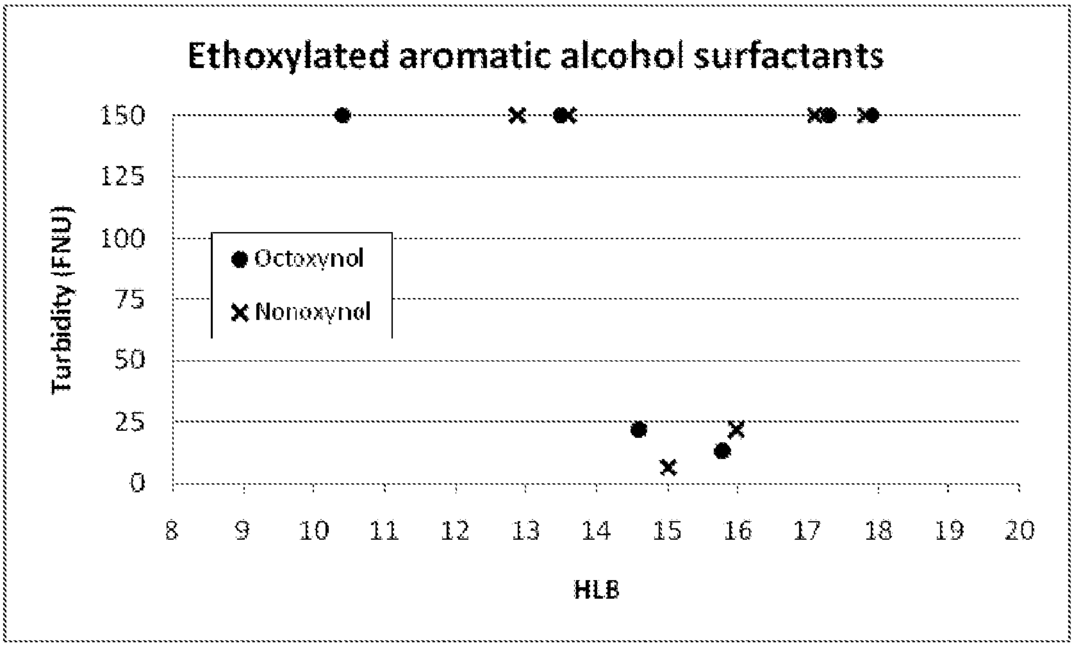

Figure 3

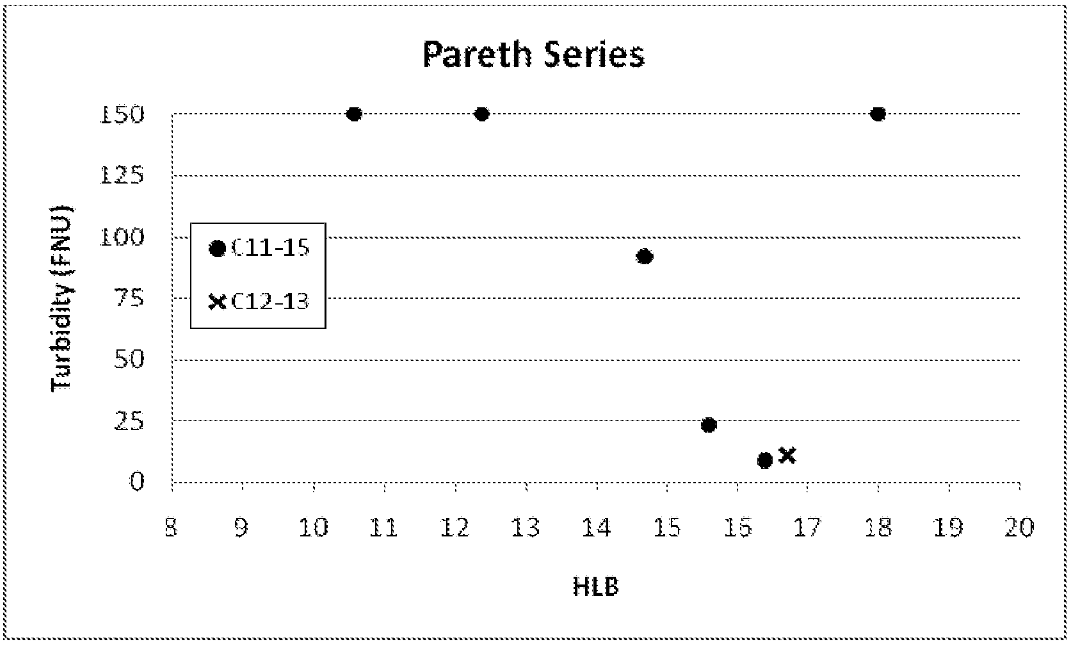


Figure 4

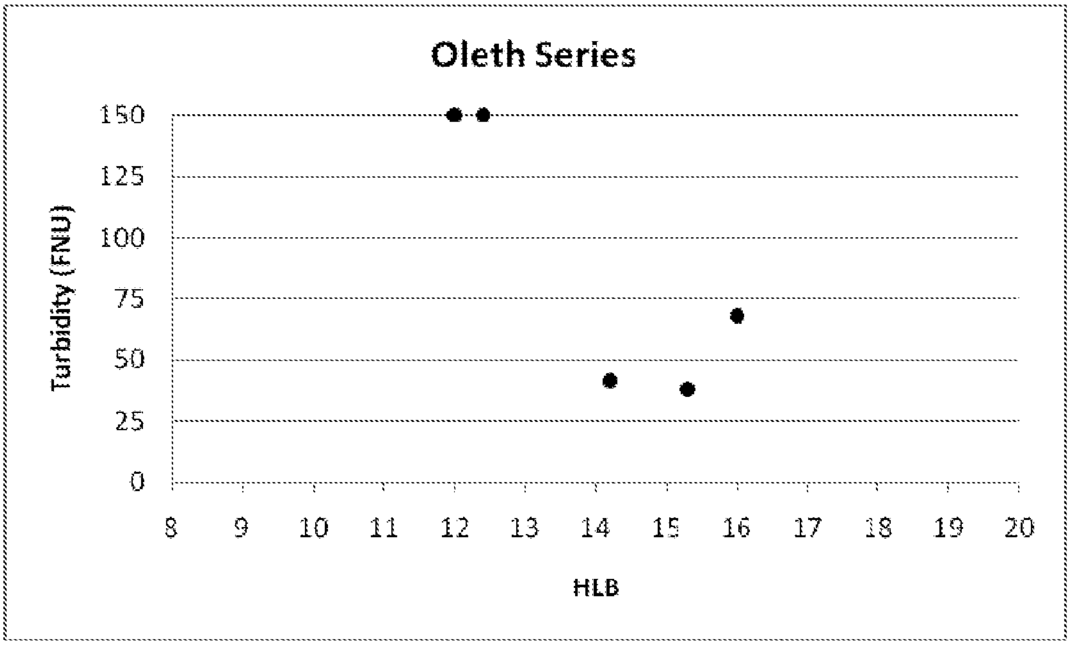

Figure 5

## Size Distribution Report by Intensity

**Sample Details**

| | |
|---|---|
| **Sample Name:** | Sample Average A2 Repeat 1 |
| **SOP Name:** | mansettings.dat |
| **General Notes:** | Average result created from record number(s): 14 15 16 17 18 |

| | | | |
|---|---|---|---|
| **File Name:** | PCL-07-211.dts | **Dispersant Name:** | Water |
| **Record Number:** | 97 | **Dispersant RI:** | 1.330 |
| **Material RI:** | 1.52 | **Viscosity (cP):** | 0.8872 |
| **Material Absorbtion:** | 0.10 | **Measurement Date and Time:** | 16 November 2007 17:46:41 |

**System**

| | | | |
|---|---|---|---|
| **Temperature (°C):** | 25.0 | **Duration Used (s):** | 60 |
| **Count Rate (kcps):** | 371.1 | **Measurement Position (mm):** | 4.65 |
| **Cell Description:** | Disposable sizing cuvette | **Attenuator:** | 7 |

**Results**

| | | | **Diam. (nm)** | **% Intensity** | **Width (nm)** |
|---|---|---|---|---|---|
| Z-Average (d.nm): | 266.4 | **Peak 1:** | 16.98 | 91.3 | 5.938 |
| PdI: | 0.363 | **Peak 2:** | 803.3 | 7.1 | 179.2 |
| **Intercept:** | 0.974 | **Peak 3:** | 330.1 | 1.2 | 53.98 |

**Result quality :** Refer to quality report

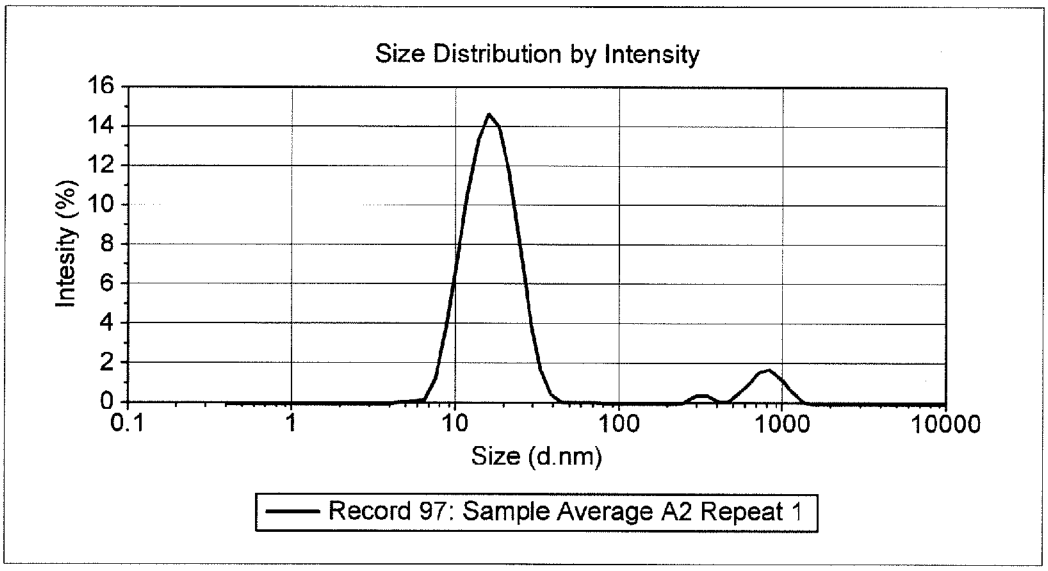

Figure 6

## Size Distribution Report by Intensity

Sample Details

**Sample Name:** Sample Average A4 Repeat 1

**SOP Name:** mansettings.dat

**General Notes:** Average result created from record number(s): 38 39 40 41 42

| | | | |
|---|---|---|---|
| **File Name:** | PCL-07-211.dts | **Dispersant Name:** | Water |
| **Record Number:** | 99 | **Dispersant RI:** | 1.330 |
| **Material RI:** | 1.52 | **Viscosity (cP):** | 0.8872 |
| **Material Absorbtion:** | 0.10 | **Measurement Date and Time:** | 16 November 2007 17:47:21 |

System

| | | | |
|---|---|---|---|
| **Temperature (°C):** | 25.0 | **Duration Used (s):** | 50 |
| **Count Rate (kcps):** | 471.8 | **Measurement Position (mm):** | 4.65 |
| **Cell Description:** | Disposable sizing cuvette | **Attenuator:** | 8 |

Results

| | | | Diam. (nm) | % Intensity | Width (nm) |
|---|---|---|---|---|---|
| Z-Average (d.nm): | 47.35 | **Peak 1:** | 13.44 | 93.1 | 4.290 |
| PdI: | 0.211 | **Peak 2:** | 371.5 | 4.1 | 132.7 |
| **Intercept:** | 0.917 | **Peak 3:** | 5258 | 2.8 | 441.1 |

**Result quality :** Refer to quality report

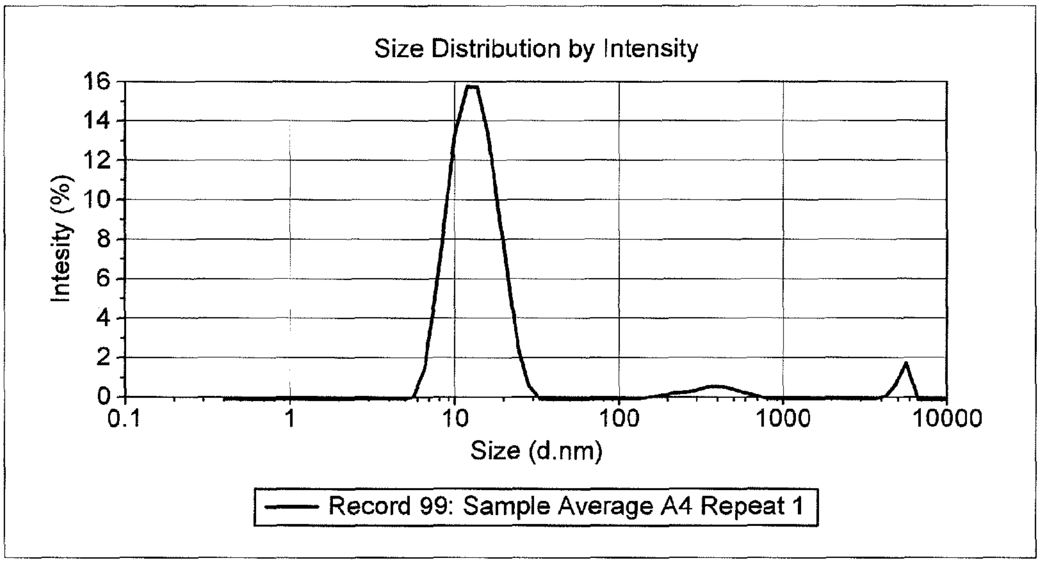

Figure 7

## Size Distribution Report by Intensity

Sample Details

**Sample Name:** Sample Average A7 Repeat 1

**SOP Name:** mansettings.dat

**General Notes:** Average result created from record number(s): 73 74 75 76 77

| | | | |
|---|---|---|---|
| **File Name:** | PCL-07-211.dts | **Dispersant Name:** | Water |
| **Record Number:** | 102 | **Dispersant RI:** | 1.330 |
| **Material RI:** | 1.52 | **Viscosity (cP):** | 0.8872 |
| **Material Absorbtion:** | 0.10 | **Measurement Date and Time:** | 16 November 2007 17:48:31 |

System

| | | | |
|---|---|---|---|
| **Temperature (°C):** | 25.0 | **Duration Used (s):** | 50 |
| **Count Rate (kcps):** | 293.3 | **Measurement Position (mm):** | 4.65 |
| **Cell Description:** | Disposable sizing cuvette | **Attenuator:** | 6 |

Results

| | | | Diam. (nm) | % Intensity | Width (nm) |
|---|---|---|---|---|---|
| Z-Average (d.nm): | 581.4 | **Peak 1:** | 1107 | 65.0 | 381.8 |
| PdI: | 0.853 | **Peak 2:** | 151.4 | 22.2 | 45.42 |
| **Intercept:** | 0.990 | **Peak 3:** | 20.99 | 8.9 | 6.834 |

**Result quality :** Refer to quality report

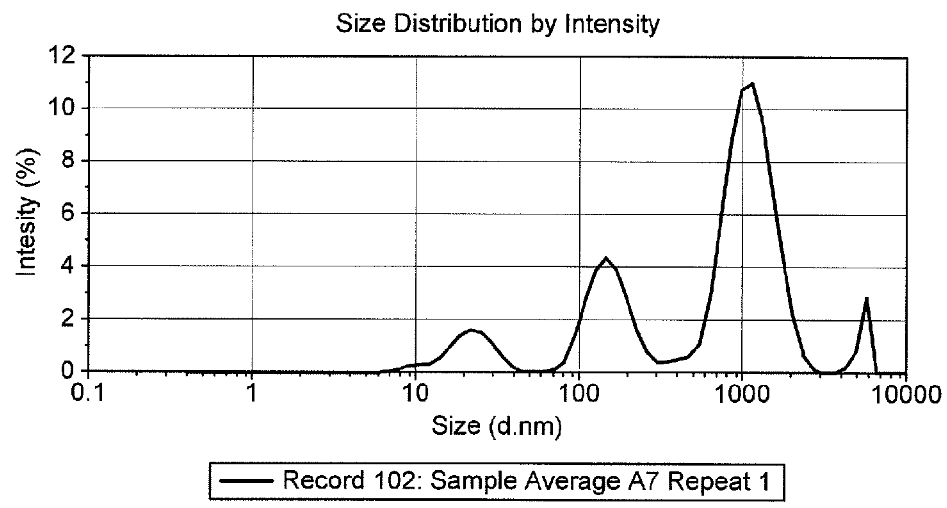

Figure 8

## Size Distribution Report by Intensity

Sample Details

| | |
|---|---|
| **Sample Name:** | Sample Average C3 run 1 |
| **SOP Name:** | mansettings.dat |
| **General Notes:** | Average result created from record number(s): 183 184 185 186 187 |

| | | | |
|---|---|---|---|
| **File Name:** | PCL-07-211.dts | **Dispersant Name:** | Water |
| **Record Number:** | 321 | **Dispersant RI:** | 1.330 |
| **Material RI:** | 1.52 | **Viscosity (cP):** | 0.8872 |
| **Material Absorbtion:** | 0.10 | **Measurement Date and Time:** | 16 November 2007 16:32:11 |

System

| | | | |
|---|---|---|---|
| **Temperature (℃):** | 25.0 | **Duration Used (s):** | 50 |
| **Count Rate (kcps):** | 296.5 | **Measurement Position (mm):** | 4.65 |
| **Cell Description:** | Disposable sizing cuvette | **Attenuator:** | 6 |

Results

| | | | **Diam. (nm)** | **% Intensity** | **Width (nm)** |
|---|---|---|---|---|---|
| **Z-Average (d.nm):** | 38.82 | **Peak 1:** | 46.76 | 99.5 | 19.92 |
| **PdI:** | 0.216 | **Peak 2:** | 4743 | 0.5 | 761.3 |
| **Intercept:** | 0.953 | **Peak 3:** | 0.000 | 0.0 | 0.000 |
| **Result quality :** | Good | | | | |

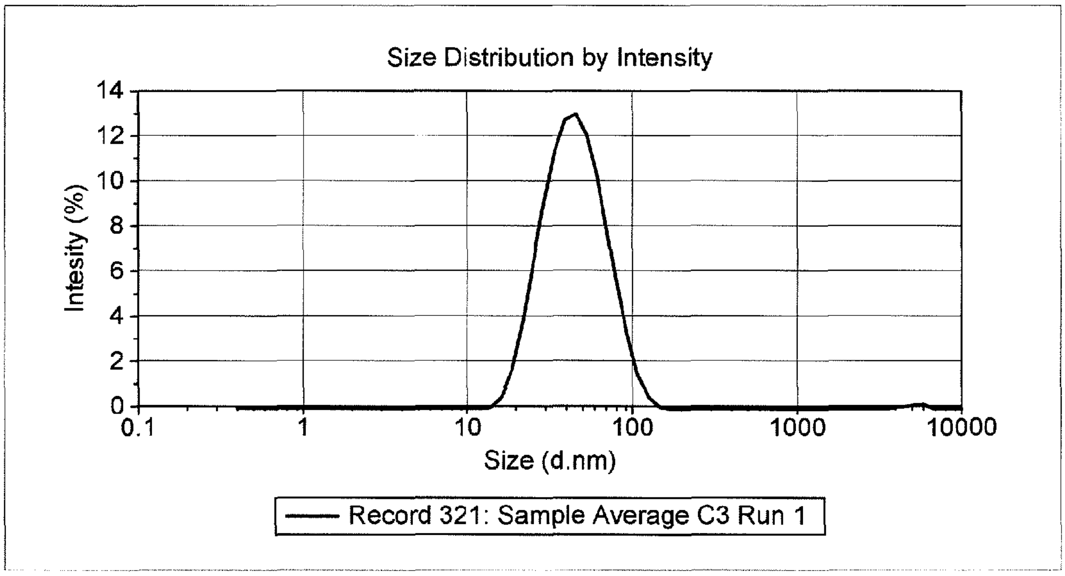

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6770299 B
- US 5853755 A
- US 6656499 B
- US 4508703 A
- US 6165500 A
- WO 0050007 A
- US 6267985 B
- US 4217344 L, L'Oreal
- US 6004580 A, Leiras Oy
- US 6245349 B, Elan
- WO 0037042 A
- US 20020146375 A1
- WO 03082222 A
- US 20050124705 A1
- US 20030152635 A1
- WO 2006013369 A
- WO 2006077362 A
- WO 99009955 A
- EP 1007002 A
- US 6426905 B
- GB 2006050134 W
- WO 2006129127 A
- GB 2006050134 A
- GB 9004813 A
- GB 9004960 A


### Non-patent literature cited in the description


- **GRIFFIN, WC.** *Journal of the Society of Cosmetic Chemists,* 1949, vol. 1, 311-326
- **GRIFFIN WC.** *Journal of the Society of Cosmetic Chemists,* 1954, vol. 5, 249-256
- **FLORENCE AT et al.** Physiochemical Principles of Pharmacy. Chapman & Hall, 1982, 234-235
- **AULTON ME.** Pharmaceutics - The Science of Dosage Form Design. 2002, 96-97
- PHARMACEUTICS - THE SCIENCE OF DOSAGE FORM DESIGN. 345-347
- **DAVIES JT et al.** Interfacial Phenomena. Academic Press, 1961
- **MCCUTCHEON.** Emulsifiers & Detergents. MC Publishing Company, 2005, vol. 1
- **MÜLLER RH et al.** *Advanced Drug Delivery Reviews,* 2002, vol. 54 (1), S131-S155
- **JAMIL H et al.** *Modern Drug Discovery,* 2004, vol. 7, 37-3940-180
- **ZUMBUEHL O ; WEDER H.** *Biochem. Biophys. ACTA,* 1981, vol. 640, 252-262100-500
- Liposomes, Transdermal Drug Delivery. Pharmaceutical Press, 2003
- **CEVC G.** *Advanced Drug Delivery Reviews,* 2004, vol. 56, 675-711
- **HOFLAND HEJ et al.** *British Journal of Dermatology,* 1995, vol. 132, 853-856
- **VAN DEN BERGH BAI et al.** *Biochemica et Biophysica Acta,* 1999, vol. 1461, 155-173
- **BOUWSTRA JA et al.** *Advanced Drug Delivery Reviews,* 2002, vol. 54 (1), S41-S55
- **PLESSIS J et al.** *International Journal of Pharmaceutics,* 1994, vol. 103, 277-282
- **UCHEGBU IF et al.** *Advances in Colloid and Interface Science,* 1995, vol. 58, 1-55
- **UCHEGBU IF et al.** *International Journal of Pharmaceutics,* 1998, vol. 172, 33-70
- **SANTUCCI E et al.** *STP Pharma Sciences,* 1996, vol. 6, 29-32
- **KRIELGAARD M.** *Advanced Drug Delivery Reviews,* 2002, vol. 54 (1), S41-S55
- Microemulsions in Cosmetics. Micelle Press, 2001
- Gattefosse Technical Brochure. 1998
- **MALZERT-FRÉON A et al.** *International Journal of Pharmaceutics,* 2006, vol. 320 (1-2), 157-164
- **EGAN RW et al.** *Journal of Biological Chemistry,* 1976, vol. 251, 4442-4447
- **GONI FM et al.** *European Journal of Biochemistry,* 1986, vol. 160, 659-665
- **WALTER et al.** *Biophysics Journal,* 1991, vol. 60, 1315-1325
- **ALMGREN M.** *Biochemica et Biophysica Acta,* 2000, vol. 1508, 146-163
- **FUNARI SS et al.** *Proceedings of the National Academy of Sciences,* 2001, vol. 98 (16), 8938-8943
- **PARTEARROYO MA et al.** *Journal of Colloid and Interface Science,* 1996, vol. 178, 156-159
- **EDWARDS K et al.** *Biophysical Journal,* 1997, vol. 73, 258-266
- **ELISABET BOIJA.** A doctoral thesis. 2006
- **JOHNSSON M et al.** *Biophysics Journal,* 2003, vol. 85, 3839-3847

- **JOHANSSON E et al.** *Biophysical Chemistry,* 2005, vol. 113, 183-192
- **SEKI, K ; TIRRELL, D.** *Macromolecules,* 1983, vol. 17, 1692-1698
- **TIRRELL, D ; TAKIGAWA, D ; SEKI, K.** *Ann. New York Acad. Sci.,* 1985, vol. 446, 237-248
- **THOMAS, JL ; DEVLIN BP ; TIRRELL, DA.** *Biochimica et Biophysica Acta,* 1996, vol. 1278, 73-78
- **TONGE, SR ; TIGHE, BJ.** *Advanced Drug Delivery Reviews,* 2001, vol. 53, 109-122
- **FICHTNER, F ; SCHONERT, H.** *Colloid & Polymer Sci.,* 1977, vol. 255, 230-232
- **THOMAS, JL ; DEVLIN BP ; TIRRELL DA.** *Biochimica et Biophysica Acta,* 1996, vol. 1278, 73-78
- **SUGAI, S ; OHNO, N.** *Biophys. Chem.,* 1980, vol. 11, 387-395
- Handbook of Industrial Surfactants. Gower Publishing Company, 1993
- Surfactant Encyclopaedia, Cosmetics & Toiletries Resource Series. 1996
- **GOHON Y et al.** *Analytical Biochemistry,* 2004, vol. 334, 318-334
- **POCANSCHI CL et al.** *Biochemistry,* 2006, vol. 45, 13954-13961
- **STASHENKO E et al.** *J. Chromatog. A.,* 1996, vol. 752, 223-232
- **CARNEY, LG ; HILL, RM.** *Arch. Ophthalmol.,* 1976, vol. 94 (5), 821-824
- **ANGERS S et al.** *Proceedings of the National Academy of Science USA,* 2000, vol. 97 (7), 3684-3689
- **PORTOGHESE PS et al.** *Journal of Medicinal Chemistry,* 2001, vol. 44 (14), 2259-2269
- **LALCHANDANI SH. et al.** *Journal of Pharmacology and Experimental Therapeutics,* 2002, vol. 303 (3), 979-984
- **M ASH ; I ASH.** Handbook of Industrial Surfactants. Gower Publishing Company, 1993
- **AULTON ME.** Pharmaceutics- The Science of Dosage Form Design. 2002